# EUROPEAN PATENT APPLICATION

(11) **EP 4 748 450 A2**
(43) Date of publication of application: **27.05.2026**
(21) Application number: 26156949.5
(22) Date of filing: 22.09.2020
(51) Int. Cl.: A61P 35/00

(54) **TREATMENT INVOLVING THERAPEUTIC ANTIBODY AND INTERLEUKIN-2 (IL2)**

(30) Priority: 24.09.2019 WO PCT/EP2019/075712
(62) Divisional of application: 20772086.3
(71) Applicant: BioNTech SE, 55131 Mainz (DE); TRON - Translationale Onkologie an der Universitätsmedizin der Johannes Gutenberg- Universität Mainz gemeinnützige GmbH, 55131 Mainz (DE)
(72) Inventor: SAHIN, Ugur, 55131 Mainz (DE); VORMEHR, Mathias, 55131 Mainz (DE); BECK, Jan David, 55118 Mainz (DE); DIKEN, Mustafa, 55130 Mainz (DE); KREITER, Sebastian, 55131 Mainz (DE)
(74) Representative: Thomann, William John

(57) **Abstract**

Tumor cells often evade an immune response, e.g., by reducing or eliminating MHC expression and/or IFN-signaling, which enables uncontrolled growth. We demonstrate herein that antibody-based immunotherapy in combination with IL2 administration is an effective therapy against such resistant tumors. Specifically, the present disclosure relates to methods of treating a subject with cancer that is at least partially resistant to an MHC-dependent T cell response comprising administering to the subject: a. a polypeptide comprising IL2 or a functional variant thereof or a polynucleotide encoding a polypeptide comprising IL2 or a functional variant thereof; and b. antibody-based immunotherapy.

## Description

### Technical Field

The present disclosure relates to methods of treating a subject with cancer that does not adequately respond to treatment based on MHC-dependent T cells, e.g., due to MHC-I deficiency of the cancer or deficiency of the cancer in IFN-signaling. These methods are, in particular, useful for the treatment of cancer diseases characterized by diseased cells expressing an antigen on their cell surface. Specifically, the present disclosure relates to methods of treating a subject with cancer that is at least partially resistant to an MHC-dependent T cell response comprising administering to the subject: a. a polypeptide comprising IL2 or a functional variant thereof (referred to herein generally as "IL2") or a polynucleotide encoding a polypeptide comprising IL2 or a functional variant thereof; and b. antibody-based immunotherapy against cancer. The present disclosure also relates to methods of preventing a cancer to develop a resistance to an MHC-dependent T cell response in a subject with cancer comprising administering to the subject: a. a polypeptide comprising IL2 or a functional variant thereof or a polynucleotide encoding a polypeptide comprising IL2 or a functional variant thereof; and b. antibody-based immunotherapy against cancer. The methods of the disclosure may further comprise administering to the subject a further cancer therapy such as chemotherapy or radiation therapy, in particular chemotherapy.

### Background

The immune system plays a crucial role during cancer development, progression and therapy. CD8⁺ T cells and NK cells can directly lyse tumor cells and high tumor-infiltration of these cells is generally regarded as favorable for the outcome of various tumor diseases. CD4⁺ T cells contribute to the antitumor immune response by secretion of IFNy or licensing of antigen-presenting dendritic cells (DCs), which in turn prime and activate CD8⁺ T cells (Kreiter et al. Nature 520, 692-6 (2015)). The recognition and elimination of tumor cells by CD8⁺ T cells depends on antigen presentation *via* the Major Histocompatibility Complex (MHC) class I. MHC class I molecules are located on the cell surface and are comprised of an α-chain and β2-microglobulin (B2M). Every human individual carries six different alleles of genes encoding for the α-chain and two redundant alleles of genes encoding for B2M. While B2M is required for the stability of the complex, the α-chain forms a groove that accommodates a peptide antigen, which can be in principal derived by any endogenous protein. If the antigen is immunogenic (for example because it is derived from a mutated or viral gene product), CD8⁺ T cells carrying T cell receptors (TCRs) matching this particular peptide-MHC class I complex can recognize the tumor cell as foreign. If the CD8⁺ T cell is primed and activated, it will lyse the tumor cell and secrete IFNy. Surrounding tumor cells are able to sense IFNγ, which leads to growth inhibition and enhanced antigen presentation through upregulation of MHC class I, making the tumor cells more susceptible to CD8⁺ T cell recognition. Additionally, CD4⁺ T cells and NK cells located in the tumor microenvironment (TME) can be sources of IFNγ, while DCs and macrophages can secrete type I IFN with similar effects on growth and antigen presentation of the tumor cells.

Several immunotherapies, such as adoptive T cell transfer, recombinant cytokines or immune checkpoint blockade (ICB) are approved for the treatment of cancer patients. Especially ICB has revolutionized the field of cancer treatment, leading to durable responses in a group of patients (Larkin et al. N Engl J Med 373, 23-34 (2015)). ICB relies on re-invigoration of preexisting CD8⁺ T cell responses against the tumor cells. Due to the success of ICB, additional T cell based immunotherapy approaches are highly promising and numerous are currently under investigation in clinical trials. However, CD8⁺ T cell based therapies have the inherent disadvantage of the dependency on functional antigen presentation by the tumor cells, which opens avenues for the development of resistance mechanisms.

Tumor cells are frequently genetically instable (Burrell et al. Nature 501, 338-45 (2013)). For this reason, they accumulate mutations, which can lead to non-functional gene products (e.g. through missense- or nonsense-mutations) or to the deletion of entire genes. The functional loss of genes that are essential for response to cancer treatment can be involved in primary, adaptive or acquired resistance (Sharma et al. Cell 168, 707-23 (2017)). Development of acquired resistance leads to selection and outgrowth of therapy-resistant tumor cell clones and frequently results in the death of the affected patients. In particular, mutations that impair antigen presentation have been observed as mechanisms of resistance against immunotherapies, including adoptive T cell transfer, ICB and vaccination (Restifo et al. J Natl Cancer Inst 88, 100-8 (1996), Zaretsky et al. N Engl J Med 375, 819-29 (2016), Sahin et al. Nature 547, 222-6 (2017)). For example, the loss of MHC class I, especially through mutation of B2M leads to a complete shutdown of antigen presentation. Consequently, the tumor cells escape the recognition and elimination by CD8⁺ T cells. Depending on the tumor entity, partial or full loss of MHC class I alleles has been observed in up to 93% of tumors (Garrido et. al. Cancer Immunol Immunother 66, 259-271 (2017)), while the loss of *B2M* in particular has been shown to affect nearly 30% of patients not responding to ICB (Sade-Feldman et al. Nat Commun 8, 1136 (2017)). Moreover, mutations of several genes involved in the IFN-signaling pathway have been associated with resistance to ICB (Zaretsky et al. N Engl J Med 375, 819-29 (2016), Gao et al. Cell 167, 397-404 (2016)). For example, functional loss of the central IFN signaling molecule Janus kinase 1 (JAK1) impedes the response to both type I IFN and IFNy. In addition to immunotherapy, impairment of antigen presentation is also a potential mechanism of resistance against classical cancer therapies (chemo- and radiotherapy), since recent reports indicate that classical cancer therapies rely on the activation of a cellular immune response against the tumor cells (Galluzzi et. al. Cancer Cell 28, 690-714 (2015), Weichselbaum et. al. Nat Rev Clin Oncol 14, 365-379 (2017)).

The present disclosure confirms that impaired antigen presentation through loss of MHC class I is a comprehensive mechanism of resistance against immunotherapy in murine tumor models. In addition to that, data is provided showing that loss of MHC class I mediates resistance against classical cancer therapies. So far, there is no therapy described to be efficient against resistant tumors that evade immune recognition through impairment of antigen presentation through the complete or partial loss of functional MHC class I or the loss of functional IFN signaling. For this reason, there is a high medical need for novel strategies to restore immune recognition and elimination of resistant tumor cells.

Described herein is a strategy for the immunotherapeutic intervention against tumors that acquired resistance through alterations, which impair antigen presentation through loss of MHC class I or impaired IFN signaling pathways. The therapy comprises an antibody binding to a tumor antigen in combination with the cytokine IL2. The described combination therapy leads to tumor infiltration by a variety of immune cells and complete rejection of resistant tumors in murine models. Additionally, the present disclosure demonstrates that classical cancer therapy such as chemotherapy further improves the control of resistant tumors by antibody and IL2 combination immunotherapy.

### Summary

The present invention generally embraces the immunotherapeutic treatment of a subject comprising the administration of a polypeptide comprising IL2 or a functional variant thereof or a polynucleotide encoding a polypeptide comprising IL2 or a functional variant thereof and antibody-based immunotherapy against cancer in order to treat tumors that acquired resistance through alterations or to treat tumors to prevent the tumors from acquiring resistance through alterations, which alterations impair antigen presentation through loss of MHC class I or impaired IFN signaling pathways.

The methods and agents described herein are particularly effective if IL2 is attached to a pharmacokinetic modifying group (hereafter referred to as "extended-pharmacokinetic (PK) IL2"). The methods and agents described herein are particularly effective if the polynucleotide encoding IL2 such as extended-PK IL2 is RNA. In one embodiment, said RNA is targeted to the liver for systemic availability. Liver cells can be efficiently transfected and are able to produce large amounts of protein.

In one aspect, provided herein is a method of treating a subject with cancer that is at least partially resistant to an MHC-dependent T cell response comprising administering to the subject:
a. a polypeptide comprising IL2 or a functional variant thereof or a polynucleotide encoding a polypeptide comprising IL2 or a functional variant thereof; and
b. antibody-based immunotherapy against cancer.

In one embodiment, the cancer does not adequately respond to treatment based on, i.e., involving, T cells such as MHC-dependent T cells, in particular CD8⁺ T cells. In one embodiment, the cancer does not adequately respond to one or more of vaccination, immune checkpoint inhibition, and T cell transfer. In one embodiment, the cancer is deficient in processing and/or presenting antigens. In one embodiment, the cancer is MHC-I deficient. In one embodiment, the deficiency in MHC-I is due to a mutation or partial or full loss of MHC-I alleles or beta2-microglobulin (B2M). In one embodiment, the cancer is deficient in stimulating T cells. In one embodiment, the cancer is deficient in IFN-signaling such as type I IFN or IFNy signaling. Such deficiency in IFN-signaling may be due to one or more mutations in one or more genes involved in the IFN-signaling pathway. In one embodiment, the gene is Janus kinase 1 (JAK1).

In one aspect, provided herein is a method of preventing a cancer to develop a resistance to an MHC-dependent T cell response in a subject with cancer comprising administering to the subject:
a. a polypeptide comprising IL2 or a functional variant thereof or a polynucleotide encoding a polypeptide comprising IL2 or a functional variant thereof; and
b. antibody-based immunotherapy against cancer.

In one embodiment, the resistance comprises that the cancer does not adequately respond to treatment based on, i.e., involving, T cells such as MHC-dependent T cells, in particular CD8⁺ T cells. In one embodiment, the resistance comprises that the cancer does not adequately respond to one or more of vaccination, immune checkpoint inhibition, and T cell transfer. In one embodiment, the resistance comprises that the cancer is deficient in processing and/or presenting antigens. In one embodiment, the resistance comprises that the cancer is MHC-I deficient. In one embodiment, the deficiency in MHC-I is due to a mutation or partial or full loss of MHC-I alleles or beta2-microglobulin (B2M). In one embodiment, the resistance comprises that the cancer is deficient in stimulating T cells. In one embodiment, the resistance comprises that the cancer is deficient in IFN-signaling such as type I IFN or IFNy signaling. Such deficiency in IFN-signaling may be due to one or more mutations in one or more genes involved in the IFN-signaling pathway. In one embodiment, the gene is Janus kinase 1 (JAK1).

In one embodiment, the polynucleotide encoding a polypeptide comprising IL2 or a functional variant thereof is RNA.

In one embodiment, the antibody-based immunotherapy against cancer comprises administering a therapeutic antibody against cancer or a polynucleotide encoding a therapeutic antibody against cancer. In one embodiment, the therapeutic antibody against cancer is directed against a tumor antigen expressed by cells of the cancer. In one embodiment, the polynucleotide encoding a therapeutic antibody against cancer is RNA.

In one embodiment, the method comprises administering to the subject:
a. RNA encoding a polypeptide comprising IL2 or a functional variant thereof; and
b. a therapeutic antibody against cancer.

In one embodiment, the cancer is associated with expression or elevated expression of an antigen. In one embodiment, the antigen is a tumor antigen. In one embodiment, the antibody-based immunotherapy against cancer is directed against said antigen.

In one embodiment, the polypeptide comprising IL2 or a functional variant thereof is extended pharmacokinetic (PK) IL2. In one embodiment, the extended-PK IL2 comprises a fusion protein. In one embodiment, the fusion protein comprises a moiety of IL2 or a functional variant thereof and a moiety selected from the group consisting of serum albumin, an immunoglobulin fragment, transferrin, Fn3, and variants thereof. In one embodiment, the serum albumin comprises mouse serum albumin or human serum albumin. In one embodiment, the immunoglobulin fragment comprises an immunoglobulin Fc domain.

In one aspect, provided herein is a medical preparation comprising:
a. a polypeptide comprising IL2 or a functional variant thereof or a polynucleotide encoding a polypeptide comprising IL2 or a functional variant thereof;
b. a therapeutic antibody against cancer or a polynucleotide encoding a therapeutic antibody against cancer; and
c. instructions for use of the medical preparation for treating or preventing cancer that is at least partially resistant to an MHC-dependent T cell response.

In one aspect, provided herein is a medical preparation comprising:
a. a polypeptide comprising IL2 or a functional variant thereof or a polynucleotide encoding a polypeptide comprising IL2 or a functional variant thereof; and
b. a therapeutic antibody against cancer or a polynucleotide encoding a therapeutic antibody against cancer
   for treating or preventing cancer that is at least partially resistant to an MHC-dependent T cell response.

In one aspect, provided herein is a medical preparation comprising:
a. a polypeptide comprising IL2 or a functional variant thereof or a polynucleotide encoding a polypeptide comprising IL2 or a functional variant thereof;
b. a therapeutic antibody against cancer or a polynucleotide encoding a therapeutic antibody against cancer; and
c. instructions for use of the medical preparation for preventing a cancer to develop a resistance to an MHC-dependent T cell response.

In one aspect, provided herein is a medical preparation comprising:
a. a polypeptide comprising IL2 or a functional variant thereof or a polynucleotide encoding a polypeptide comprising IL2 or a functional variant thereof; and
b. a therapeutic antibody against cancer or a polynucleotide encoding a therapeutic antibody against cancer
   for preventing a cancer to develop a resistance to an MHC-dependent T cell response.

In one embodiment, the medical preparation is a kit.

In one embodiment, the medical preparation comprises the polypeptide comprising IL2 or a functional variant thereof or polynucleotide encoding a polypeptide comprising IL2 or a functional variant thereof, and the therapeutic antibody against cancer or polynucleotide encoding a therapeutic antibody against cancer in separate containers.

In one embodiment, the medical preparation is a pharmaceutical composition. In one embodiment, the pharmaceutical composition further comprises one or more pharmaceutically acceptable carriers, diluents and/or excipients.

In one aspect, provided herein is a polypeptide comprising IL2 or a functional variant thereof or a polynucleotide encoding a polypeptide comprising IL2 or a functional variant thereof for treating or preventing cancer that is at least partially resistant to an MHC-dependent T cell response, wherein the polypeptide comprising IL2 or a functional variant thereof or polynucleotide encoding a polypeptide comprising IL2 or a functional variant thereof is to be administered together with a therapeutic antibody against cancer or a polynucleotide encoding a therapeutic antibody against cancer.

In one aspect, provided herein is a therapeutic antibody against cancer or a polynucleotide encoding a therapeutic antibody against cancer for treating or preventing cancer that is at least partially resistant to an MHC-dependent T cell response, wherein the therapeutic antibody against cancer or polynucleotide encoding a therapeutic antibody against cancer is to be administered together with a polypeptide comprising IL2 or a functional variant thereof or a polynucleotide encoding a polypeptide comprising IL2 or a functional variant thereof.

In one aspect, provided herein is a polypeptide comprising IL2 or a functional variant thereof or a polynucleotide encoding a polypeptide comprising IL2 or a functional variant thereof for preventing a cancer to develop a resistance to an MHC-dependent T cell response, wherein the polypeptide comprising IL2 or a functional variant thereof or polynucleotide encoding a polypeptide comprising IL2 or a functional variant thereof is to be administered together with a therapeutic antibody against cancer or a polynucleotide encoding a therapeutic antibody against cancer.

In one aspect, provided herein is a therapeutic antibody against cancer or a polynucleotide encoding a therapeutic antibody against cancer for preventing a cancer to develop a resistance to an MHC-dependent T cell response, wherein the therapeutic antibody against cancer or polynucleotide encoding a therapeutic antibody against cancer is to be administered together with a polypeptide comprising IL2 or a functional variant thereof or a polynucleotide encoding a polypeptide comprising IL2 or a functional variant thereof.

In one embodiment, the medical preparation, the polypeptide comprising IL2 or a functional variant thereof or polynucleotide encoding a polypeptide comprising IL2 or a functional variant thereof, or the therapeutic antibody against cancer or polynucleotide encoding a therapeutic antibody against cancer are for use in the method of the invention as described above.

Preferred embodiments of the medical preparation, the polypeptide comprising IL2 or a functional variant thereof or polynucleotide encoding a polypeptide comprising IL2 or a functional variant thereof, or the therapeutic antibody against cancer or polynucleotide encoding a therapeutic antibody against cancer are as described above for the method of the invention.

### Brief description of the drawings

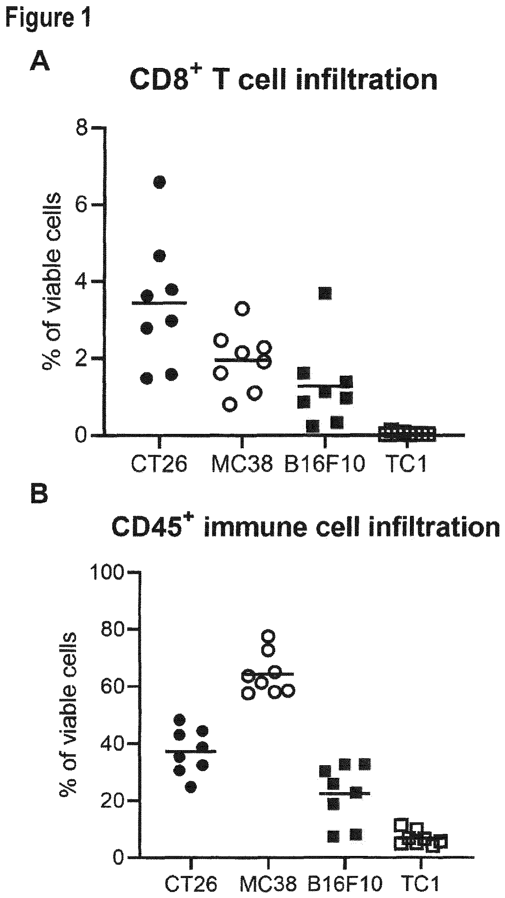

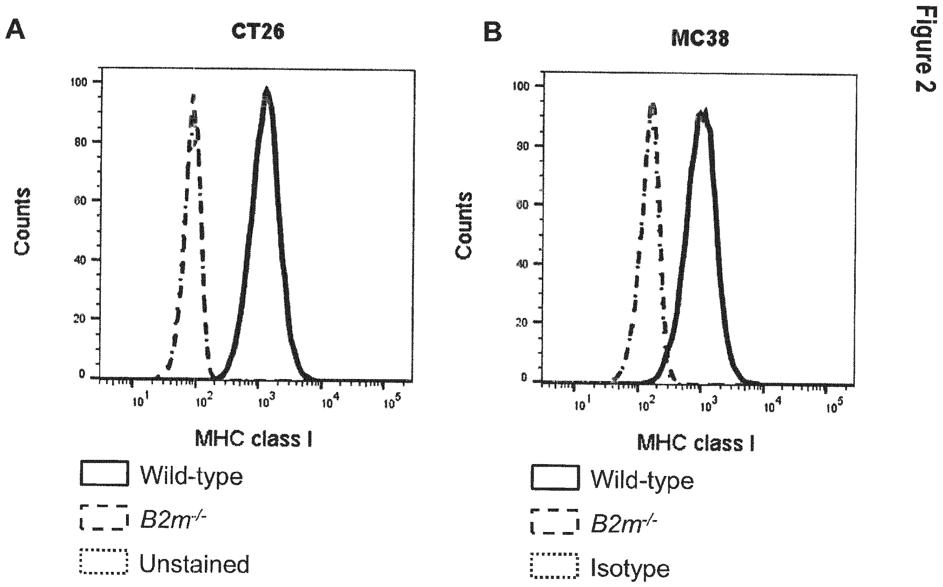

**Figure 1****: Tumor infiltration by CD8⁺ T cells and CD45⁺ immune cells in different murine tumor models.**
   Balb/c mice were inoculated subcutaneously (s.c.) with 5x10⁵ CT26 cells. C57BI/6 mice were inoculated subcutaneously (s.c.) with 5x10⁵ MC38 cells, 3x10⁵ B16F10 cells or 1x10⁵ TC1 cells. Proportion of intratumoral CD8⁺ T cells (A) and total immune cells (B) determined by flow cytometry 20 days after tumor inoculation. Dots represent individual mice, lines represent the group mean.
**Figure 2****: Loss of MHC class I surface expression by CT26 murine colon carcinoma and MC38 murine colon adenocarcinoma tumor cell lines after genetic knock-out of *B2m.***
   MHC class I surface expression of control and *B2m*^{-/-} CT26 **(A)** and MC38 cells **(B).** *B2m*^{-/-} clones generated by transient transfection with Cas9 mRNA and *B2m* targeting sgRNA. Expression of MHC class I on the cell surface determined by flow cytometry.
**Figure 3****: Loss of MHC class I surface expression by B16F10 murine melanoma and TC1 murine lung epithelial cancer tumor cell lines after genetic knock-out of *B2m.***
   MHC class I surface expression of control and *B2m*^{-/-} B16F10 **(A)** and TC1 cells **(B).** *B2m*^{-/-} clones generated as described in Figure 2. Expression of MHC class I on the cell surface determined by flow cytometry. B16F10 cells were treated with 25 ng/mL IFNy for 24 h prior to flow cytometry analysis.

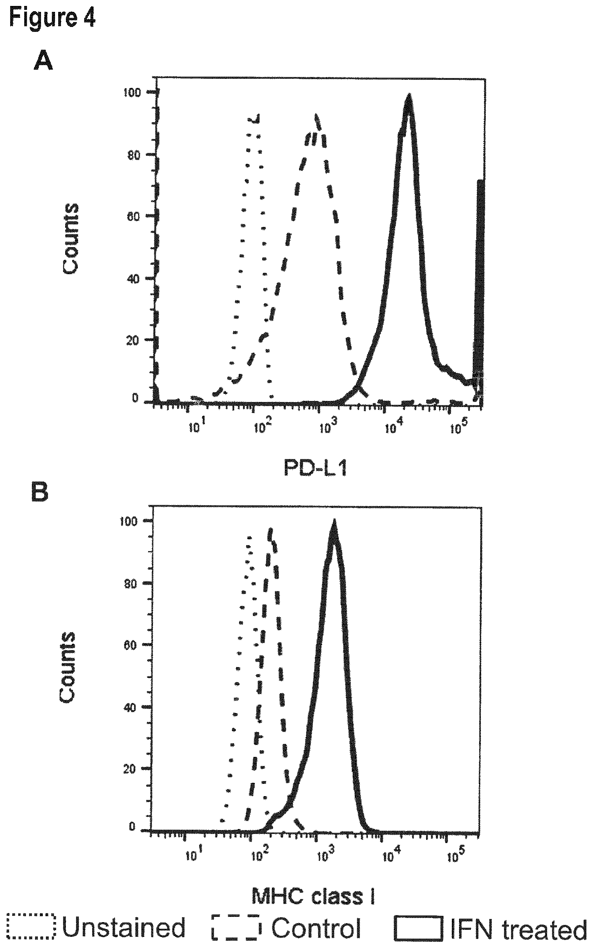

**Figure 4****: IFN response of B16F10 murine melanoma cells induces PD-L1 and MHC class I upregulation.**
   Expression of PD-L1 **(A)** and MHC class I **(B)** by B16F10 cells determined by flow cytometry after 24 h stimulation with 1000 U/mL IFN-α. Control was unstimulated.

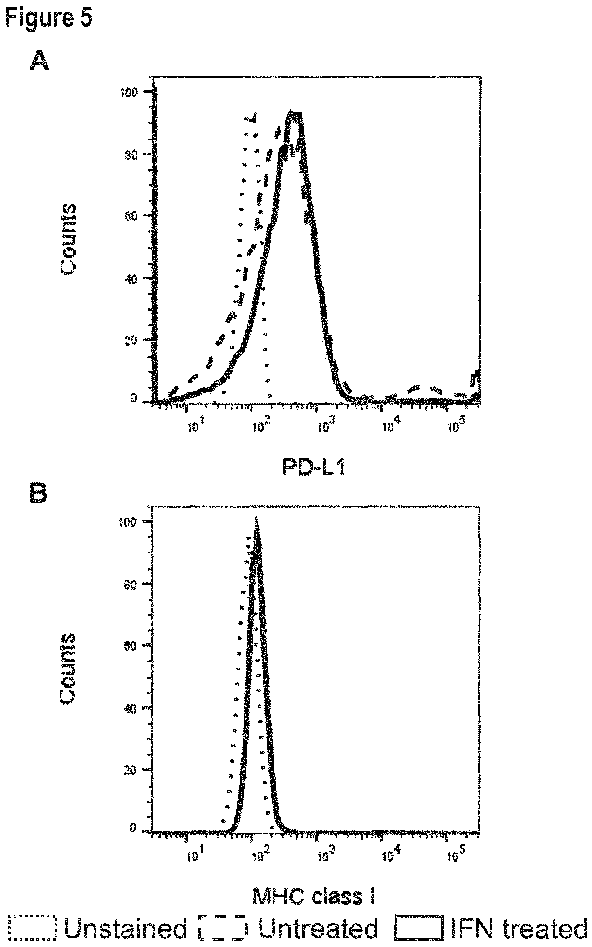

**Figure 5****: Impaired IFN response by B16F10 murine melanoma cells after genetic knock-out of *Jak1.***
   Expression of PD-L1 **(A)** and MHC class I **(B)** by B16F10-*Jak1*^{-/-} cells determined by flow cytometry after IFN stimulation as described in Figure 4. Control was unstimulated. B16F10-*Jak1*^{-/-} cells were generated as described in Figure 2 with sgRNA targeting *Jak1.*

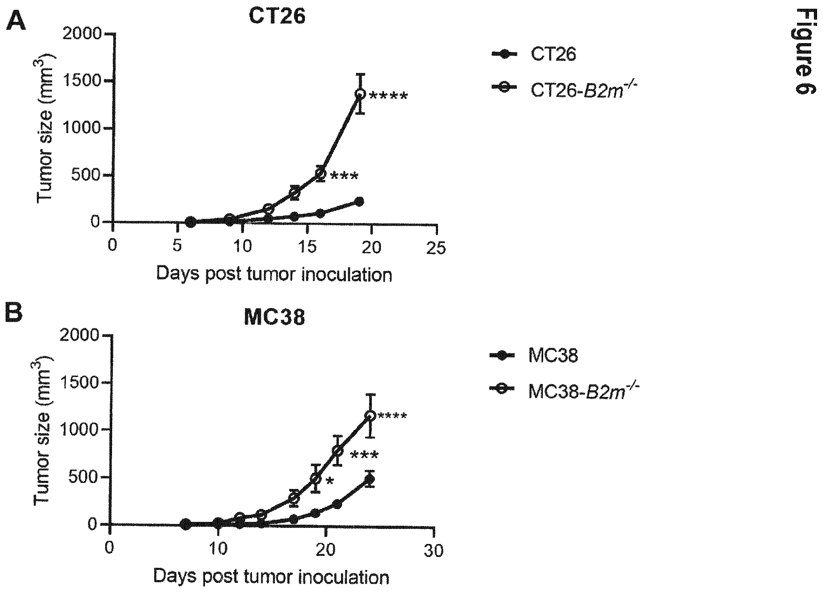

**Figure 6****: Growth comparison of parental CT26 murine colon carcinoma and MC38 murine colon adenocarcinoma tumors with *B2m*^{-/-} variants.**
   Tumor growth of Balb/c mice (n=5 per group) inoculated subcutaneously (s.c.) with 5x10⁵ CT26 or CT26-*B2m*^{-/-} cells **(A)** and C57Bl/6 mice (n=5) inoculated subcutaneously (s.c.) with 5x10⁵ MC38 or MC38-*B2m*^{-/-} cells **(B).** Data shown are group mean±SEM. Statistical significance was determined using two-way ANOVA followed by Sidak's multiple comparison test. All analyses were two-tailed and carried out using GraphPad Prism 8. ns P>0.05, *P≤0.05, **P<0.01, ***P<0.001, ****P<0.0001.

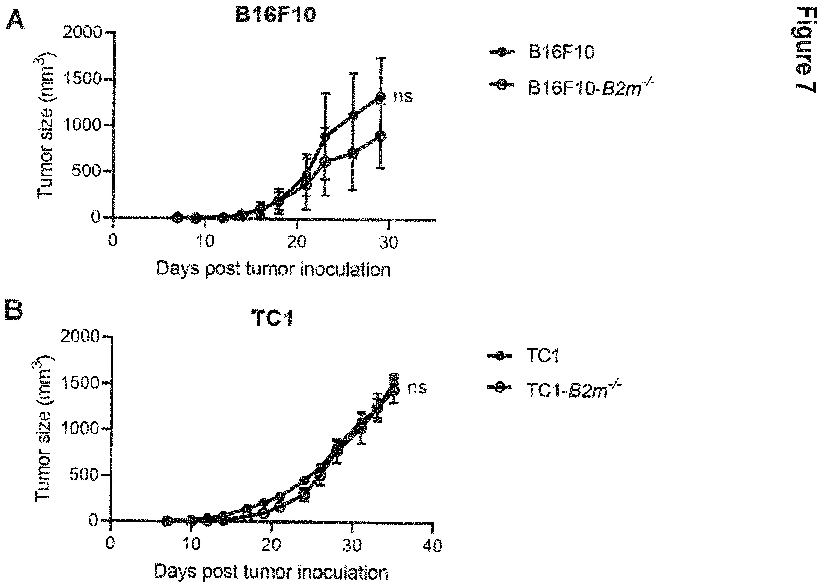

**Figure 7****: Growth comparison of parental B16F10 murine melanoma and TC1 murine lung epithelial tumors with *B2m*^{-/-} variants.**
   Tumor growth of C57BI/6 mice (n=10 per group) inoculated subcutaneously (s.c.) with 3x10⁵ B16F10 or B16F10-*B2m*^{-/-} cells **(A)** and C57Bl/6 mice (n=10) inoculated subcutaneously (s.c.) with 1x10⁵ TC1 or TC1-*B2m*^{-/-} cells **(B).** Data shown are group mean±SEM. Statistical significance was determined using two-way ANOVA followed by Sidak's multiple comparison test. All analyses were two-tailed and carried out using GraphPad Prism 8. ns P>0.05, *P≤0.05, **P<0.01, ***P<0.001, ****P<0.0001.

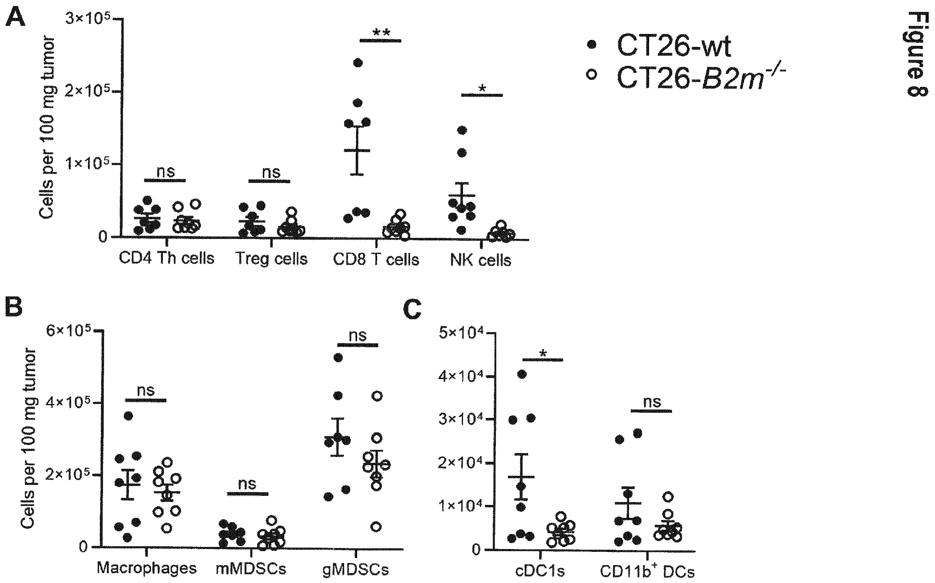

**Figure 8****: Comparison of the immune cell infiltration of CT26 and CT26-*B2m*^{-/-} murine colon carcinoma tumors.**
   Balb/c mice were inoculated subcutaneously (s.c.) with 5x10⁵ CT26 or CT26-*B2m*^{-/-} cells. Numbers of intratumoral lymphoid **(A),** myeloid **(B)** and dendritic cell (DC) subsets (C) determined by flow cytometry and normalized to the tumor weight 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

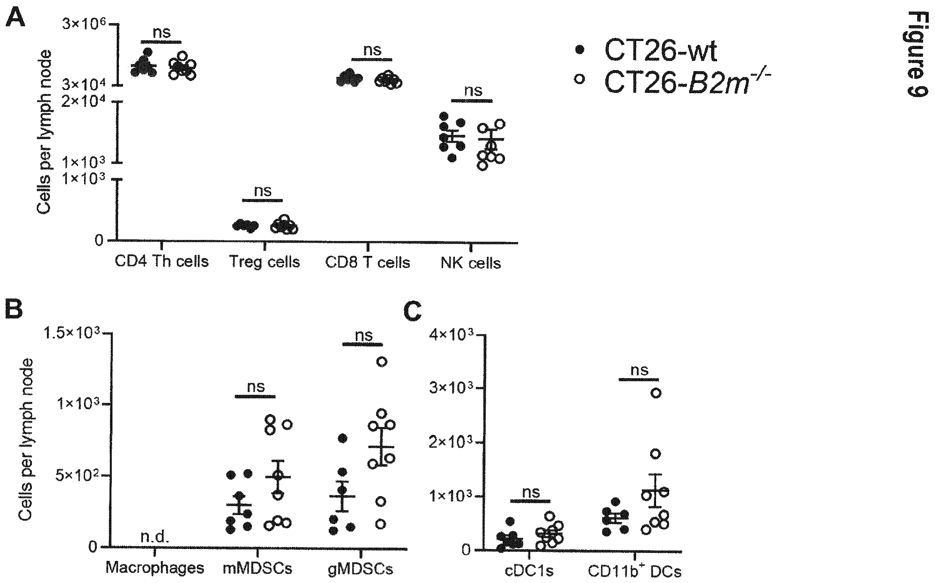

**Figure 9****: Comparison of the immune cell composition of tumor-draining lymph nodes taken from CT26 or CT26-*B2m*^{-/-} murine colon carcinoma tumor bearing mice.**
   Balb/c mice were inoculated subcutaneously (s.c.) with 5x10⁵ CT26 or CT26-*B2m*^{-/-} cells. Numbers of lymphoid **(A),** myeloid **(B)** and dendritic cell (DC) subsets **(C)** in the tumor-draining lymph nodes determined by flow cytometry 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

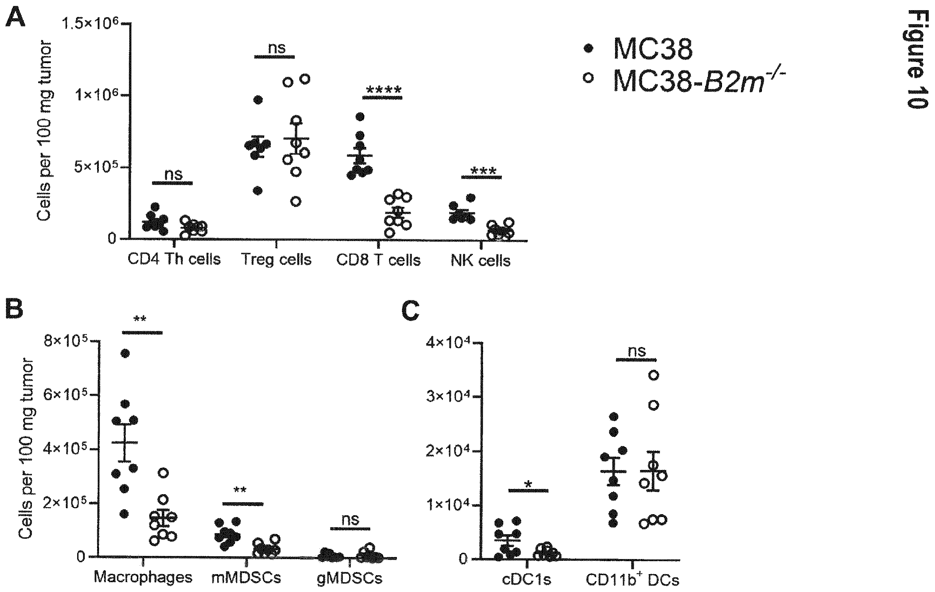

**Figure 10****: Comparison of the immune cell infiltration of MC38 and MC38-*B2m***^{-/-} **murine colon adenocarcinoma tumors.**
   C57BI/6 mice were inoculated subcutaneously (s.c.) with 5x10⁵ MC38 or MC38-*B2m*^{-/-} cells. Numbers of intratumoral lymphoid **(A),** myeloid **(B)** and dendritic cell (DC) subsets **(C)** determined by flow cytometry and normalized to the tumor weight 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

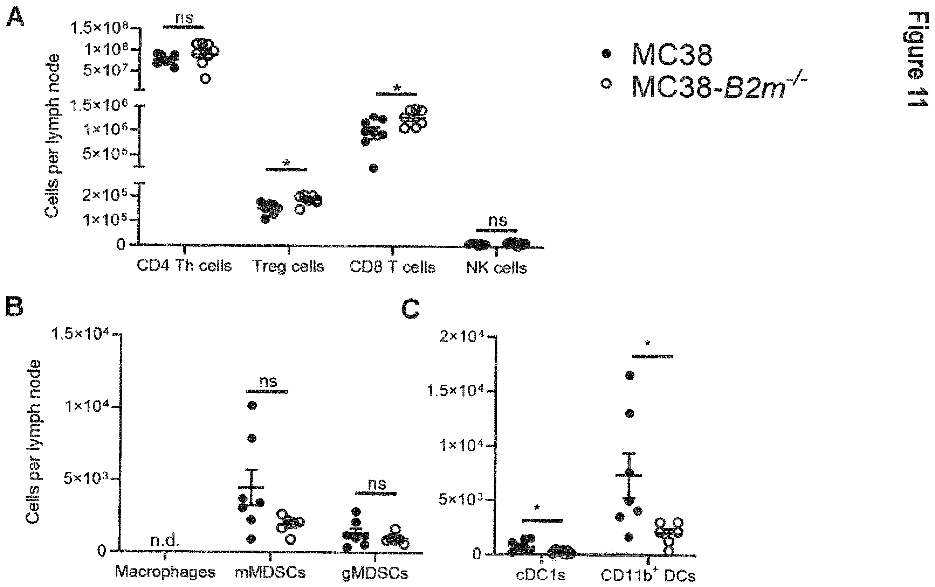

**Figure 11****: Comparison of the immune cell composition of tumor-draining lymph nodes taken from MC38 or MC38.*B2m*^{-/-} murine colon adenocarcinoma tumor bearing mice.**
   C57BI/6 mice were inoculated subcutaneously (s.c.) with 5x10⁵ MC38 or MC38-*B2m*^{-/-} cells. Numbers of lymphoid **(A),** myeloid **(B)** and dendritic cell (DC) subsets **(C)** in the tumor-draining lymph nodes determined by flow cytometry 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

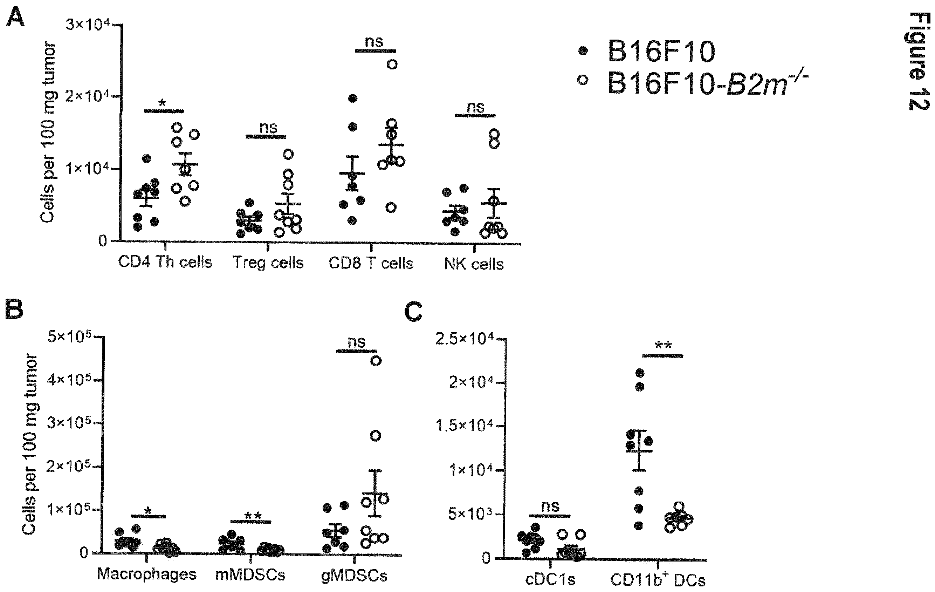

**Figure 12****: Comparison of the immune cell infiltration of B16F10 and B16F10-*B2m*^{-/-} murine melanoma tumors.**
   C57BI/6 mice were inoculated subcutaneously (s.c.) with 3x10⁵ B16F10 or B16F10-*B2m*^{*-*/*-*} cells. Numbers of intratumoral lymphoid **(A),** myeloid **(B)** and dendritic cell (DC) subsets **(C)** determined by flow cytometry and normalized to the tumor weight 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

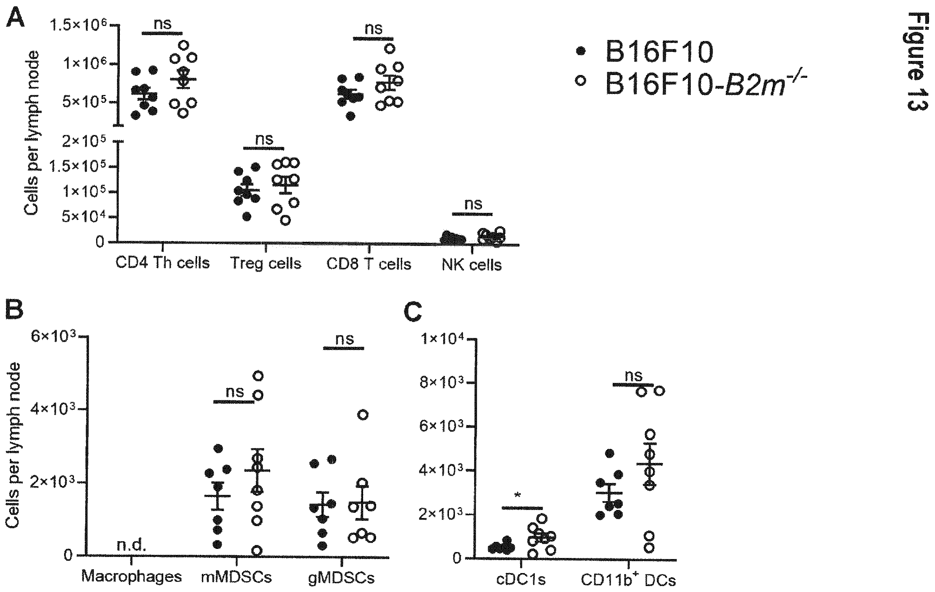

**Figure 13****: Comparison of the immune cell composition of tumor-draining lymph nodes taken from B16F10 and B16F10-*B2m***^{-/-} **murine melanoma tumor bearing mice.**
   C57BI/6 mice were inoculated subcutaneously (s.c.) with 3x10⁵ B16F10 or B16F10-*B2m*^{-/-} cells. Numbers of lymphoid **(A),** myeloid **(B)** and dendritic cell (DC) subsets **(C)** in the tumor-draining lymph nodes determined by flow cytometry 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

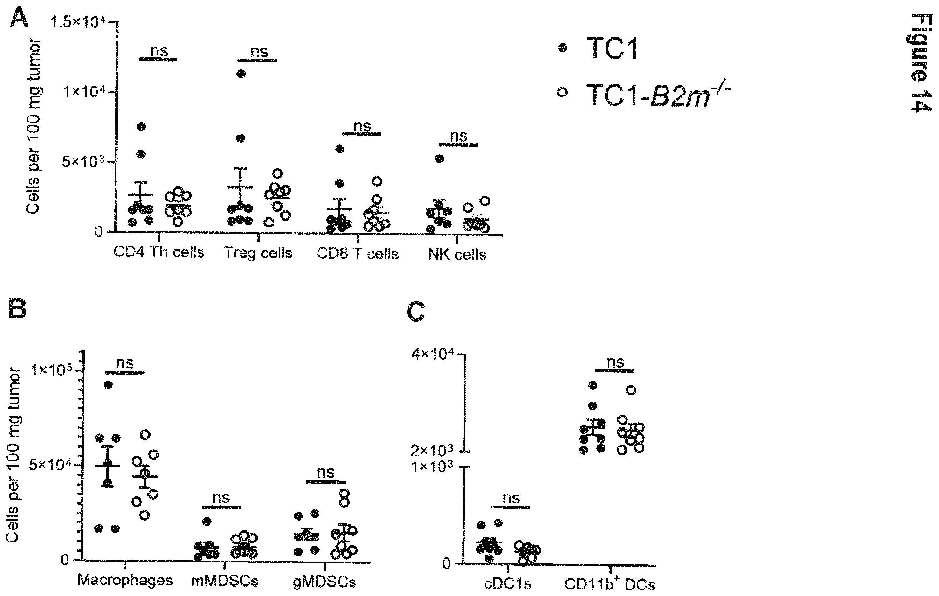

**Figure 14****: Comparison of the immune cell infiltration of TC1 and TC1-*B2m*^{-/-} murine lung epithelial tumors.**
   C57BI/6 mice were inoculated subcutaneously (s.c.) with 1x10⁵ TC1 or TC1-*B2m*^{-/-} cells. Numbers of intratumoral lymphoid **(A),** myeloid **(B)** and dendritic cell (DC) subsets **(C)** determined by flow cytometry and normalized to the tumor weight 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

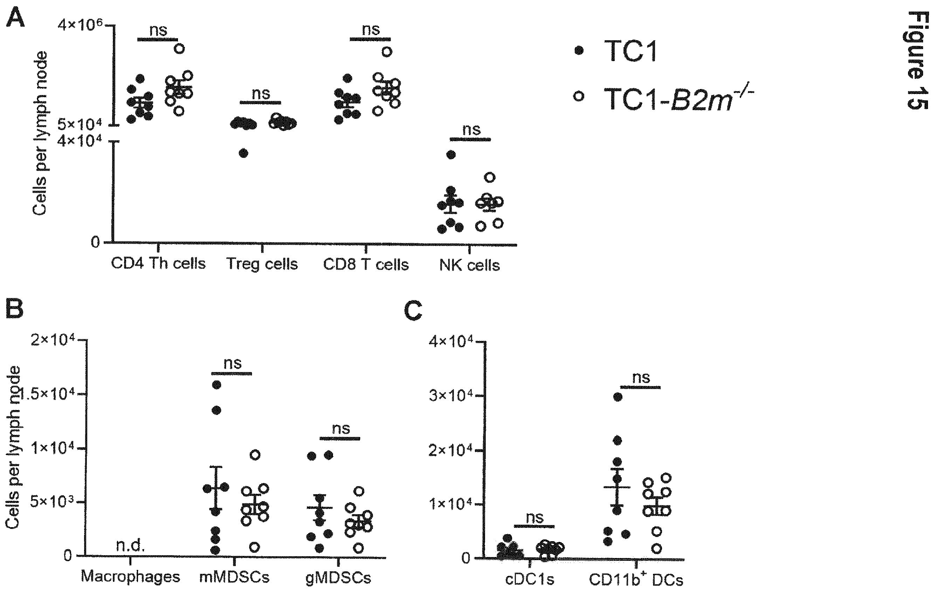

**Figure 15****: Comparison of the immune cell composition of tumor-draining lymph nodes taken from TC1 and TC1-*B2m*^{-/-} murine lung epithelial tumor bearing mice.**
   C57BI/6 mice were inoculated subcutaneously (s.c.) with 1x10⁵ TC1 or TC1-*B2m*^{-/-} cells. Numbers of lymphoid **(A),** myeloid **(B)** and dendritic cell (DC) subsets **(C)** in the tumor-draining lymph nodes determined by flow cytometry 20 days after tumor inoculation. Dots represent individual mice and lines represent group mean±SEM. Outliers were removed using Grubb's test. Statistical significance was determined using Student's unpaired t-test. All analyses were two-tailed and carried out using GraphPad Prism 8. ns P>0.05, *P≤0.05, **P<0.01, ***P<0.001, ****P<0.0001.

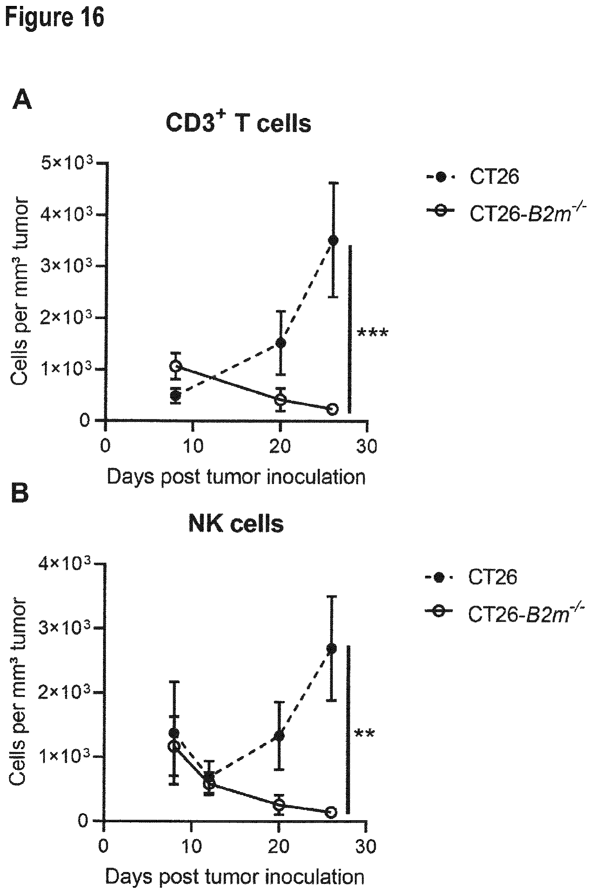

**Figure 16****: Differential infiltration of CT26 and CT26-*B2m*^{-/-} murine colon carcinoma tumors by T cells and NK cells on different time points.**
   Balb/c mice (n=5 per group and per time point) were inoculated subcutaneously (s.c.) with 5x10⁵ CT26 or CT26-*B2m*^{-/-} cells. Numbers of intratumoral CD3⁺ T cells **(A)** and NK cells **(B)** determined by flow cytometry on day 8, 12 (NK cells only), 20 and 26 after tumor inoculation and normalized to the tumor volume. Data shown are group mean±SEM. Statistical significance was determined using Mixed-effects analysis followed by Sidak's multiple comparison test. All analyses were two-tailed and carried out using GraphPad Prism 8. ns P>0.05, *P≤0.05, **P<0.01, ***P<0.001, ****P<0.0001.

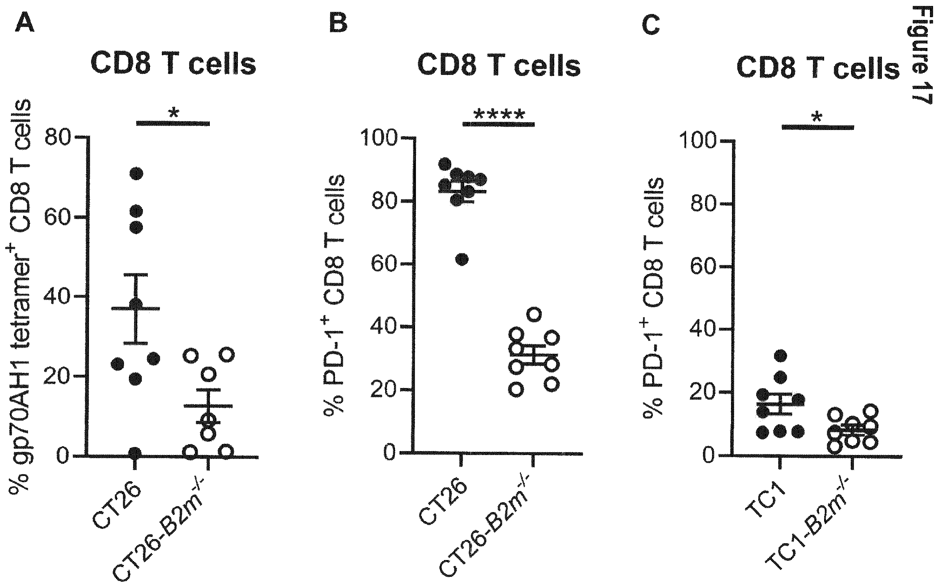

**Figure 17****: Different phenotype of CD8⁺ T cells intratumoral CT26 and CT26-*B2m*^{-/-} murine colon carcinoma tumors or TC1 and TC1-*B2m*^{-/-} murine lung epithelial tumors.**
   Balb/c mice were inoculated subcutaneously (s.c.) with 5x10⁵ CT26 or CT26-*B2m*^{-/-} cells. C57BI/6 were inoculated subcutaneously (s.c.) with 1x10⁵ TC1 or TC1-*B2m*^{-/-} cells. Frequency of intratumoral gp70-specific **(A)** and PD-1⁺ CD8⁺ T cells **(B)** from CT26 and CT26-*B2m*^{-/-} tumors and frequency of intratumoral PD-1⁺ CD8⁺ T cells **(C)** from TC1 and TC1-*B2m*^{-/-} tumors determined by flow cytometry 20 days after tumor inoculation. Dots represent individual mice and lines represent group mean±SEM. Statistical significance was determined using Student's unpaired t-test. All analyses were two-tailed and carried out using GraphPad Prism 8. ns P>0.05, *P≤0.05, **P<0.01, ***P<0.001, ****P<0.0001.

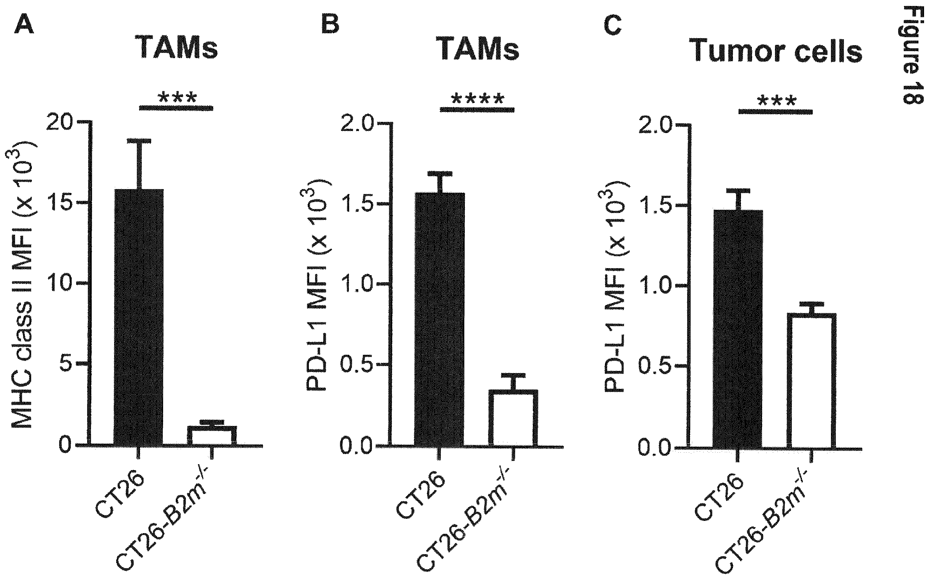

**Figure 18****: Different phenotype of intratumoral macrophages (TAMs) and tumor cells from CT26** and **CT26-*B2m*^{-/-} murine colon carcinoma tumors.**
   Balb/c mice (n=8 per group) were inoculated subcutaneously (s.c.) with 5x10⁵ CT26 or CT26-*B2m*^{-/-} cells. MHC class II **(A)** and PD-L1 expression **(B)** by intratumoral TAMs and PD-L1 expression by tumor cells **(C)** determined by flow cytometry 20 days after tumor inoculation. Data are mean+SEM. Statistical significance was determined using Student's unpaired t-test. All analyses were two-tailed and carried out using GraphPad Prism 8. ns P>0.05, *P≤0.05, **P<0.01, ***P<0.001, ****P<0.0001.

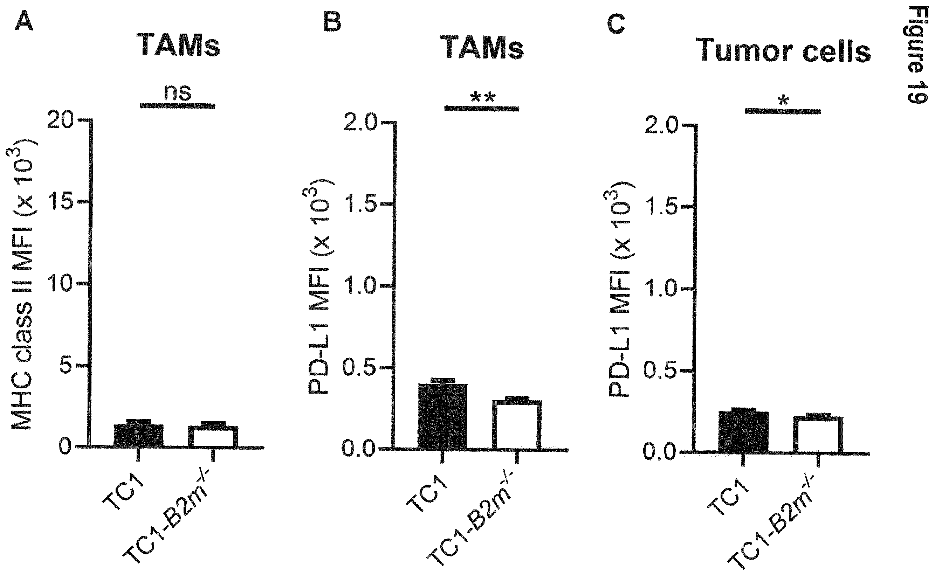

**Figure 19****: Different phenotype of intratumoral macrophages (TAMs) and tumor cells from TC1 and TC1-*B2m*^{-/-} murine lung epithelial tumors.**
   C57BI/6 (n=8 per group) were inoculated subcutaneously (s.c.) with 1x10⁵ TC1 or TC1-*B2m*^{-/-} cells. MHC class II **(A)** and PD-L1 expression **(B)** by intratumoral TAMs and PD-L1 expression by tumor cells **(C)** determined as described in Figure 18. Data are mean+SEM. Statistical significance was determined using Student's unpaired t-test. All analyses were two-tailed and carried out using GraphPad Prism 8. ns P>0.05, *P≤0.05, **P<0.01, ***P<0.001, ****P<0.0001.

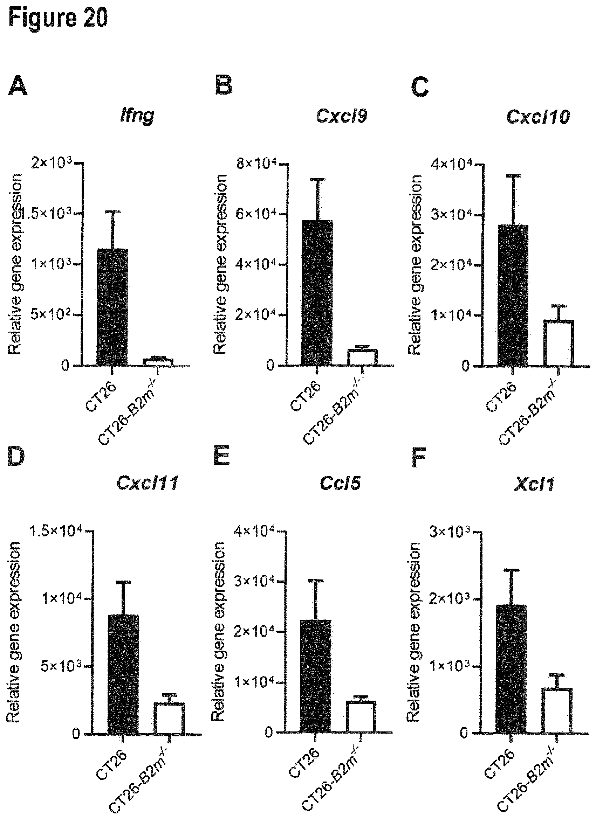

**Figure 20****: Different expression levels of *Ifng* and chemokines in the TME of CT26 and CT26-*B2m***^{**-** /-} **murine colon carcinoma tumors.**
   Balb/c mice were inoculated subcutaneously (s.c.) with 5x10⁵ CT26 or CT26-*B2m*^{-/-} cells. Intratumoral relative gene expression levels of *Ifng **(A),** Cxcl9* **(B),** Cxcl10 **(C),** *Cxcl11* **(D),** *Ccl5* **(E)** and *Xcl1* **(F)** determined by qRT-PCR 19 days after tumor inoculation. Data are mean+SEM of n=3 mice per group.

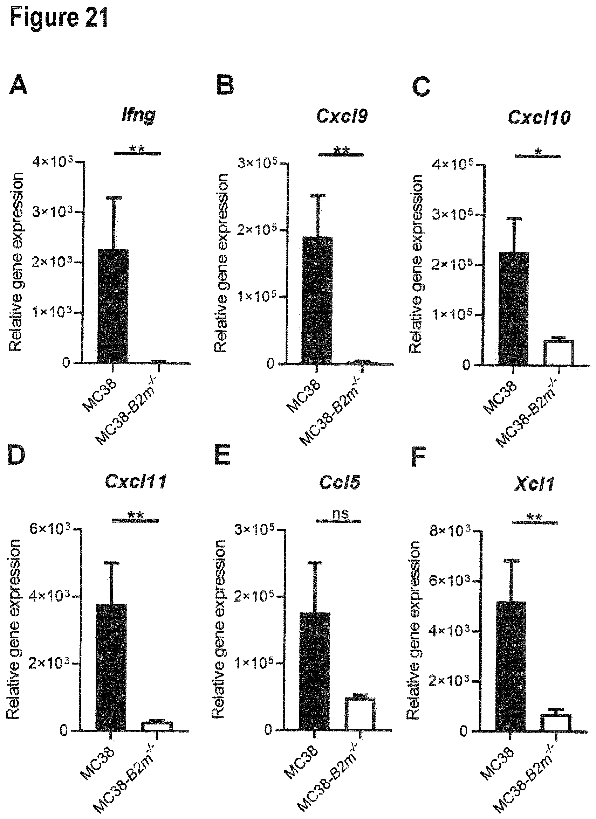

**Figure 21****: Different expression levels of *Ifng* and chemokines in the TME of MC38 and MC38-*B2m***^{**-**/-} **murine colon adenocarcinoma tumors.**
   C57BI/6 mice were inoculated subcutaneously (s.c.) with 5x10⁵ MC38 or MC38-*B2m*^{-/-} cells. Intratumoral relative gene expression levels of *Ifng* (**A**)*, Cxcl9* (**B**), *Cxcl10 (C), Cxcl11* (**D**), *Ccl5* **(E)** and *Xcl1* **(F)** determined as described in Figure 20. Data are mean+SEM of n=5 mice per group. Statistical significance was determined using Mann-Whitney-U-test. All analyses were two-tailed and carried out using GraphPad Prism 8. ns P>0.05, *P≤0.05, **P<0.01, ***P<0.001, ****P<0.0001.

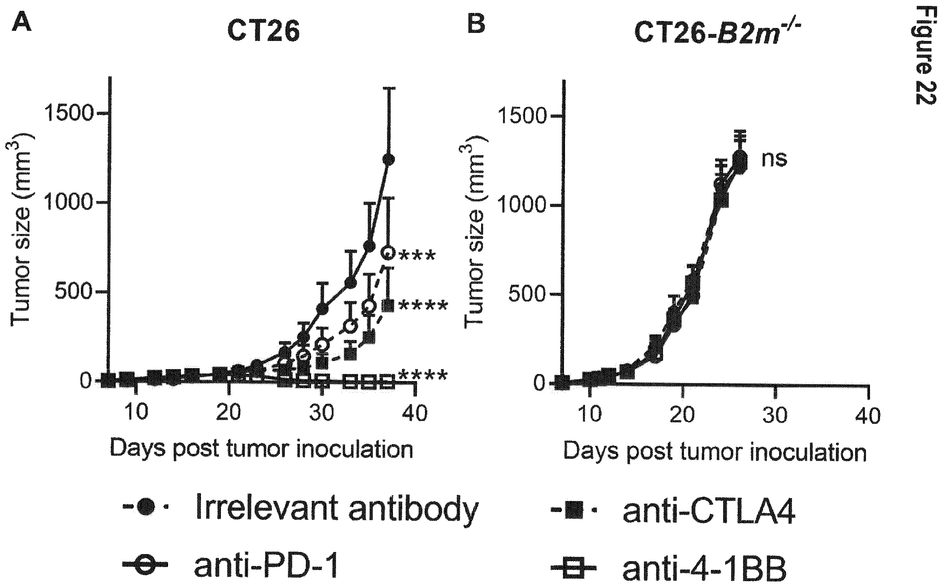

**Figure 22****: B2m deficiency leads to resistance against anti-PD-1, anti-CTLA4 and anti-4-1BB antibody therapy in the CT26 murine colon carcinoma model.**
   Balb/c mice (n=10 per group) were inoculated subcutaneously (s.c.) with 5x10⁵ CT26 or CT26-*B2m*^{-/-}cells. Mice were injected intraperitoneally (i.p.) with 200 µg antibody targeting PD-1, CTLA4 or 4-1BB on day 0, 3, 7 and 10 after the tumors reached a mean volume of 32-36 mm³. Control group injected with an antibody binding to an irrelevant antigen. Tumor growth of CT26 **(A)** or CT26-*B2m*^{-/-} **(B)** tumor bearing mice. Data shown are mean+SEM. Statistical significance was determined using two-way ANOVA followed by Dunnet's multiple comparison test and is shown for the last depicted time point. All analyses were two-tailed and carried out using GraphPad Prism 8. ns P>0.05, *P≤0.05, **P<0.01, ***P<0.001, ****P<0.0001.

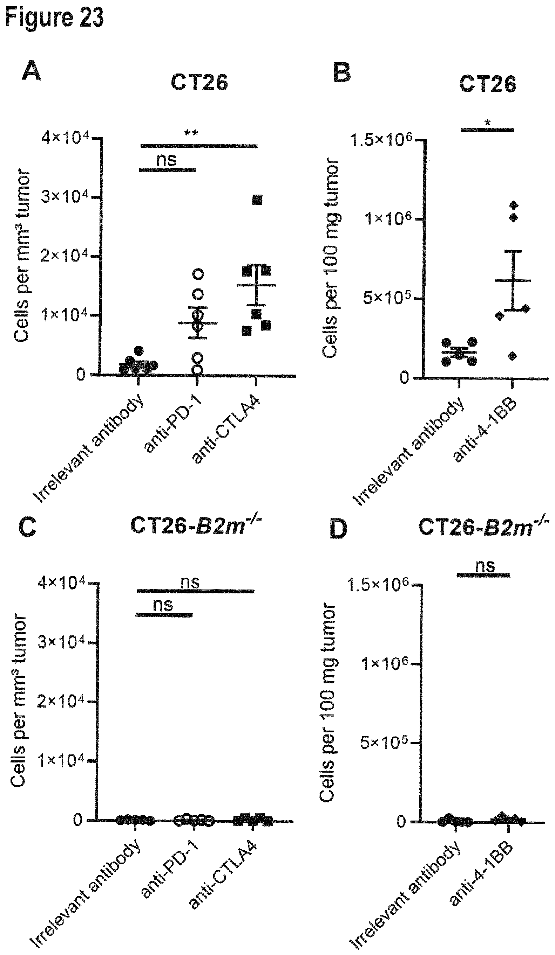

**Figure 23****: Differential infiltration of CD8⁺ T cells in CT26 and CT26-*B2m*^{-/-} murine colon carcinoma tumors after treatment with anti-PD-1, anti-CTLA4 and anti-4-1BB antibody therapy.**
   Balb/c mice were inoculated subcutaneously (s.c.) with 5x10⁵ CT26 or CT26-*B2m*^{-/-} cells and treated as described in Figure 22. Numbers of intratumoral CD8⁺ T cells from CT26 tumors treated with anti-PD-1 or anti-CTLA4 **(A)** or anti-4-1BB **(B)** and CT26-*B2m*^{-/-} tumors treated with anti-PD-1 or anti-CTLA4 **(C)** or anti-4-1BB **(D)** determined by flow cytometry and normalized to the tumor volume or the tumor weight. Tumors treated with anti-4-1BB antibody analyzed 7 days after the first treatment and tumors treated with anti-PD-1 or anti-CTLA4 antibody analyzed on 10 days after the first treatment. Dots represent individual mice and lines represent group mean±SEM. Statistical significance was determined using one-way ANOVA followed by Dunnet's multiple comparison test **(A** and **C)** or Student's unpaired *t*-test **(B** and **D).** All analyses were two-tailed and carried out using GraphPad Prism 8. ns P>0.05, *P≤0.05, **P<0.01, ***P<0.001, ****P<0.0001.

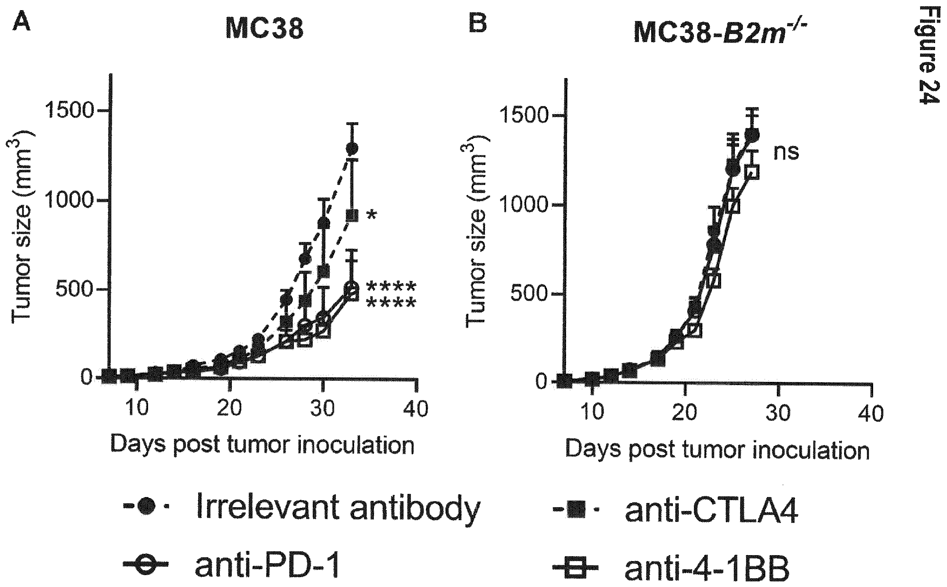

**Figure 24****: B2m deficiency leads to resistance against anti-PD-1, anti-CTLA4 and anti-4-1BB antibody therapy in the MC38 murine colon adenocarcinoma model.**
   C57BI/6 mice (n=10 per group) were inoculated subcutaneously (s.c.) with 5x10⁵ MC38 or MC38-*B2m*^{-/-} cells. Mice were treated with antibodies as described in Figure 22 on days 0, 4, 7 and 10. MC38 bearing mice received two additional injections on day 14 and 17. Tumor growth of MC38 **(A)** or MC38-*B2m*^{-/-} **(B)** tumor bearing mice. Data shown are mean+SEM. Statistical significance was determined using two-way ANOVA followed by Dunnet's multiple comparison test and is shown for the last depicted time point. All analyses were two-tailed and carried out using GraphPad Prism 8. ns P>0.05, *P≤0.05, **P<0.01, ***P<0.001, ****P<0.0001.

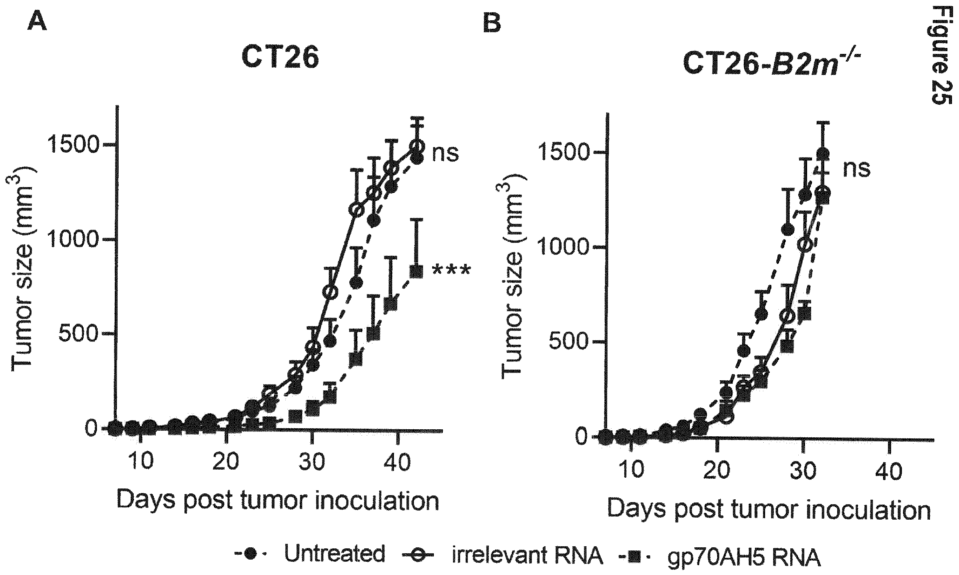

**Figure 25****: *B2m* deficiency leads to resistance against therapeutic RNA vaccination in the CT26 murine colon carcinoma model.**
   Balb/c mice (n=10 per group) were inoculated subcutaneously (s.c.) with 5x10⁵ CT26 or CT26-*B2m*^{-/-} cells. Mice were injected intravenously (i.v.) with 40 µg gp70AH5 RNA-LPX or RNA-LPX not coding for any antigen (irrelevant RNA) on day 5, 8, 12 and 19 after tumor inoculation. The control group was untreated. Tumor growth of CT26 **(A)** or CT26-*B2m*^{-/-} **(B)** tumor bearing mice. Data shown are mean+SEM. Statistical significance was determined using two-way ANOVA followed by Dunnet's multiple comparison test and is shown for the last depicted time point. All analyses were two-tailed and carried out using GraphPad Prism 8. ns P>0.05, *P≤0.05, **P<0.01, ***P<0.001, ****P<0.0001.

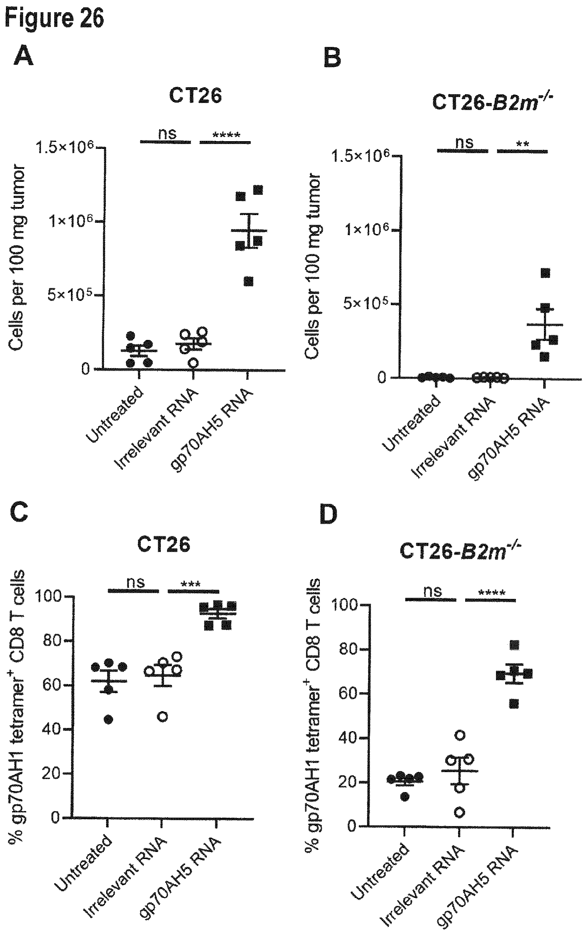

**Figure 26****: Differential infiltration and antigen specificity of CD8⁺ T cells in CT26 and CT26-*B2m*^{-/-} murine colon carcinoma tumors after therapeutic RNA vaccination.**
   Balb/c mice were inoculated subcutaneously (s.c.) with 5x10⁵ CT26 or CT26-*B2m*^{-/-} cells and treated as described in Figure 25. Numbers of intratumoral CD8⁺ T cells from CT26 **(A)** and CT26-*B2m*^{-/-} **(B)** tumors determined by flow cytometry 17 days after the first treatment and normalized to the tumor weight. Frequency of gp70-specific CD8⁺ T cells from CT26 **(C)** and CT26-*B2m*^{-/-} **(D)** tumors. Dots represent individual mice and lines represent group mean±SEM. Statistical significance was determined using one-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

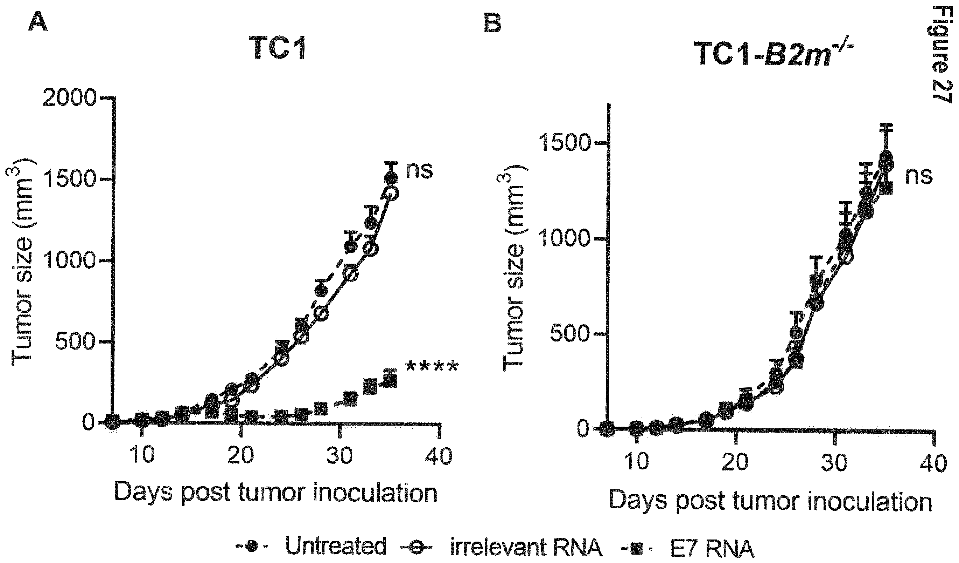

**Figure 27****: *B2m* deficiency leads to resistance against therapeutic RNA vaccination in the TC1 murine lung epithelial tumor model.**
   C57BI/6 mice (n=10 per group) were inoculated subcutaneously (s.c.) with 1x10⁵ TC1 or TC1-*B2m*^{-/-} cells. Mice were injected intravenously (i.v.) with 20 µg E7 RNA-LPX or RNA-LPX not coding for any antigen (irrelevant RNA) on day 0 and 7 after the tumors reached a mean volume of approximately 20 mm³. The control group was untreated. Tumor growth of TC1 **(A)** or TC1-*B2m*^{-/-} **(B)** tumor bearing mice. Data shown are mean+SEM. Statistical significance was determined using two-way ANOVA followed by Dunnet's multiple comparison test and is shown for the last depicted time point. All analyses were two-tailed and carried out using GraphPad Prism 8. ns P>0.05, *P≤0.05, **P<0.01, ***P<0.001, ****P<0.0001.

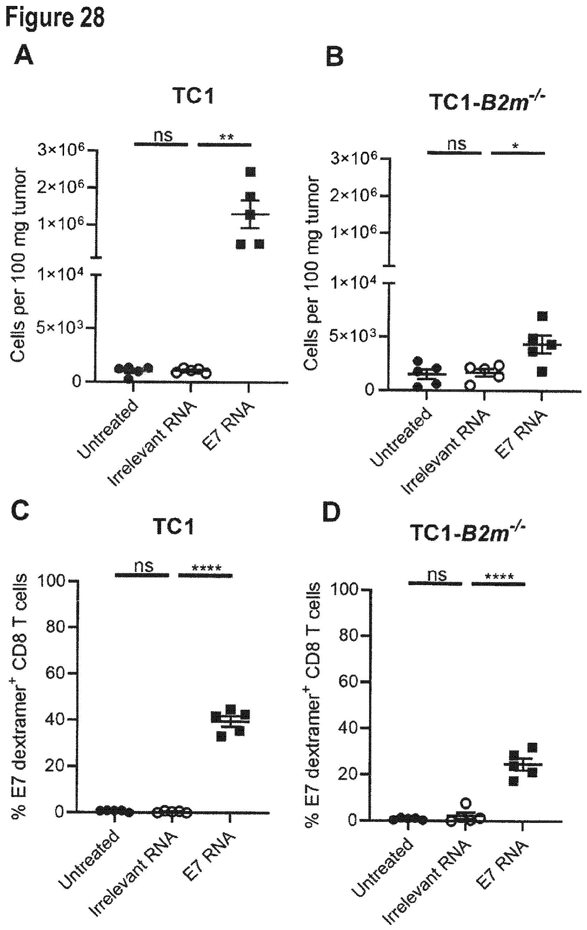

**Figure 28****: Differential infiltration and antigen specificity of CD8⁺ T cells in TC1 and TC1-*B2m***^{-/-} **murine lung epithelial tumors after therapeutic RNA vaccination.**
   C57BI/6 mice (n=10 per group) were inoculated subcutaneously (s.c.) with 1x10⁵ TC1 or TC1-*B2m*^{-/-} cells and treated as described in Figure 27. Numbers of intratumoral CD8⁺ T cells from TC1 **(A)** and TC1-*B2m*^{- /-} **(B)** determined by flow cytometry 9 days after the first treatment and normalized to the tumor weight. Frequency of E7-specific CD8⁺ T cells from TC1 **(C)** and TC1-*B2m*^{-/-} **(D)** tumors. Dots represent individual mice and lines represent group mean±SEM. Statistical significance was determined using one-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

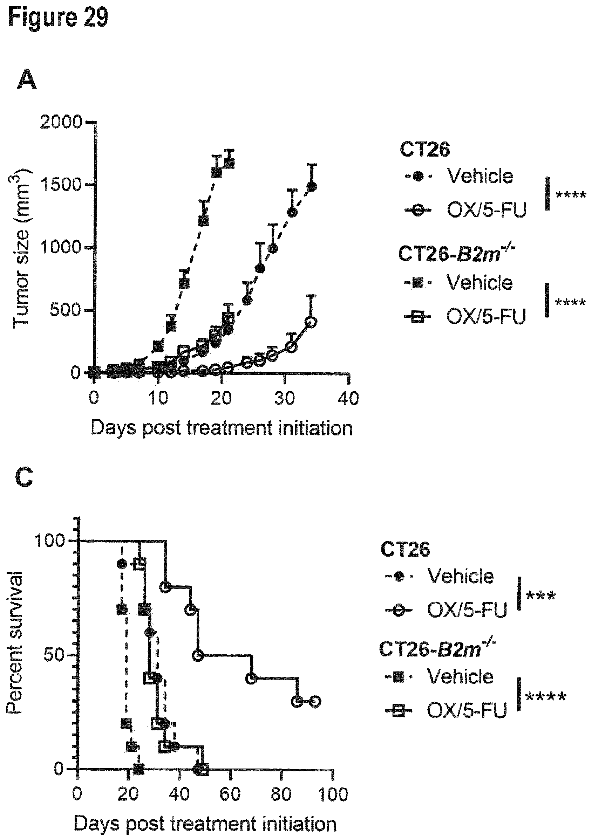

**Figure 29****: *B2m* deficiency prevents tumor rejection after treatment with chemotherapy in the CT26 murine colon carcinoma model.**
   Balb/c mice (n=9-10 per group) were inoculated subcutaneously (s.c.) with 5x10⁵ CT26 or CT26-*B2m*^{-/-} cells. Mice were injected intraperitoneally (i.p.) with 5 mg/kg Oxaliplatin (OX) and intravenously (i.v.) with 60 mg/kg 5-fluorouracil (5-FU) on day 0, 7 and 14 after the tumors reached a mean volume of approximately 6 mm³. The control groups received vehicle. Tumor growth curves shown as mean+SEM **(A)** and survival **(B)** of tumor bearing mice. Statistical significance was determined using two-way ANOVA followed by Sidak's multiple comparison test and is shown for the last depicted time point. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

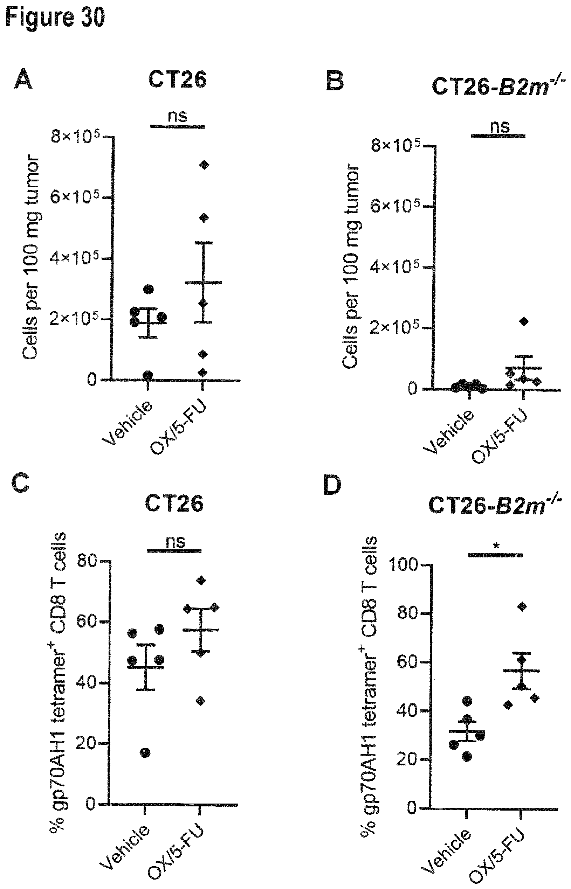

**Figure 30****: Differential infiltration and antigen specificity of CD8⁺ T cells in CT26 and CT26-*B2m*^{-/-} murine colon carcinoma tumors after treatment with chemotherapy.**
   Balb/c mice were inoculated subcutaneously (s.c.) with 5x10⁵ CT26 or CT26-*B2m*^{-/-} cells and treated as described in Figure 29. Numbers of intratumoral CD8⁺ T cell from CT26 **(A)** and CT26-*B2m*^{-/-} **(B)** tumors determined by flow cytometry 13 days after the first treatment. Frequency of gp70-specific CD8⁺ T cells from CT26 **(C)** and CT26-*B2m*^{-/-} **(D)** tumors. Dots represent individual mice and lines represent group mean±SEM. Statistical significance was determined using Student's unpaired t-test. All analyses were two-tailed and carried out using GraphPad Prism 8. ns P>0.05, *P≤0.05, **P<0.01, ***P<0.001, ****P<0.0001.

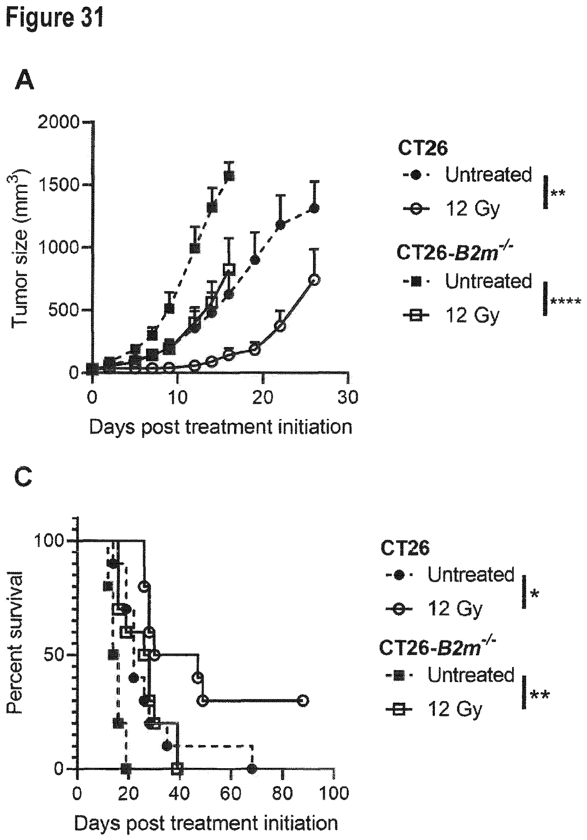

**Figure 31****: *B2m* deficiency prevents tumor rejection after treatment with radiotherapy in the CT26 murine colon carcinoma model.**
   Balb/c mice (n=10 per group) were inoculated subcutaneously (s.c.) with 5x10⁵ CT26 or CT26-*B2m*^{-/-} cells. Tumors were locally irradiated with 12 Gy after they reached a mean volume of approximately 30 mm³. The control groups were untreated. Tumor growth curves shown as mean+SEM **(A)** and survival **(B)** of tumor bearing mice. Statistical significance was determined using two-way ANOVA followed by Sidak's multiple comparison test and is shown for the last depicted time point. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

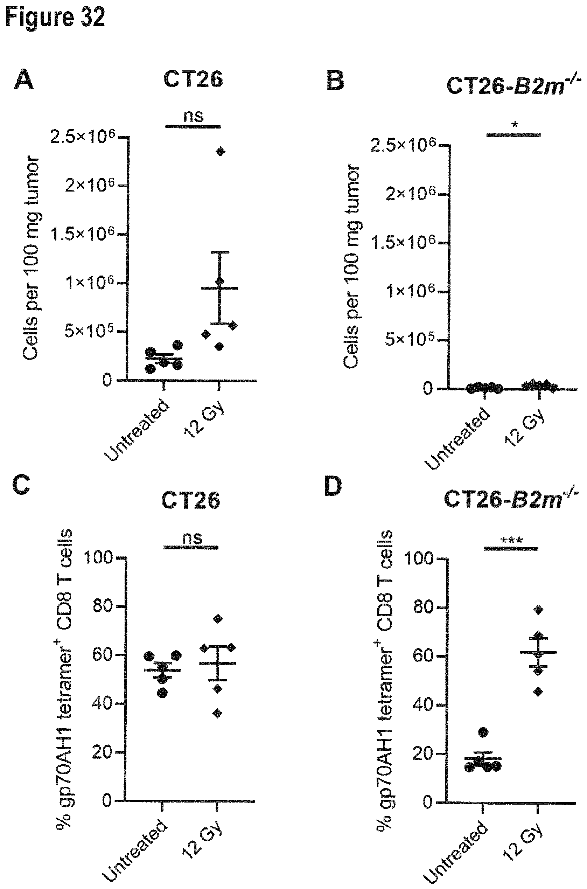

**Figure 32****: Differential infiltration and antigen specificity of CD8⁺ T cells in CT26 and CT26-*B2m*^{-/-} murine colon carcinoma tumors after treatment with radiotherapy.**
   Balb/c mice were inoculated subcutaneously (s.c.) with 5x10⁵ CT26 or CT26-*B2m*^{-/-} cells and treated as described in Figure 31. Numbers of intratumoral CD8⁺ T cell from CT26 **(A)** and CT26-*B2m*^{-/-} **(B)** tumors determined by flow cytometry 8 days after the treatment. Frequency of gp70-specific CD8⁺ T cells from CT26 **(C)** and CT26-*B2m*^{-/-} **(D)** tumors. Dots represent individual mice and lines represent group mean±SEM. Statistical significance was determined using Student's unpaired t-test. All analyses were two-tailed and carried out using GraphPad Prism 8. ns P>0.05, *P≤0.05, **P<0.01, ***P<0.001, ****P<0.0001.

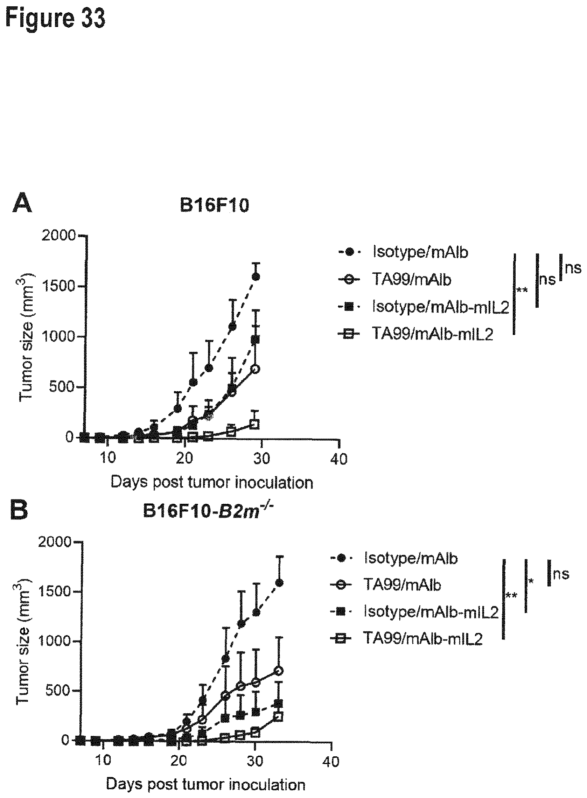

**Figure 33****: anti-Trp1 antibody and mAIb-mIL2 RNA combination therapy reduces the growth of B16F10 and B16F10-*B2m***^{-/-} **murine melanoma tumors.**
   C57BI/6 mice (n=9-10 per group) were inoculated subcutaneously (s.c.) with 3x10⁵ B16F10 or B16F10-*B2m^{-l-}* cells. Mice were injected intraperitoneally (i.p.) with 200 µg anti-Trp1 antibody (TA99) or isotype control on day 5, 8, 12, 15, 19, 22 and 26 and intravenously (i.v.) with 1 µg RNA coding for mAlb-mlL2 or mAlb (not coding for any cytokine) formulated with TransIT^{®} on day 5, 12, 19 and 26 after tumor inoculation. The control group was treated with isotype and mAlb RNA (not coding for any cytokine). Tumor growth of B16F10 **(A)** or B16F10-*B2m*^{-/-} **(B)** tumor bearing mice. Data shown are mean+SEM. Statistical significance was determined using two-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

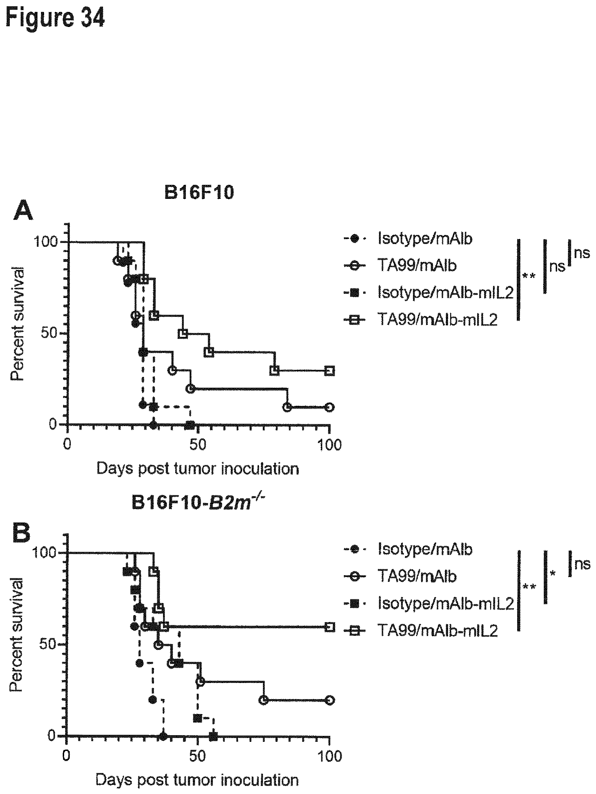

**Figure 34****: anti-Trp1 antibody and mAIb-mIL2 RNA combination therapy leads to long-term survival in the B16F10 and B16F10-*B2m***^{-/-} **murine melanoma models.**
   C57BI/6 mice (n=9-10 per group) were inoculated subcutaneously (s.c.) with 3x10⁵ B16F10 or B16F10-*B2m^{-l-}* cells and treated as described in Figure 33. Survival of B16F10 **(A)** or B16F10-*B2m*^{-/-} **(B)** tumor bearing mice. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

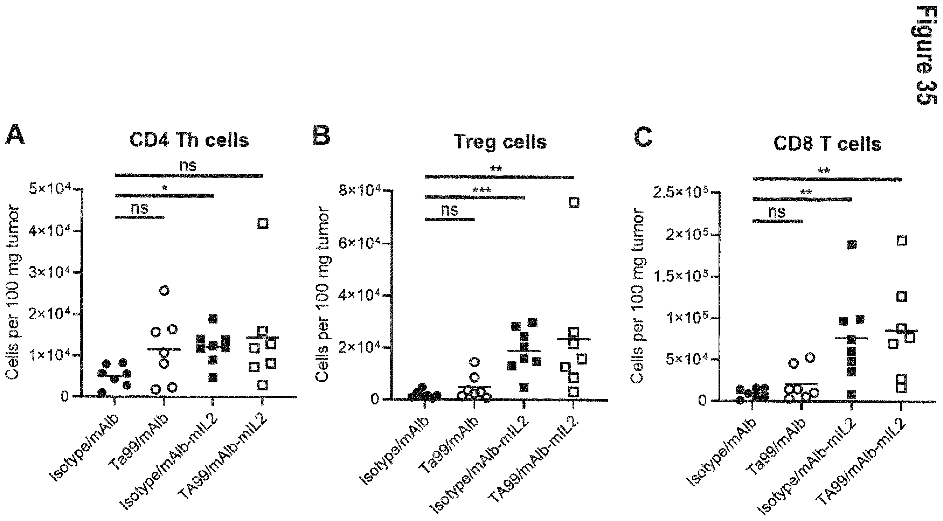

**Figure 35****: mAIb-mIL2 RNA alone or in combination with anti-Trp1 antibody increases the infiltration of CD4⁺ T cells and CD8⁺ T cells in the B16F10-*B2m***^{-/-} **murine melanoma model.** C57BI/6 mice were inoculated subcutaneously (s.c.) with 3x10⁵ B16F10-*B2m*^{-/-} cells and treatment as described in Figure 33 was initiated 9 days after tumor inoculation. Numbers of intratumoral CD4⁺ Th cells **(A),** Treg cells **(B)** and CD8⁺ T cells **(C)** determined by flow cytometry and normalized to the tumor weight 20 days after tumor inoculation. Dots represent individual mice, lines represent the group mean. Significant differences were determined using Kruskal-Wallis test followed by Dunn's multiple comparison test. All analyses were two-tailed and carried out using GraphPad Prism 8. ns P>0.05, *P≤0.05, **P<0.01, ***P<0.001, ****P<0.0001.

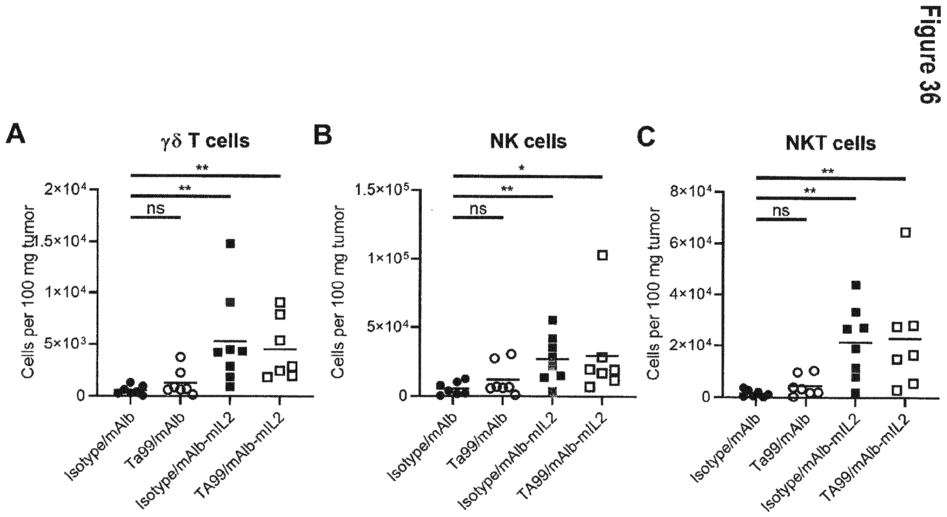

**Figure 36****: mAIb-mIL2 RNA alone or in combination with anti-Trp1 antibody increases the infiltration of γδ T cells, NK cells and NKT cells in the B16F10-*B2m***^{-/-} **murine melanoma model.** C57BI/6 mice were inoculated subcutaneously (s.c.) with 3x10⁵ B16F10-*B2m*^{-/-} cells and treatment as described in Figure 33 was initiated 9 days after tumor inoculation. Numbers of intratumoral δγ T cells **(A),** NK cells **(B)** and NKT cells **(C)** determined by flow cytometry and normalized to the tumor weight 20 days after tumor inoculation. Dots represent individual mice, lines represent the group mean. Significant differences were determined using Kruskal-Wallis test followed by Dunn's multiple comparison test. All analyses were two-tailed and carried out using GraphPad Prism 8. ns P>0.05, *P≤0.05, **P<0.01, ***P<0.001, ****P<0.0001.

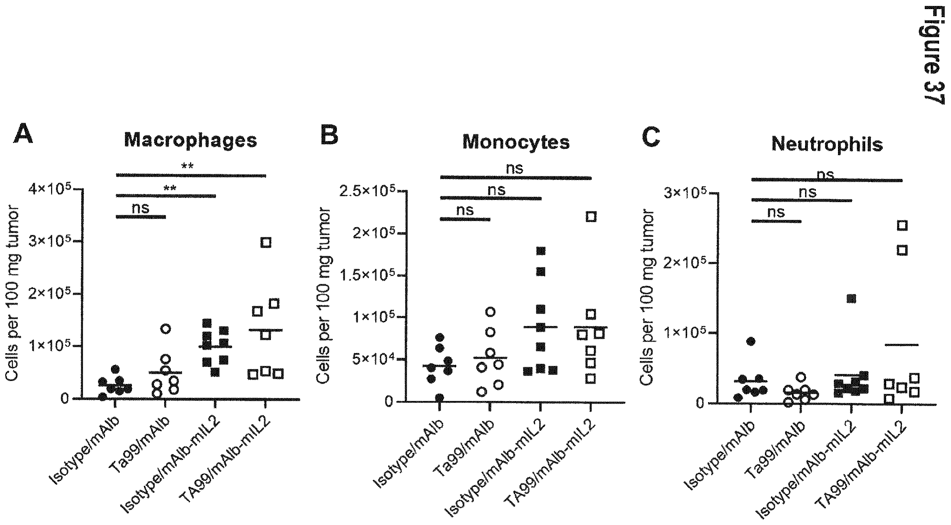

**Figure 37****: mAIb-mIL2 RNA alone or in combination with anti-Trp1 antibody increases the infiltration of macrophages in the B16F10-*B2m***^{-/-} **murine melanoma model.** C57BI/6 mice were inoculated subcutaneously (s.c.) with 3x10⁵ B16F10-*B2m*^{-/-} cells and treatment as described in Figure 33 was initiated 9 days after tumor inoculation. Numbers of intratumoral macrophages **(A),** monocytes **(B)** and neutrophils **(C)** determined by flow cytometry and normalized to the tumor weight 20 days after tumor inoculation. Dots represent individual mice, lines represent the group mean. Significant differences were determined using Kruskal-Wallis test followed by Dunn's multiple comparison test. All analyses were two-tailed and carried out using GraphPad Prism 8. ns P>0.05, *P≤0.05, **P<0.01, ***P<0.001, ****P<0.0001.

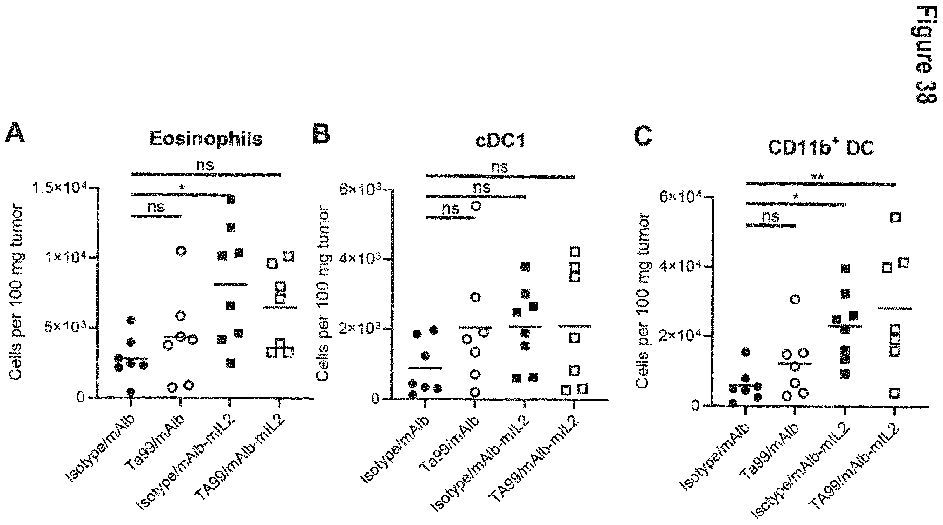

**Figure 38****: mAIb-mIL2 RNA alone or in combination with anti-Trp1 antibody increases the infiltration of eosinophils and DCs in the B16F10-*B2m*^{-/-} murine melanoma model.**
   C57BI/6 mice were inoculated subcutaneously (s.c.) with 3x10⁵ B16F10-*B2m*^{-/-} cells and treatment as described in Figure 33 was initiated 9 days after tumor inoculation. Numbers of intratumoral eosinophils **(A),** cDC1 **(B)** and CD11b⁺ DCs **(C)** determined by flow cytometry and normalized to the tumor weight 20 days after tumor inoculation. Dots represent individual mice, lines represent the group mean. Significant differences were determined using Kruskal-Wallis test followed by Dunn's multiple comparison test. All analyses were two-tailed and carried out using GraphPad Prism 8. ns P>0.05, *P≤0.05, **P<0.01, ***P<0.001, ****P<0.0001.

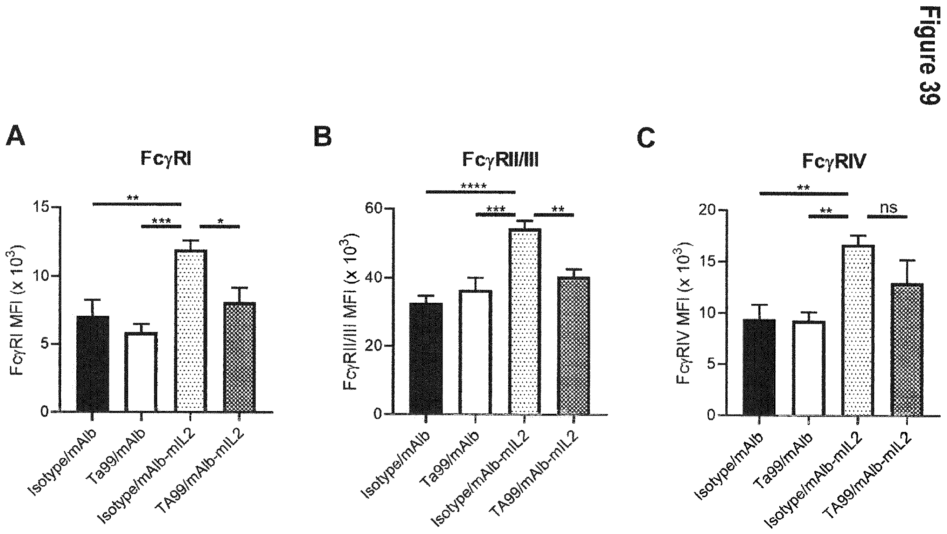

**Figure 39****: mAIb-mIL2 RNA induces upregulation of FcγRs by intratumoral macrophages in the B16F10-B2m**^{-/-} **murine melanoma model.**
   C57BI/6 (n=7-8 per group) mice were inoculated subcutaneously (s.c.) with 3x10⁵ B16F10-*B2m*^{-/-} cells and treatment as described in Figure 33 was initiated 9 days after tumor inoculation. Expression of FcγRI **(A),** Fc FcγRII/III **(B)** and FcyRIV (C) by intratumoral macrophages determined by flow cytometry 20 days after tumor inoculation. Data shown are mean+SEM. Statistical significance was determined using one-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

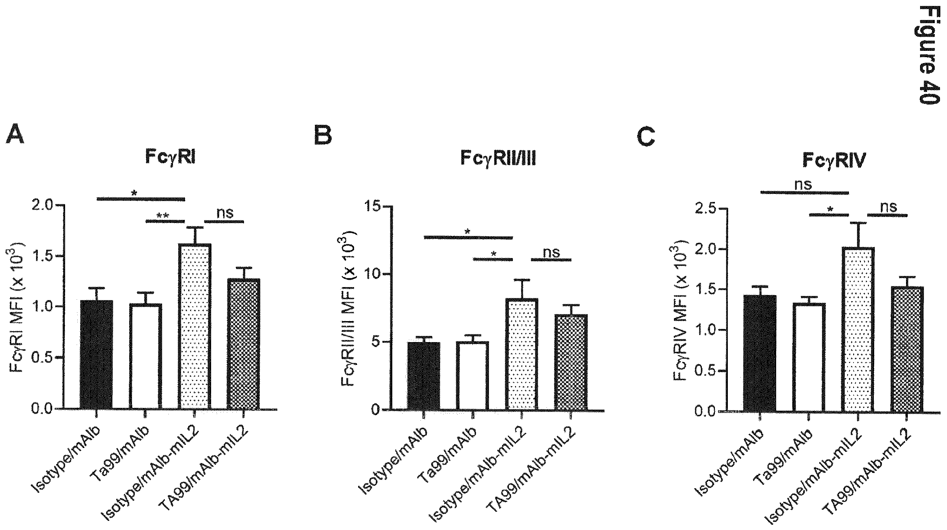

**Figure 40****: mAIb-mIL2 RNA induces upregulation of FcγRs by intratumoral monocytes in the B16F10-*B2m*^{-/-} murine melanoma model.**
   C57BI/6 (n=7-8 per group) mice were inoculated subcutaneously (s.c.) with 3x10⁵ B16F10-*B2m*^{-/-} cells and treatment as described in Figure 33 was initiated 9 days after tumor inoculation. Expression of FcγRI **(A),** Fc FcγRII/III **(B)** and FcyRIV **(C)** by intratumoral macrophages determined by flow cytometry 20 days after tumor inoculation. Data shown are mean+SEM. Statistical significance was determined using one-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

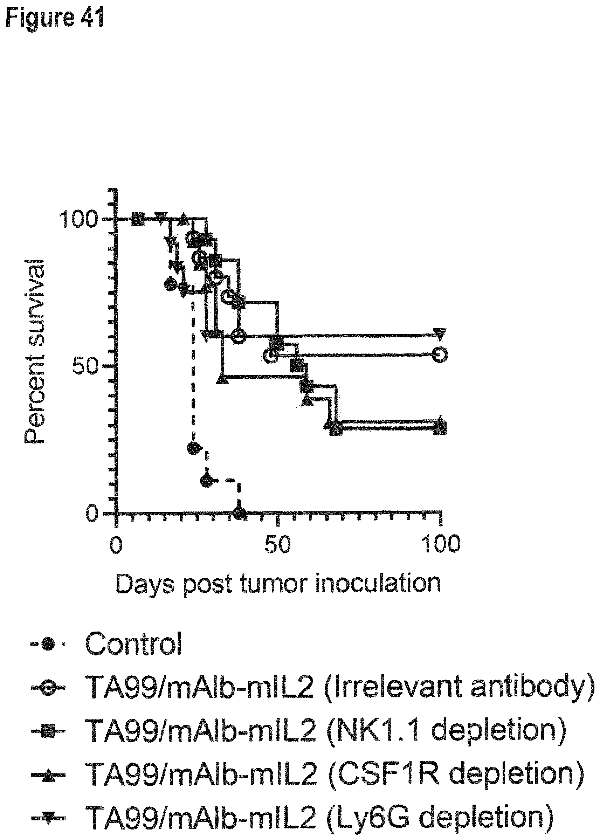

**Figure 41****: Depletion of macrophages, but not NK cells or neutrophils seems to impair the survival of mice treated with mAIb-mIL2 RNA in combination with anti-Trp1 antibody in the B16F10-*B2m***^{-/-} **murine melanoma model.**
   C57BI/6 mice (n=10 for control group, n=8-15 for all other groups) were inoculated subcutaneously (s.c.) with 3x10⁵ B16F10-*B2m*^{-/-} cells and treated as described in Figure 33 with anti-Trp1 (TA99) antibody on day 5, 8, 12, 15, 19, 22 and with RNA coding for mAlb-mIL2 on day 5, 12 and 19 after tumor inoculation. The control group received an isotype control antibody and RNA coding for mAlb (not coding for any cytokine). Depleting antibodies against NK1.1, CSF1R or Ly6G or an irrelevant control antibody (non-depleting) were injected intraperitoneally (i.p.) with a loading dose of 400 µg on day 4 and following doses of 200 µg (irrelevant antibody, NK1.1 or Ly6G) or 300 µg (CSF1R) on day 7, 11, 14, 18 and 20 after tumor inoculation.

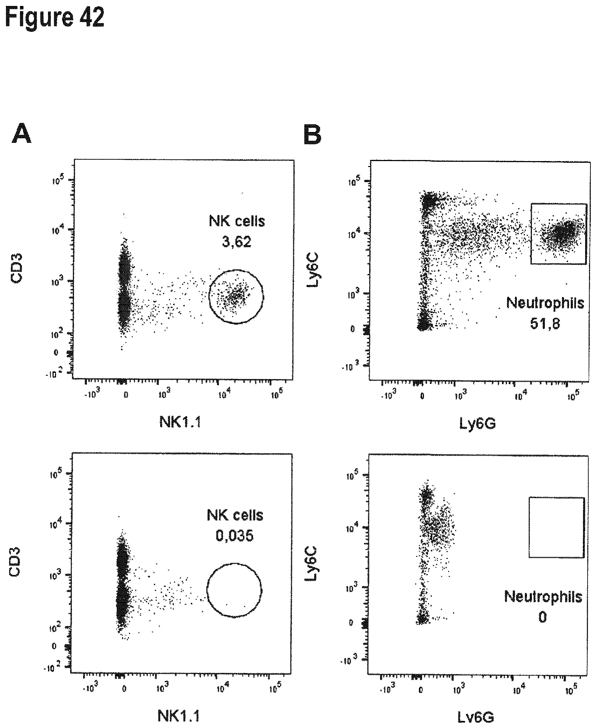

**Figure 42****: Flow cytometric analysis of blood samples to confirm immune cell depletion.**
   Flow cytometric analysis of NK1.1 (NK cell) **(A)** or Ly6G (neutrophil) **(B)** depletion in blood from mice treated as described in Figure 41 one day after injection of depleting antibodies. Upper panels show blood from a representative mouse injected with control antibody, lower panels show representative mice injected with depleting antibodies.

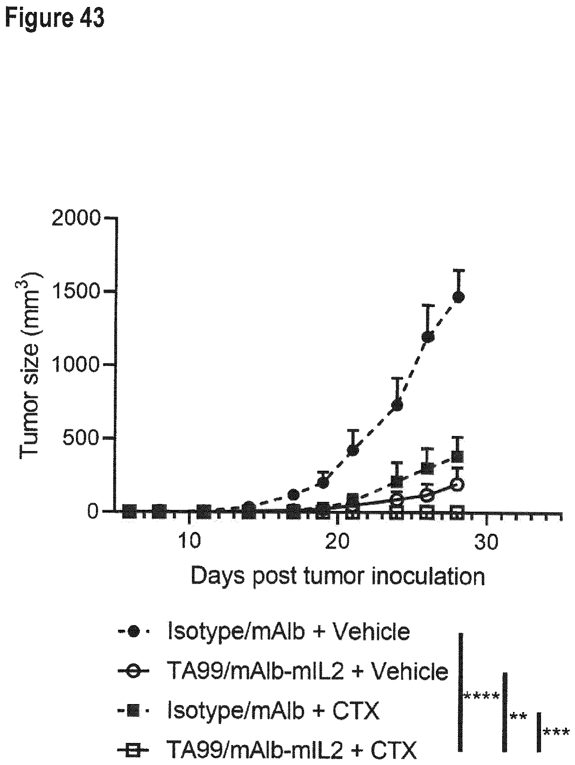

**Figure 43****: The tumor growth reduction of mAIb-mIL2 RNA in combination with anti-Trp1 antibody is enhanced by chemotherapy in the B16F10-*B2m***^{-/-} **murine melanoma model.**
   C57BI/6 mice (n=12-13 per group) were inoculated subcutaneously (s.c.) with 3x10⁵ B16F10-*B2m*^{-/-} cells and injected intraperitoneally (i.p.) with 200 mg/kg cyclophosphamide (CTX) or vehicle as control on day 6 after tumor inoculation. Mice were treated as described in Figure 33 with or without anti-Trp1 (TA99) antibody on day 7, 10, 14, 17 and 21 and RNA coding for mAlb-mIL2 on day 7, 14 and 21 after tumor inoculation. Control groups were treated with isotype control antibody or RNA coding for mAlb (not coding for any cytokine). Data shown are mean+SEM. Statistical significance was determined using two-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

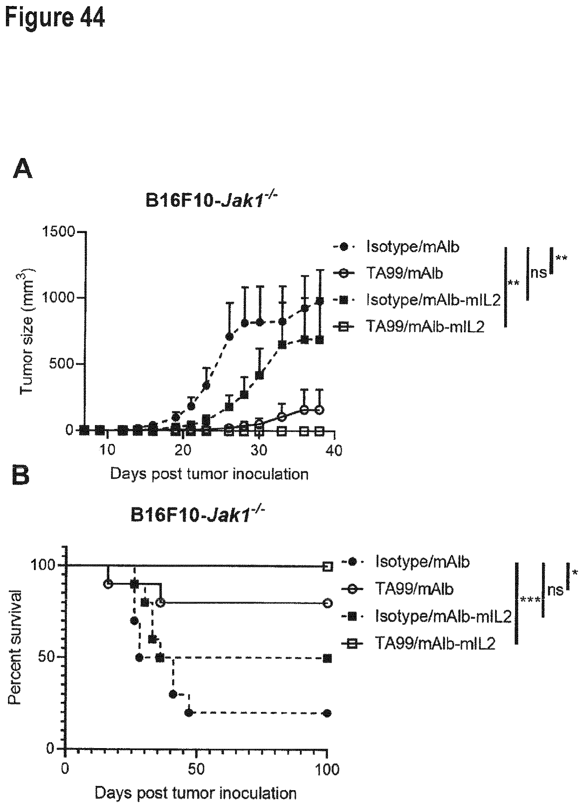

**Figure 44****: anti-Trp1 antibody and mAIb-mIL2 RNA combination therapy reduces the growth of B16F10-*Jak1*^{-/} murine melanoma tumors.**
   C57BI/6 mice (n=10 per group) were inoculated subcutaneously (s.c.) with 3x10⁵ B16F10-*Jak1*^{-/-} cells and treated as described in Figure 33 with anti-Trp1 (TA99) antibody on day 5, 8, 12, 15 and 19 and RNA coding for mAlb-mIL2 on day 5, 12 and 19 after tumor inoculation. Control groups were treated with isotype control antibody or RNA coding for mAlb (not coding for any cytokine). Tumor growth **(A)** and survival **(B)** of tumor bearing mice. Data shown are mean+SEM. Statistical significance was determined using two-way ANOVA followed by Dunnet's multiple comparison test. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

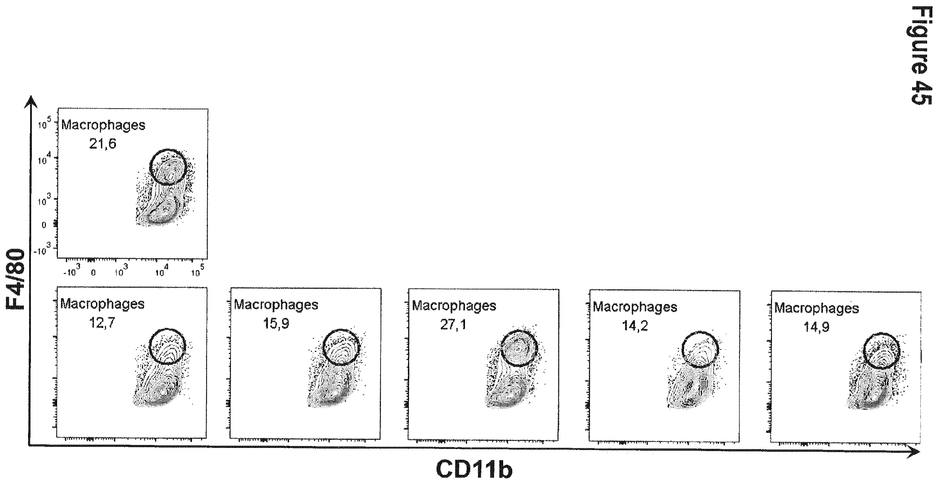

**Figure 45****: Comparison of the immune cell infiltration of TC1 and TC1-*B2m*^{-/-} murine lung epithelial tumors.**
   C57BI/6 mice were inoculated subcutaneously (s.c.) with 3x10⁵ B16F10-*B2m*^{-/-} cells. Anti-CSF1R antibody was injected intraperitoneally (i.p.) with a dose of 600 µg on day 10 and with a dose of 350 µg on day 12 after tumor inoculation. The presence of a GR-1⁻ CD11b⁺ F4/80⁺ macrophage population was analyzed by flow cytometry 13 days after tumor inoculation. Upper panel represents an untreated control, lower panels show individual mice injected with antibody.

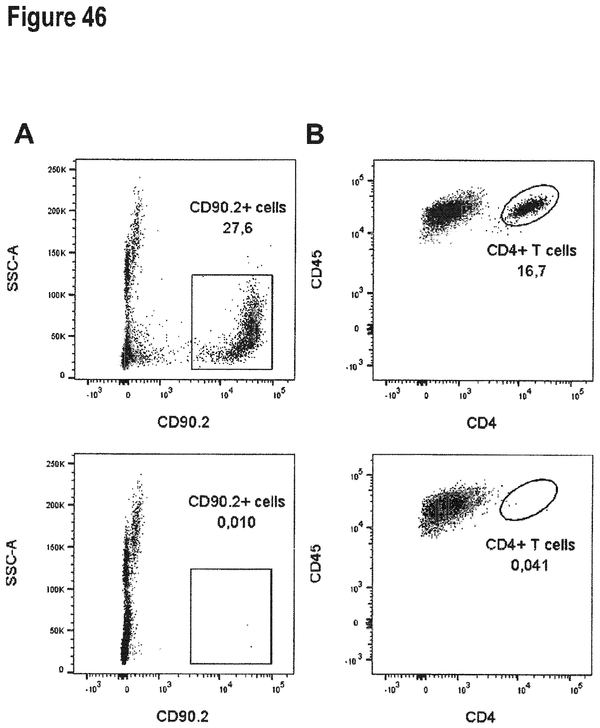

**Figure 46****: Flow cytometric analysis of blood samples to confirm CD4⁺ T cell and total lymphocyte depletion.**
   C57BI/6 mice (n=10 for control group, n=15 for all other groups) were inoculated subcutaneously (s.c.) with 3x10⁵ B16F10-*B2m*^{-/-} cells and treated as described in Figure 33 with anti-Trp1 (TA99) antibody on day 5, 8, 12, 15 and 19 and with RNA coding for mAlb-mIL2 on day 5, 12 and 19 after tumor inoculation. Depleting antibodies against CD4 or CD90.2 or an irrelevant control antibody (non-depleting) were injected intraperitoneally (i.p.) with a loading dose of 400 µg on day 3 and following doses of 200 µg on day 7, 10, 14 and 20 (on day 20 only control and anti-CD4) after tumor inoculation. Depleting antibody against CSF1R was injected intraperitoneally (i.p.) with a loading dose of 600 µg on day 2 and following doses of 350 µg on day 5, 7, 10, 12 and 14 after tumor inoculation. Flow cytometric analysis of CD4 (CD4⁺ T cells) **(A)** or CD90.2 (total lymphocytes) **(B)** depletion in blood from mice 2 days after injection of depleting antibodies. Upper panels show blood from representative mice injected with control antibody, lower panels show representative mice injected with depleting antibodies.

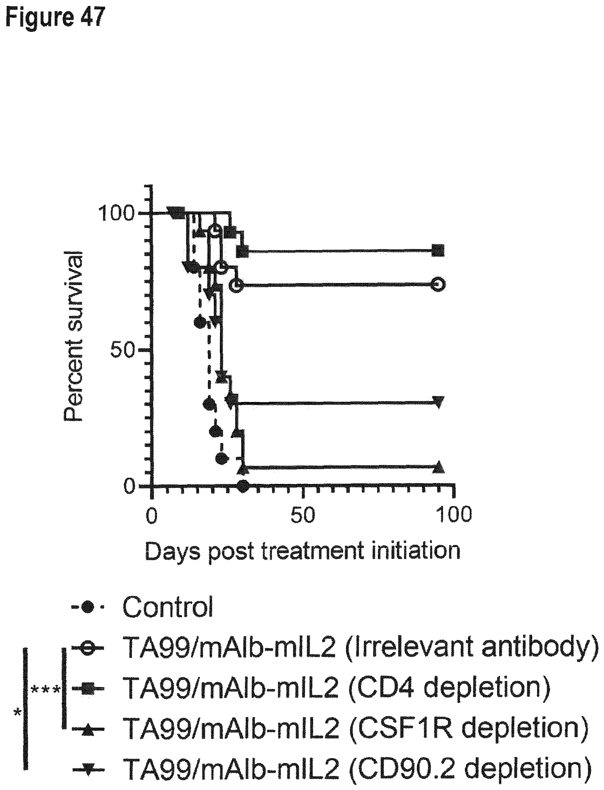

**Figure 47****: Depletion of macrophages or total lymphocytes impair the survival of mice treated with mAIb-mIL2 RNA in combination with anti-Trp1 antibody in the B16F10-*B2m*^{-/-} murine melanoma model.**
   C57BI/6 mice were inoculated and treated as described in Figure 46. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

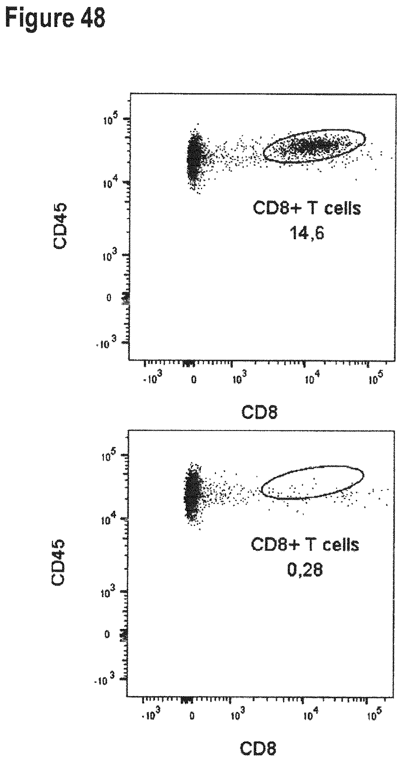

**Figure 48****: Flow cytometric analysis of blood samples to confirm CD8⁺ T cell depletion.**
   C57BI/6 mice (n=8 for control group, n=14 for the group receiving irrelevant control antibody, n=15 for the group receiving anti-IFNy, n=13 for the group receiving anti-CD8) were inoculated and treated with anti-Trp1 (TA99) and with RNA coding for mAlb-mIL2 as described in Figure 46. Depleting antibody against CD8 or neutralizing antibody against IFNy or an irrelevant control antibody (non-depleting) were injected intraperitoneally (i.p.) with a loading dose of 400 µg on day 3 and following doses of 200 µg anti-CD8 on day 7 and 10 or 250 µg control antibody or anti-IFNγ on day 5, 7 and 10 after tumor inoculation. Flow cytometric analysis of CD8 (CD8⁺ T cells) depletion in blood from mice 2 days after injection of anti-CD8 antibody. Upper panel shows blood from a representative mouse injected with control antibody, lower panel shows representative mouse injected with depleting antibody.

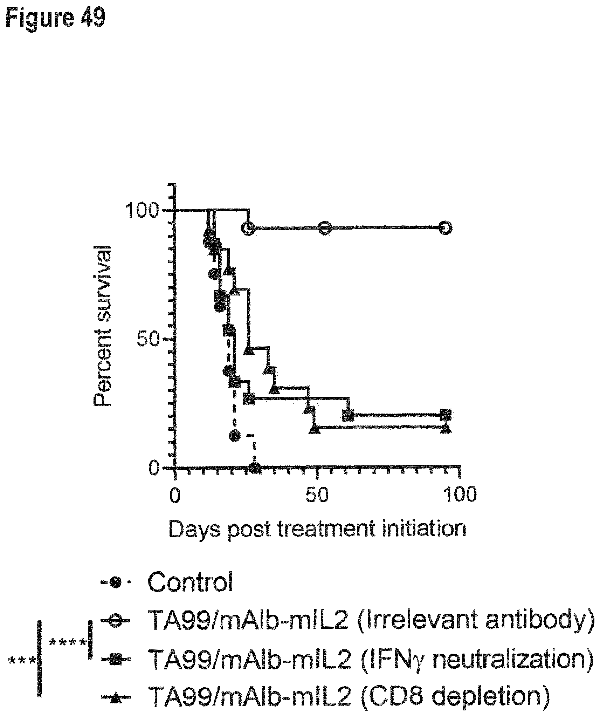

**Figure 49****: Depletion of CD8⁺ T cells or IFNγ neutralization impair the survival of mice treated with mAIb-mIL2 RNA in combination with anti-Trp1 antibody in the B16F10-*B2m*^{-/-} murine melanoma model.**
   C57BI/6 mice were inoculated and treated as described in Figure 47. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

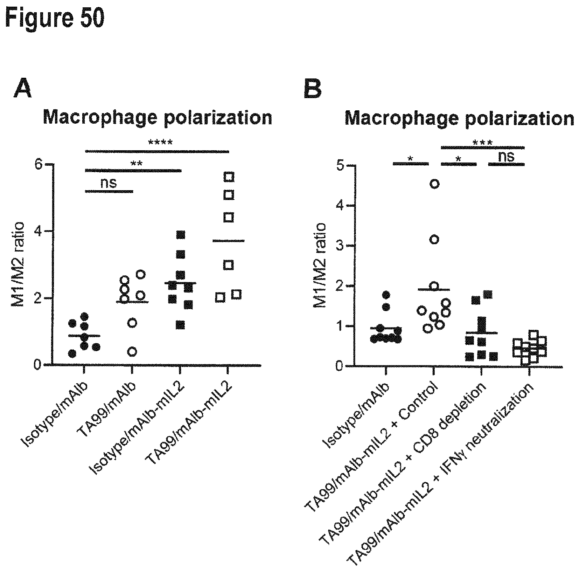

**Figure 50****: CD8⁺ T cells and IFNγ are required for proinflammatory polarization of macrophages in response to mAIb-mIL2 alone or in combination with anti-Trp1 antibody in the B16F10-*B2m*^{-/-} murine melanoma model.**
   C57BI/6 mice were inoculated subcutaneously (s.c.) with 3x10⁵ B16F10-*B2m*^{-/-} cells and treatment as described in Figure 33 was initiated 9 days after tumor inoculation. In **(B)** mice were additionally injected with anti-IFNγ, anti-CD8 or irrelevant control antibody with a loading dose of 400 µg on day 7 and following doses of 200 µg anti-CD8 on day 11 and 14 or 250 µg control antibody or anti-IFNγ on day 9, 11 and 14 after tumor inoculation. Polarization of intratumoral macrophages after treatment with mAIb-mIL2 and anti-Trp1 (TA99) combination therapy, the respective monotherapies or controls only 20 days after tumor inoculation **(A)** and after treatment with mAlb-mIL2 and anti-Trp1 (TA99) combination therapy and injection of anti-CD8 or anti-IFNγ antibodies 18 days after tumor inoculation **(B).**

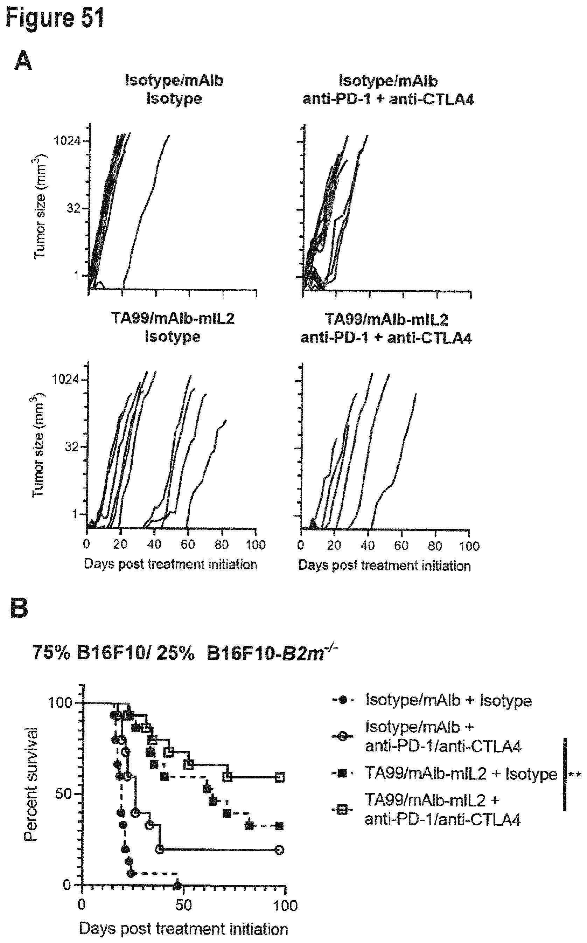

**Figure 51****: Antibody and mAIb-mIL2 combination therapy improves the antitumoral activity of anti-PD-1 and anti-CTLA4 therapy against heterogeneous tumors consisting of B16F10 and B16F10-*B2m*^{*-*/*-*} cells.** C57BI/6 mice (n=15 per group) were inoculated subcutaneously (s.c.) with 3x10⁵ cells of a mixture containing 75% B16F10 and 25% B16F10-*B2m*^{-/-} cells. Mice were treated as described in Figure 33 with anti-Trp1 (TA99) antibody on day 3, 7, 10, 14 and 17 and with RNA coding for mAlb-mIL2 on day 3, 10 and 17 after tumor inoculation. Mice were additionally injected intraperitoneally (i.p.) with 200 µg anti-PD-1 on day 3, 7, 10, 14 and 17 and with anti-CTLA4 with a loading dose of 200 µg on day 3 and following doses of 100 µg on day 7, 10, 14 and 17. Isotypes and mAlb RNA served as controls. Single tumor growth curves **(A)** and survival **(B)** of tumor bearing mice. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

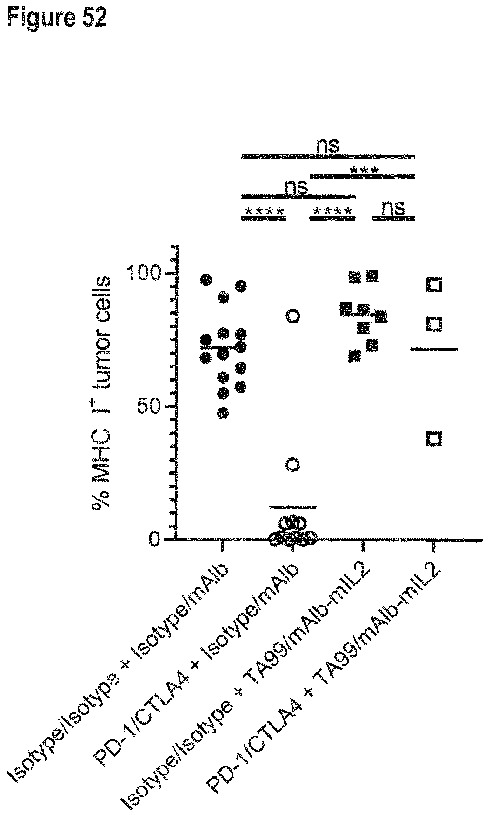

**Figure 52****: Antibody and mAIb-mIL2 combination therapy prevents acquired resistance against PD-1 and anti-CTLA4 in the B16F10 murine melanoma model.**
   C57BI/6 mice were inoculated and treated as described in Figure 51. Tumors were taken when mice reached endpoint criteria and the fraction of MHC class I⁺ tumor cells was determined in tumor cell suspensions by flow cytometry after 24 h stimulation with 25 ng/mL IFNγ. Statistical significance was determined using one-way ANOVA followed by Dunnet's multiple comparison test. All analyses were two-tailed and carried out using GraphPad Prism 8. ns P>0.05, *P≤0.05, **P<0.01, ***P<0.001, ****P<0.0001.

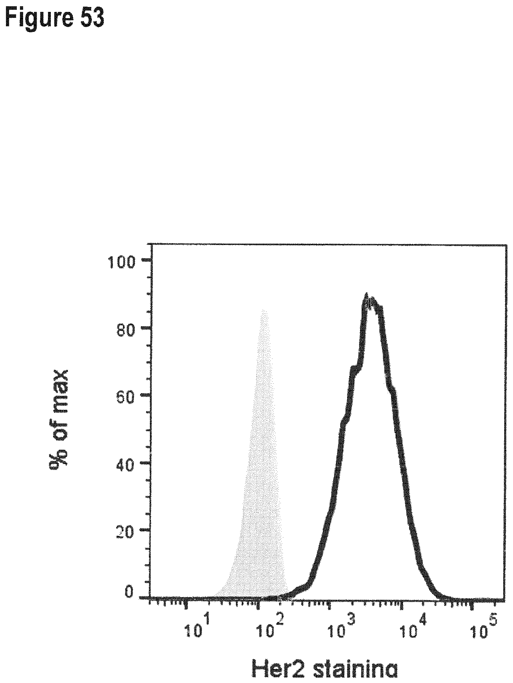

**Figure 53****: Her2 surface expression of MC38-Her2-*B2m***^{-/-} **cells.**
   MC38-Her2-*B2m*^{-/-} cells were incubated with 20 µg/mL anti-Her2 antibody for 20 min at 4 °C and binding of antibody was detected using an anti-mouse 2^{nd} antibody and analyzed by flow cytometry.

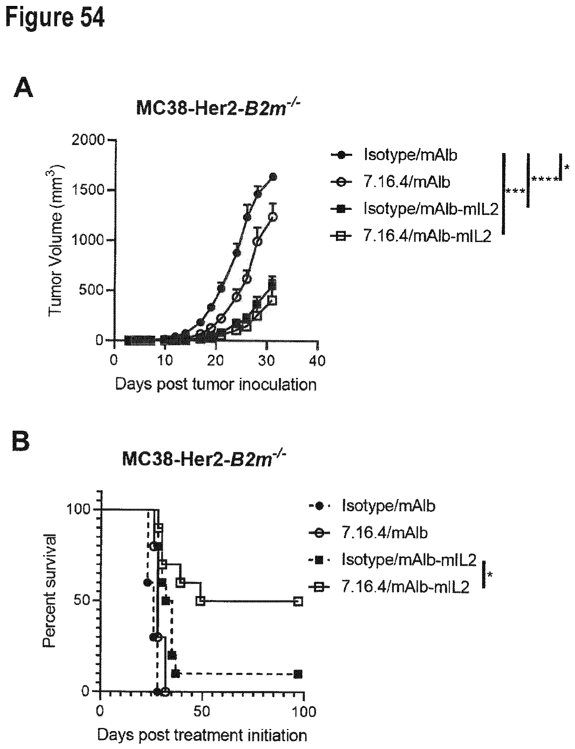

**Figure 54****: anti-Her2 antibody and mAIb-mIL2 RNA combination therapy reduces the tumor growth and leads to long-term survival in the MC38-Her2-*B2m***^{-/-} **murine colon adenocarcinoma model.** C57BI/6 mice (n=10 per group) were inoculated subcutaneously (s.c.) with 5x10⁵ MC38-Her2-*B2m*^{-/-} cells. Mice were injected intraperitoneally (i.p.) with 200 µg anti-Her2 antibody (7.16.4) or isotype control on day 3, 6, 10, 13, 17 and 24 and with RNA coding for mAlb-mIL2 or mAlb (not coding for any cytokine) as described in Figure 33 on day 3, 10, 17 and 24 after tumor inoculation. The control group was treated with isotype and mAlb RNA. Tumor growth shown as mean+SEM **(A)** and survival **(B)** of tumor bearing mice. Statistical significance was determined using two-way ANOVA followed by Dunnet's multiple comparison test and is shown for the last depicted time point **(A)** or by log-rank (Mantel-Cox) test **(B).** All analyses were two-tailed and carried out using GraphPad Prism 8. ns P>0.05, *P≤0.05, **P<0.01, ***P<0.001, ****P<0.0001.

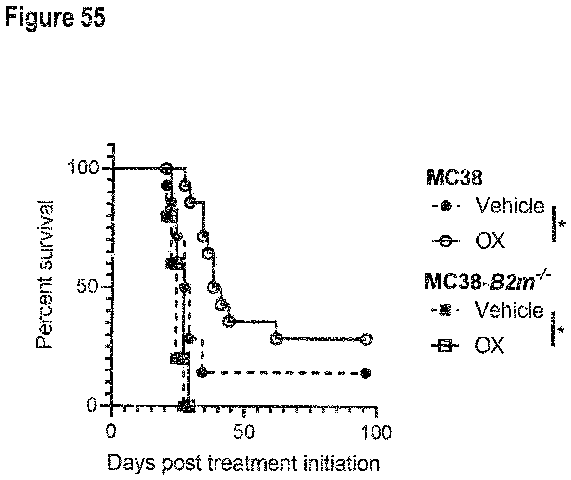

**Figure 55****: *B2m* deficiency prevents tumor rejection after treatment with chemotherapy in the MC38 murine colon adenocarcinoma model.**
   C57BI/6 mice were inoculated subcutaneously (s.c.) with 5x10⁵ MC38 (n=15 per group) or MC38-*B2m*^{-/-} (n=10 per group) cells. Mice were injected intraperitoneally (i.p.) with 5 mg/kg Oxaliplatin on day 4, 11 and 18 after tumor inoculation. The control groups received vehicle. Survival of tumor bearing mice. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

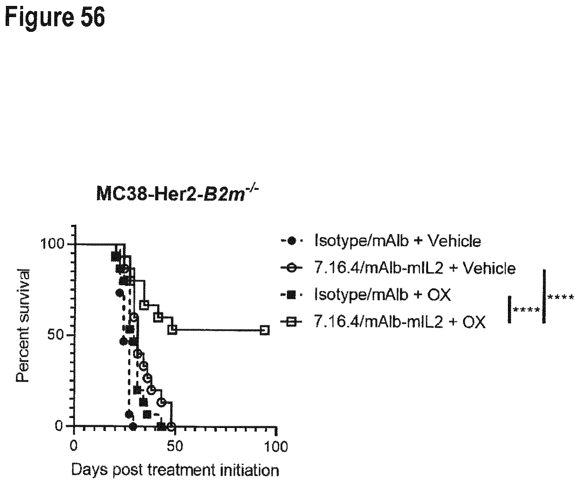

**Figure 56****: anti-Trp1 antibody and mAIb-mIL2 RNA combination therapy synergize with chemotherapy in the MC38-Her2-*B2m***^{-/-} **murine colon adenocarcinoma model.**
   C57BI/6 mice (n=15 per group) were inoculated subcutaneously (s.c.) with 5x10⁵ MC38-Her2-*B2m*^{-/-} cells and treated with oxaliplatin (OX) as described in Figure 55. Mice were additionally treated as described in Figure 54 with anti-Her2 (7.16.4) antibody on day 5, 8, 12, 15 and 19 and RNA coding for mAlb-mlL2 on day 7, 14 and 21. Control groups were treated with vehicle, isotype control antibody or RNA coding for mAlb (not coding for any cytokine). Survival of tumor bearing mice. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

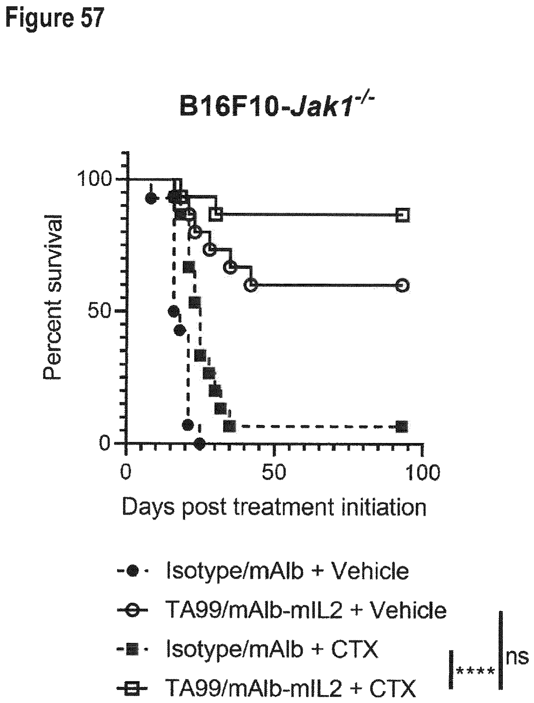

**Figure 57****: The tumor growth reduction of mAIb-mIL2 RNA in combination with anti-Trp1 antibody is enhanced by chemotherapy in the B16F10-*B2m***^{-/-} **murine melanoma model.**
   C57BI/6 mice (n=14 for the group receiving only controls, n=15 for all other groups) were inoculated subcutaneously (s.c.) with 5x10⁵ B16F10-*Jak1*^{-/-} cells and injected intraperitoneally (i.p.) with 150 mg/kg cyclophosphamide (CTX) or vehicle as control on day 7 after tumor inoculation. Mice were treated as described in Figure 33 with anti-Trp1 (TA99) antibody on 8, 11, 15, 18 and 22 and RNA coding for mAlb-mIL2 on day 8, 15 and 22 after tumor inoculation. Control groups were treated with vehicle, isotype control antibody or RNA coding for mAlb (not coding for any cytokine). Survival of tumor bearing mice. Significant differences in survival were determined using log-rank (Mantel-Cox) test. All analyses were two-tailed and carried out using GraphPad Prism 8. ns P>0.05, *P≤0.05, **P<0.01, ***P<0.001, ****P<0.0001.

### Detailed description

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present disclosure will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, the elements of the present disclosure will be described. These elements are listed with specific embodiments, however, it should be understood that they may be combined in any manner and in any number to create additional embodiments. The variously described examples and embodiments should not be construed to limit the present disclosure to only the explicitly described embodiments. This description should be understood to disclose and encompass embodiments which combine the explicitly described embodiments with any number of the disclosed elements. Furthermore, any permutations and combinations of all described elements should be considered disclosed by this description unless the context indicates otherwise.

The term "about" means approximately or nearly, and in the context of a numerical value or range set forth herein in one embodiment means ± 20%, ± 10%, ± 5%, or ± 3% of the numerical value or range recited or claimed.

The terms "a" and "an" and "the" and similar reference used in the context of describing the disclosure (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the disclosure and does not pose a limitation on the scope of the claims. No language in the specification should be construed as indicating any non-claimed element essential to the practice of the disclosure.

Unless expressly specified otherwise, the term "comprising" is used in the context of the present document to indicate that further members may optionally be present in addition to the members of the list introduced by "comprising". It is, however, contemplated as a specific embodiment of the present disclosure that the term "comprising" encompasses the possibility of no further members being present, i.e., for the purpose of this embodiment "comprising" is to be understood as having the meaning of "consisting of".

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the present disclosure was not entitled to antedate such disclosure.

In the following, definitions will be provided which apply to all aspects of the present disclosure. The following terms have the following meanings unless otherwise indicated. Any undefined terms have their art recognized meanings.

### Definitions

A cancer which is "at least partially resistant to an MHC-dependent T cell response" means that the MHC-dependent T cell response against the cancer is reduced, in particular because the cancer has developed resistance mechanisms as described herein, compared to the normal situation, e.g., the situation wherein a cancer is not resistant to an MHC-dependent T cell response, e.g., wherein a productive MHC-dependent T cell response against the cancer can occur ultimately resulting in the attack and killing of tumor cells and/or wherein the cancer does respond to treatment based on, i.e., involving, T cells. Such cancer which is not at least partially resistant to an MHC-dependent T cell response preferably shows normal processing and/or presentation of antigens as well as normal IFN-signaling. Accordingly, a cancer which is "at least partially resistant to an MHC-dependent T cell response" may partially or fully escape a productive MHC-dependent T cell response against the cancer and/or tumor cell killing by MHC-dependent T cells. The MHC-dependent T cell response may be an endogenous reaction against the cancer and/or may be a reaction which results from an induction of an MHC-dependent T cell response, e.g., due to immunotherapy involving T cells.

The term "does not adequately respond" means that the response is reduced compared to the normal situation, e.g., the situation wherein a cancer is not at least partially resistant to an MHC-dependent T cell response and does respond to treatment based on, i.e., involving, T cells. Such cancer which is not at least partially resistant to an MHC-dependent T cell response preferably shows normal processing and/or presentation of antigens as well as normal IFN-signaling. The term "deficient" means that a property or activity an object is deficient in is reduced compared to the normal situation wherein such deficiency is not present.

Preferably, a cancer which is at least partially resistant to an MHC-dependent T cell response and/or does not adequately respond to treatment based on T cells 1) may be deficient in mechanisms resulting in processing and/or presenting antigens, e.g., the cancer may be MHC-I deficient, wherein the deficiency in MHC-I may be due to a mutation or partial or full loss of MHC-I alleles or beta2-microglobulin (B2M); 2) may be deficient in stimulating T cells, e.g. due to a deficiency in IFN-signaling such as type I IFN or IFNy signaling; or 3) may have lost antigen that is presented in the context of MHC for recognition by e.g. CD8⁺ T cells, or a combination thereof.

Terms such as "reduce", "decrease", "inhibit" or "impair" as used herein relate to an overall decrease or the ability to cause an overall decrease, preferably of 5% or greater, 10% or greater, 20% or greater, more preferably of 50% or greater, more preferably of 75% or greater and most preferably 100%, in the level, e.g. in the level of binding.

Terms such as "increase", "enhance" or "exceed" preferably relate to an increase or enhancement by about at least 10%, preferably at least 20%, preferably at least 30%, more preferably at least 40%, more preferably at least 50%, even more preferably at least 80%, and most preferably at least 100%, at least 200%, at least 500%, or even more.

According to the disclosure, the term "peptide" refers to substances which comprise about two or more, about 3 or more, about 4 or more, about 6 or more, about 8 or more, about 10 or more, about 13 or more, about 16 or more, about 20 or more, and up to about 50, about 100 or about 150, consecutive amino acids linked to one another via peptide bonds. The term "protein" or "polypeptide" refers to large peptides, in particular peptides having at least about 150 amino acids, but the terms "peptide", "protein" and "polypeptide" are used herein usually as synonyms.

A "therapeutic protein" has a positive or advantageous effect on a condition or disease state of a subject when provided to the subject in a therapeutically effective amount. In one embodiment, a therapeutic protein has curative or palliative properties and may be administered to ameliorate, relieve, alleviate, reverse, delay onset of or lessen the severity of one or more symptoms of a disease or disorder. A therapeutic protein may have prophylactic properties and may be used to delay the onset of a disease or to lessen the severity of such disease or pathological condition. The term "therapeutic protein" includes entire proteins or peptides, and can also refer to therapeutically active fragments thereof. It can also include therapeutically active variants of a protein. Examples of therapeutically active proteins include, but are not limited to, cytokines.

"Fragment", with reference to an amino acid sequence (peptide or protein), relates to a part of an amino acid sequence, i.e., a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 50 %, at least 60 %, at least 70 %, at least 80%, at least 90% of the amino acid residues from an amino acid sequence. A fragment of an amino acid sequence preferably comprises at least 6, in particular at least 8, at least 12, at least 15, at least 20, at least 30, at least 50, or at least 100 consecutive amino acids from an amino acid sequence.

By "variant" or "variant protein" or "variant polypeptide" herein is meant a protein that differs from a wild type protein by virtue of at least one amino acid modification. The parent polypeptide may be a naturally occurring or wild type (WT) polypeptide, or may be a modified version of a wild type polypeptide. Preferably, the variant polypeptide has at least one amino acid modification compared to the parent polypeptide, e.g., from 1 to about 20 amino acid modifications, and preferably from 1 to about 10 or from 1 to about 5 amino acid modifications compared to the parent.

By "parent polypeptide", "parent protein", "precursor polypeptide", or "precursor protein" as used herein is meant an unmodified polypeptide that is subsequently modified to generate a variant. A parent polypeptide may be a wild type polypeptide, or a variant or engineered version of a wild type polypeptide.

By "wild type" or "WT" or "native" herein is meant an amino acid sequence that is found in nature, including allelic variations. A wild type protein or polypeptide has an amino acid sequence that has not been intentionally modified.

For the purposes of the present disclosure, "variants" of an amino acid sequence (peptide, protein or polypeptide) comprise amino acid insertion variants, amino acid addition variants, amino acid deletion variants and/or amino acid substitution variants. The term "variant" includes all splice variants, posttranslationally modified variants, conformations, isoforms and species homologs, in particular those which are naturally expressed by cells. The term "variant" includes, in particular, fragments of an amino acid sequence.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in peptide and protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids. In one embodiment, conservative amino acid substitutions include substitutions within the following groups:
glycine, alanine;
valine, isoleucine, leucine;
aspartic acid, glutamic acid;
asparagine, glutamine;
serine, threonine;
lysine, arginine; and
phenylalanine, tyrosine.

Preferably the degree of similarity, preferably identity between a given amino acid sequence and an amino acid sequence which is a variant of said given amino acid sequence will be at least about 60%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5. "Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit according to the disclosure at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook et al. (1989), for example. Furthermore, the peptides and amino acid variants described herein may be readily prepared with the aid of known peptide synthesis techniques such as, for example, by solid phase synthesis and similar methods.

In one embodiment, a fragment or variant of an amino acid sequence (peptide or protein) is preferably a "functional fragment" or "functional variant". The term "functional fragment" or "functional variant" of an amino acid sequence relates to any fragment or variant exhibiting one or more functional properties identical or similar to those of the amino acid sequence from which it is derived, i.e., it is functionally equivalent. With respect to cytokines such as IL2, one particular function is one or more immunomodulatory activities displayed by the amino acid sequence from which the fragment or variant is derived and/or binding to the receptor(s) the amino acid sequence from which the fragment or variant is derived binds to. The term "functional fragment" or "functional variant", as used herein, in particular refers to a variant molecule or sequence that comprises an amino acid sequence that is altered by one or more amino acids compared to the amino acid sequence of the parent molecule or sequence and that is still capable of fulfilling one or more of the functions of the parent molecule or sequence, e.g., binding to a target molecule or contributing to binding to a target molecule. In one embodiment, the modifications in the amino acid sequence of the parent molecule or sequence do not significantly affect or alter the binding characteristics of the molecule or sequence. In different embodiments, binding of the functional fragment or functional variant may be reduced but still significantly present, e.g., binding of the functional variant may be at least 50%, at least 60%, at least 70%, at least 80%, or at least 90% of the parent molecule or sequence. However, in other embodiments, binding of the functional fragment or functional variant may be enhanced compared to the parent molecule or sequence.

An amino acid sequence (peptide, protein or polypeptide) "derived from" a designated amino acid sequence (peptide, protein or polypeptide) refers to the origin of the first amino acid sequence. Preferably, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof. For example, it will be understood by one of ordinary skill in the art that the IL2 compounds suitable for use herein may be altered such that they vary in sequence from the naturally occurring or native sequences from which they were derived, while retaining the desirable activity of the native sequences.

As used herein, an "instructional material" or "instructions" includes a publication, a recording, a diagram, or any other medium of expression which can be used to communicate the usefulness of the compositions and methods of the invention. The instructional material of the kit of the invention may, for example, be affixed to a container which contains the compositions of the invention or be shipped together with a container which contains the compositions. Alternatively, the instructional material may be shipped separately from the container with the intention that the instructional material and the compositions be used cooperatively by the recipient.

"Isolated" means altered or removed from the natural state. For example, a nucleic acid or a peptide naturally present in a living animal is not "isolated", but the same nucleic acid or peptide partially or completely separated from the coexisting materials of its natural state is "isolated". An isolated nucleic acid or protein can exist in substantially purified form, or can exist in a non-native environment such as, for example, a host cell.

The term "recombinant" in the context of the present invention means "made through genetic engineering". Preferably, a "recombinant object" such as a recombinant cell in the context of the present invention is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

The term "genetic modification" includes the transfection of cells with nucleic acid. The term "transfection" relates to the introduction of nucleic acids, in particular RNA, into a cell. For purposes of the present invention, the term "transfection" also includes the introduction of a nucleic acid into a cell or the uptake of a nucleic acid by such cell, wherein the cell may be present in a subject, e.g., a patient. Thus, according to the present invention, a cell for transfection of a nucleic acid described herein can be present *in vitro* or *in vivo,* e.g. the cell can form part of an organ, a tissue and/or an organism of a patient. According to the invention, transfection can be transient or stable. For some applications of transfection, it is sufficient if the transfected genetic material is only transiently expressed. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. Such stable transfection can be achieved by using virus-based systems or transposon-based systems for transfection. Generally, nucleic acid encoding a cytokine such as IL2 is transiently transfected into cells. RNA can be transfected into cells to transiently express its coded protein.

The term "immune effector cell" or "effector cell" in the context of the present invention relates to a cell which exerts effector functions during an immune reaction. For example, immune effector cells comprise T cells (cytotoxic T cells, helper T cells, tumor infiltrating T cells), B cells, natural killer cells, neutrophils, macrophages, and dendritic cells. The terms "T cell" and "T lymphocyte" are used interchangeably herein and include T helper cells (CD4+ T cells) and cytotoxic T cells (CTLs, CD8+ T cells) which comprise cytolytic T cells. The term "MHC-dependent T cell" or similar terms relate to a T cell which recognizes an antigen when presented in the context of MHC and preferably exerts effector functions of T cells, e.g., killing of target cells expressing an antigen.

T cells belong to a group of white blood cells known as lymphocytes, and play a central role in cell-mediated immunity. They can be distinguished from other lymphocyte types, such as B cells and natural killer cells by the presence of a special receptor on their cell surface called T cell receptor (TCR). The thymus is the principal organ responsible for the maturation of T cells. Several different subsets of T cells have been discovered, each with a distinct function.

T helper cells assist other white blood cells in immunologic processes, including maturation of B cells into plasma cells and activation of cytotoxic T cells and macrophages, among other functions. These cells are also known as CD4+ T cells because they express the CD4 glycoprotein on their surface. Helper T cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein on their surface. These cells recognize their targets by binding to antigen associated with MHC class I, which is present on the surface of nearly every cell of the body.

All T cells have a T cell receptor (TCR) existing as a complex of several proteins. The TCR of a T cell is able to interact with immunogenic peptides (epitopes) bound to major histocompatibility complex (MHC) molecules and presented on the surface of target cells. Specific binding of the TCR triggers a signal cascade inside the T cell leading to proliferation and differentiation into a maturated effector T cell. In the majority of T cells, the actual T cell receptor is composed of two separate peptide chains, which are produced from the independent T cell receptor alpha and beta (TCRα and TCRβ) genes and are called α- and β-TCR chains. A much less common (2% of total T cells) group of T cells, the γδ T cells (gamma delta T cells) possess a distinct T cell receptor (TCR) on their surface, which is made up of one γ-chain and one δ-chain.

All T cells originate from hematopoietic stem cells in the bone marrow. Hematopoietic progenitors derived from hematopoietic stem cells populate the thymus and expand by cell division to generate a large population of immature thymocytes. The earliest thymocytes express neither CD4 nor CD8, and are therefore classed as double-negative (CD4-CD8-) cells. As they progress through their development they become double-positive thymocytes (CD4+CD8+), and finally mature to single-positive (CD4+CD8- or CD4-CD8+) thymocytes that are then released from the thymus to peripheral tissues.

As used herein, the term "NK cell" or "Natural Killer cell" refers to a subset of peripheral blood lymphocytes defined by the expression of CD56 or CD16 and the absence of the T cell receptor.

MHC molecules in humans are normally referred to as HLA (human leukocyte antigen) molecules. There are two principal classes of MHC molecules: class I and class II. MHC class I antigens are found on nearly all nucleated cells of the body. The primary function of this class of MHC molecules is to display (or present) peptide fragments of intracellular proteins to CTLs. Based on this display, CTLs will attack those displaying MHC-bound peptides, including disease-associated peptides (antigens) such as cancer antigens. CD8-positive T cells are usually cytotoxic (therefore named cytotoxic T cells = CTL), recognize peptides of 9 to 10 amino acids which are intracellularly processed from proteins of any subcellular localization and which are presented on the cellular surface by MHC class I molecules. Thus, the surface expression of MHC class I molecules plays a crucial role in determining the susceptibility of target cells to CTLs.

A problem often encountered in cancer immunotherapy is an impairment of the immunogenicity in cancer tissue, e.g., resulting in resistance to an MHC-dependent T cell response of a cancer. This so-called "immune escape" can be understood on the basis of phenotype differences encountered in neoplastic cells. For example, tumor cells show decreased ability to process and present antigens, have a decreased ability to stimulate T cells, e.g., autologous T cells, e.g., due to a deficiency in IFN-signaling, show complete down-regulation of immunogenic proteins associated with transformed cells and/or no or low expression of leukocyte adhesion molecules or other accessory molecules and selective down-regulation of certain MHC class I and class II alleles or B2M. MHC loss of function or expression can be caused by loss of single MHC alleles, MHC haplotypes or complete MHC class I loss due to bi-allelic B2M gene loss. Tumors that have lost the expression of MHC are thus resistant to any treatment based on MHC-dependent T cells. Indeed, impairment of MHC function is one of the key "immune escape" mechanisms of tumor cells and thus limits the application of T cell mediated immune therapy. According to the invention, a cancer that is at least partially resistant to an MHC-dependent T cell response may have acquired one or more of these immune escape mechanisms.

Genomic instability is a hallmark of cancer. It enables the tumor to evolve, adapt, and develop resistance to treatment. The interaction with the host immune system determines the capacity of a given tumor cell clone to survive and disseminate. Therefore, a process of "selection", especially due to T-cell immune pressure on e.g. MHC-I deficient tumor variants, might represent a natural process, Many therapies such as vaccination select for resistance and therefore do not effectively deal with the hurdle. Disclosed herein is a treatment wherein the cancer cells are subject to an alternative selection pressure arising by switching the cancer cell targeting. This alternative selection pressure also allows to avoid the development of resistance to MHC-dependent T cell responses.

### Interferons

Interferons (IFNs) are a group of signaling proteins made and released by host cells in response to the presence of several pathogens, such as viruses, bacteria, parasites, and also tumor cells. In a typical scenario, a virus-infected cell will release interferons causing nearby cells to heighten their anti-viral defenses.

Based on the type of receptor through which they signal, interferons are typically divided among three classes: type I interferon, type II interferon, and type III interferon.

All type I interferons bind to a specific cell surface receptor complex known as the IFN-α/β receptor (IFNAR) that consists of IFNAR1 and IFNAR2 chains.

The type I interferons present in humans are IFNα, IFNβ, IFNε, IFNκ and IFNω. In general, type I interferons are produced when the body recognizes a virus that has invaded it. They are produced by fibroblasts and monocytes. Once released, type I interferons bind to specific receptors on target cells, which leads to expression of proteins that will prevent the virus from producing and replicating its RNA and DNA.

The IFNα proteins are produced mainly by plasmacytoid dendritic cells (pDCs). They are mainly involved in innate immunity against viral infection. The genes responsible for their synthesis come in 13 subtypes that are called IFNA1, IFNA2, IFNA4, IFNA5, IFNA6, IFNA7, IFNA8, IFNA10, IFNA13, IFNA14, IFNA16, IFNA17, IFNA21. These genes are found together in a cluster on chromosome 9.

The IFNβ proteins are produced in large quantities by fibroblasts. They have antiviral activity that is involved mainly in innate immune response. Two types of IFNβ have been described, IFNβ1 and IFNβ3. The natural and recombinant forms of IFNβ1 have antiviral, antibacterial, and anticancer properties.

Type II interferon (IFNγ in humans) is also known as immune interferon and is activated by IL12. Furthermore, type II interferons are released by cytotoxic T cells and T helper cells.

Type III interferons signal through a receptor complex consisting of IL10R2 (also called CRF2-4) and IFNLR1 (also called CRF2-12). Although discovered more recently than type I and type II IFNs, recent information demonstrates the importance of type III IFNs in some types of virus or fungal infections.

In general, type I and II interferons are responsible for regulating and activating the immune response.

The term "autologous" is used to describe anything that is derived from the same subject. For example, "autologous transplant" refers to a transplant of tissue or organs derived from the same subject. Such procedures are advantageous because they overcome the immunological barrier which otherwise results in rejection.

The term "allogeneic" is used to describe anything that is derived from different individuals of the same species. Two or more individuals are said to be allogeneic to one another when the genes at one or more loci are not identical.

The term "syngeneic" is used to describe anything that is derived from individuals or tissues having identical genotypes, i.e., identical twins or animals of the same inbred strain, or their tissues.

The term "heterologous" is used to describe something consisting of multiple different elements. As an example, the transfer of one individual's bone marrow into a different individual constitutes a heterologous transplant. A heterologous gene is a gene derived from a source other than the subject.

### Nucleic acids

The term "polynucleotide" or "nucleic acid", as used herein, is intended to include DNA and RNA such as genomic DNA, cDNA, mRNA, recombinantly produced and chemically synthesized molecules. A nucleic acid may be single-stranded or double-stranded. RNA includes *in vitro* transcribed RNA (IVT RNA) or synthetic RNA.

Nucleic acids may be comprised in a vector. The term "vector" as used herein includes any vectors known to the skilled person including plasmid vectors, cosmid vectors, phage vectors such as lambda phage, viral vectors such as retroviral, adenoviral or baculoviral vectors, or artificial chromosome vectors such as bacterial artificial chromosomes (BAC), yeast artificial chromosomes (YAC), or P1 artificial chromosomes (PAC). Said vectors include expression as well as cloning vectors. Expression vectors comprise plasmids as well as viral vectors and generally contain a desired coding sequence and appropriate DNA sequences necessary for the expression of the operably linked coding sequence in a particular host organism (e.g., bacteria, yeast, plant, insect, or mammal) or in *in vitro* expression systems. Cloning vectors are generally used to engineer and amplify a certain desired DNA fragment and may lack functional sequences needed for expression of the desired DNA fragments.

In one embodiment of all aspects of the invention, nucleic acid such as nucleic acid encoding IL2, or nucleic acid encoding an antibody is expressed in cells of the subject treated to provide the IL2 or antibody. In one embodiment of all aspects of the invention, the nucleic acid is transiently expressed in cells of the subject. Thus, in one embodiment, the nucleic acid is not integrated into the genome of the cells. In one embodiment of all aspects of the invention, the nucleic acid is RNA, preferably *in vitro* transcribed RNA.

The nucleic acids described herein may be recombinant and/or isolated molecules.

In the present disclosure, the term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered RNAs are considered analogs of naturally-occurring RNA.

In certain embodiments of the present disclosure, the RNA is messenger RNA (mRNA) that relates to a RNA transcript which encodes a peptide or protein. As established in the art, mRNA generally contains a 5' untranslated region (5'-UTR), a peptide coding region and a 3' untranslated region (3'-UTR). In some embodiments, the RNA is produced by *in vitro* transcription or chemical synthesis. In one embodiment, the mRNA is produced by *in vitro* transcription using a DNA template where DNA refers to a nucleic acid that contains deoxyribonucleotides.

In one embodiment, RNA is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

In one embodiment, the RNA described herein may have modified nucleosides. In some embodiments, the RNA comprises a modified nucleoside in place of at least one (e.g., every) uridine.

The term "uracil," as used herein, describes one of the nucleobases that can occur in the nucleic acid of RNA. The structure of uracil is:

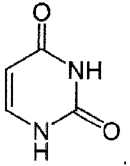

The term "uridine," as used herein, describes one of the nucleosides that can occur in RNA. The structure of uridine is:

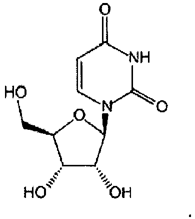

UTP (uridine 5'-triphosphate) has the following structure:

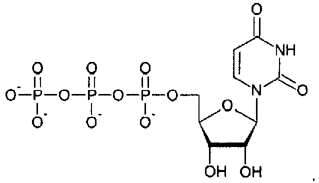

Pseudo-UTP (pseudouridine 5'-triphosphate) has the following structure:

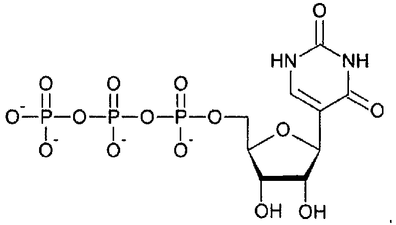

"Pseudouridine" is one example of a modified nucleoside that is an isomer of uridine, where the uracil is attached to the pentose ring via a carbon-carbon bond instead of a nitrogen-carbon glycosidic bond.

Another exemplary modified nucleoside is N1-methyl-pseudouridine (m1Ψ), which has the structure:

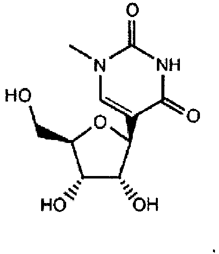

N1-methyl-pseudo-UTP has the following structure:

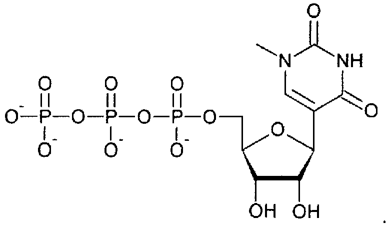

Another exemplary modified nucleoside is 5-methyl-uridine (m5U), which has the structure:

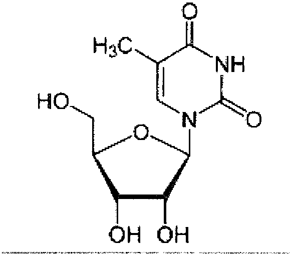

In some embodiments, one or more uridine in the RNA described herein is replaced by a modified nucleoside. In some embodiments, the modified nucleoside is a modified uridine.

In some embodiments, RNA comprises a modified nucleoside in place of at least one uridine. In some embodiments, RNA comprises a modified nucleoside in place of each uridine.

In some embodiments, the modified nucleoside is independently selected from pseudouridine (ψ), N1-methyl-pseudouridine (m1ψ), and 5-methyl-uridine (m5U). In some embodiments, the modified nucleoside comprises pseudouridine (ψ). In some embodiments, the modified nucleoside comprises N1-methyl-pseudouridine (m1ψ). In some embodiments, the modified nucleoside comprises 5-methyl-uridine (m5U). In some embodiments, RNA may comprise more than one type of modified nucleoside, and the modified nucleosides are independently selected from pseudouridine (ψ), N1-methyl-pseudouridine (m1ψ), and 5-methyl-uridine (m5U). In some embodiments, the modified nucleosides comprise pseudouridine (ψ) and N1-methyl-pseudouridine (m1ψ). In some embodiments, the modified nucleosides comprise pseudouridine (ψ) and 5-methyl-uridine (m5U). In some embodiments, the modified nucleosides comprise N1-methyl-pseudouridine (m1ψ) and 5-methyl-uridine (m5U). In some embodiments, the modified nucleosides comprise pseudouridine (ψ), N1-methyl-pseudouridine (m1ψ), and 5-methyl-uridine (m5U).

In some embodiments, the modified nucleoside replacing one or more uridine in the RNA may be any one or more of 3-methyl-uridine (m³U), 5-methoxy-uridine (mo⁵U), 5-aza-uridine, 6-aza-uridine, 2-thio-5-aza-uridine, 2-thio-uridine (s²U), 4-thio-uridine (s⁴U), 4-thio-pseudouridine, 2-thio-pseudouridine, 5-hydroxy-uridine (ho⁵U), 5-aminoallyl-uridine, 5-halo-uridine (e.g., 5-iodo-uridine or 5-bromo-uridine), uridine 5-oxyacetic acid (cmo⁵U), uridine 5-oxyacetic acid methyl ester (mcmo^{s}U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s²U), 5-aminomethyl-2-thio-uridine (nm⁵s²U), 5-methylaminomethyl-uridine (mnm⁵U), 1-ethyl-pseudouridine, 5-methylaminomethyl-2-thio-uridine (mnm⁵s²U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s²U), 5-propynyl-uridine, 1-propynyl-pseudouridine, 5-taurinomethyl-uridine (τm⁵U), 1-taurinomethyl-pseudouridine, 5-taurinomethyl-2-thio-uridine(τm5s2U), 1-taurinomethyl-4-thio-pseudouridine), 5-methyl-2-thio-uridine (m⁵s²U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ ψ), 5-(isopentenylaminomethyl)uridine (inm⁵U), 5-(isopentenylaminomethyl)-2-thio-uridine (inm⁵s²U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s²Um), 5-methoxycarbonylmethyl-2'-O-methyl-uridine (mcm⁵Um), 5-carbamoylmethyl-2'-O-methyl-uridine (ncm⁵Um), 5-carboxymethylaminomethyl-2'-O-methyl-uridine (cmnm⁵Um), 3,2'-O-dimethyl-uridine (m³Um), 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, or any other modified uridine known in the art.

In some embodiments, the RNA according to the present disclosure comprises a 5'-cap. In one embodiment, the RNA of the present disclosure does not have uncapped 5'-triphosphates. In one embodiment, the RNA may be modified by a 5'- cap analog. The term "5'-cap" refers to a structure found on the 5'-end of an mRNA molecule and generally consists of a guanosine nucleotide connected to the mRNA via a 5'- to 5'-triphosphate linkage. In one embodiment, this guanosine is methylated at the 7-position. Providing an RNA with a 5'-cap or 5'-cap analog may be achieved by *in vitro* transcription, in which the 5'-cap is co-transcriptionally expressed into the RNA strand, or may be attached to RNA post-transcriptionally using capping enzymes.

In some embodiments, the building block cap for RNA is m₂^{7,3'-O}Gppp(m₁^{2'-O})ApG (also sometimes referred to as m₂^{7,3'O}G(5')ppp(5')m^{2'-O}ApG), which has the following structure:

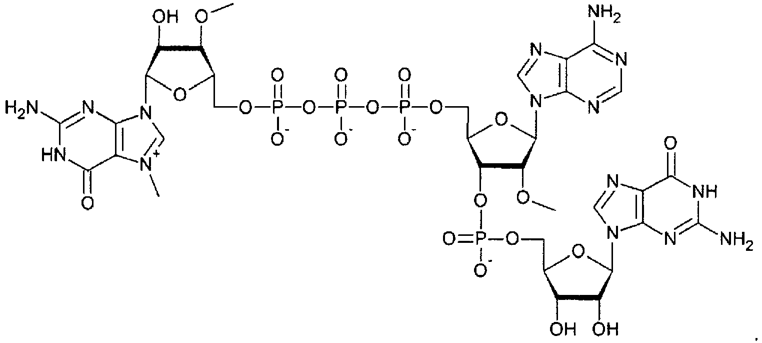

Below is an exemplary Cap1 RNA, which comprises RNA and m₂^{7,3'O}G(5')ppp(5')m^{2'-O}ApG:

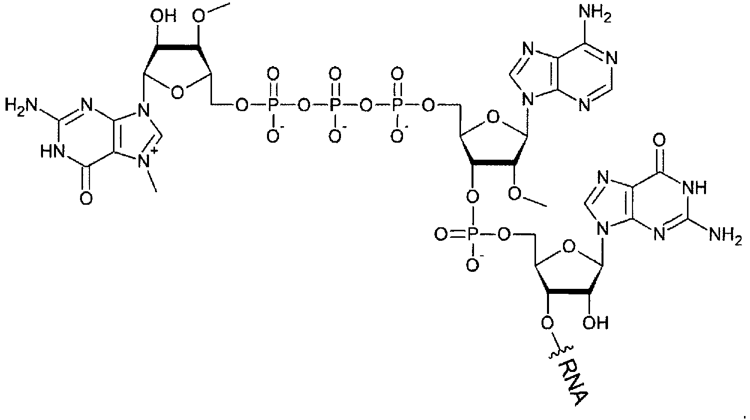

Below is another exemplary Cap1 RNA (no cap analog):

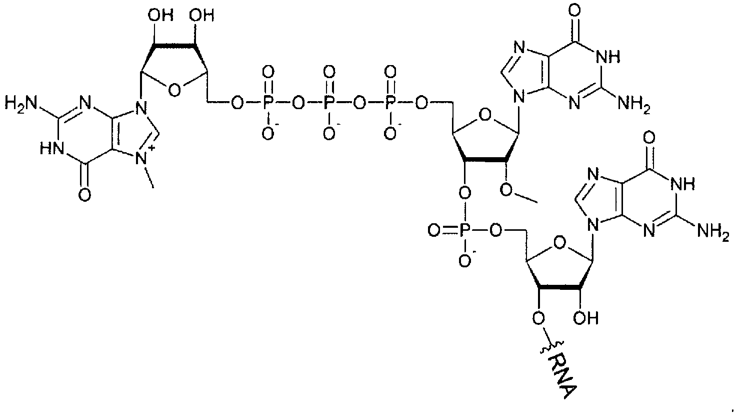

In some embodiments, the RNA is modified with "Cap0" structures using, in one embodiment, the cap analog anti-reverse cap (ARCA Cap (m₂^{7,3'O}G(5')ppp(5')G)) with the structure:

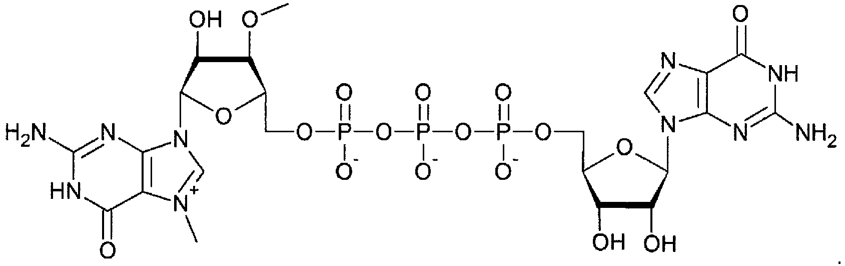

Below is an exemplary Cap0 RNA comprising RNA and m₂^{7,3'O}G(5')ppp(5')G:

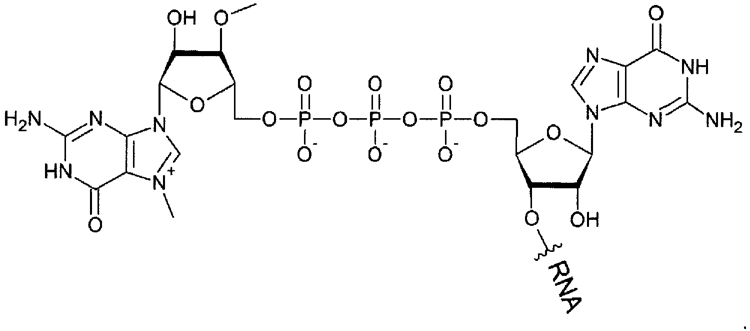

In some embodiments, the "Cap0" structures are generated using the cap analog Beta-S-ARCA (m₂^{7,2'O}G(5')ppSp(5')G) with the structure:

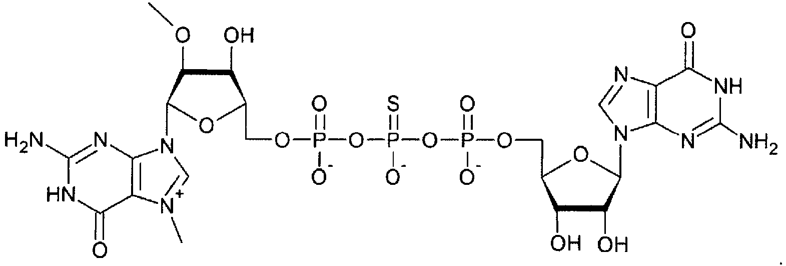

Below is an exemplary Cap0 RNA comprising Beta-S-ARCA (m₂^{7,2'O}G(5')ppSp(5')G) and RNA:

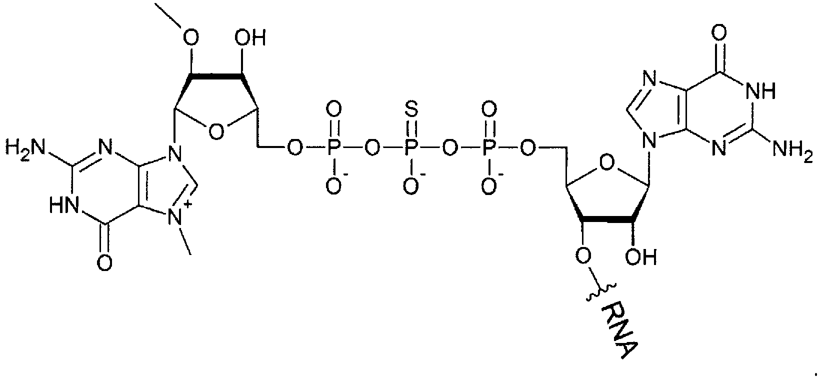

In some embodiments, RNA according to the present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5' end, upstream of the start codon of a protein-encoding region. A 5'-UTR is downstream of the 5'-cap (if present), e.g. directly adjacent to the 5'-cap. A 3'-UTR, if present, is located at the 3' end, downstream of the termination codon of a protein-encoding region, but the term "3'-UTR" does preferably not include the poly(A) sequence. Thus, the 3'-UTR is upstream of the poly(A) sequence (if present), e.g. directly adjacent to the poly(A) sequence.

In some embodiments, the RNA according to the present disclosure comprises a 3'-poly(A) sequence. As used herein, the term "poly(A) sequence" or "poly-A tail" refers to an uninterrupted or interrupted sequence of adenylate residues which is typically located at the 3' end of an RNA molecule. Poly(A) sequences are known to those of skill in the art and may follow the 3' UTR in the RNAs described herein. The poly(A) sequence may be of any length. In some embodiments, a poly(A) sequence comprises or consists of at least 20, at least 30, at least 40, at least 80, or at least 100 and up to 500, up to 400, up to 300, up to 200, or up to 150 nucleotides, and, in particular, about 110 nucleotides. In some embodiments, the poly(A) sequence only consists of A nucleotides. In some embodiments, the poly(A) sequence essentially consists of A nucleotides, but is interrupted by a random sequence of the four nucleotides (A, C, G, and U), as disclosed in WO 2016/005324 A1, hereby incorporated by reference. Such random sequence may be 5 to 50, 10 to 30, or 10 to 20 nucleotides in length. A poly(A) cassette present in the coding strand of DNA that essentially consists of dA nucleotides, but is interrupted by a random sequence having an equal distribution of the four nucleotides (dA, dC, dG, dT) and having a length of e.g. 5 to 50 nucleotides shows, on DNA level, constant propagation of plasmid DNA in *E. coli* and is still associated, on RNA level, with the beneficial properties with respect to supporting RNA stability and translational efficiency. In some embodiments, no nucleotides other than A nucleotides flank a poly(A) sequence at its 3' end, i.e., the poly(A) sequence is not masked or followed at its 3' end by a nucleotide other than A.

In the context of the present disclosure, the term "transcription" relates to a process, wherein the genetic code in a DNA sequence is transcribed into RNA. Subsequently, the RNA may be translated into peptide or protein.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence.

As used herein, the terms "linked," "fused", or "fusion" are used interchangeably. These terms refer to the joining together of two or more elements or components or domains.

### Cytokines

Cytokines are a category of small proteins (~5-20 kDa) that are important in cell signaling. Their release has an effect on the behavior of cells around them. Cytokines are involved in autocrine signaling, paracrine signaling and endocrine signaling as immunomodulating agents. Cytokines include chemokines, interferons, interleukins, lymphokines, and tumour necrosis factors but generally not hormones or growth factors (despite some overlap in the terminology). Cytokines are produced by a broad range of cells, including immune cells like macrophages, B lymphocytes, T lymphocytes and mast cells, as well as endothelial cells, fibroblasts, and various stromal cells. A given cytokine may be produced by more than one type of cell. Cytokines act through receptors, and are especially important in the immune system; cytokines modulate the balance between humoral and cell-based immune responses, and they regulate the maturation, growth, and responsiveness of particular cell populations. Some cytokines enhance or inhibit the action of other cytokines in complex ways.

### IL2

Interleukin-2 (IL2) is a cytokine that induces proliferation of antigen-activated T cells and stimulates natural killer (NK) cells. The biological activity of IL2 is mediated through a multi-subunit IL2 receptor complex (IL2R) of three polypeptide subunits that span the cell membrane: p55 (IL2Rα, the alpha subunit, also known as CD25 in humans), p75 (IL2Rβ, the beta subunit, also known as CD122 in humans) and p64 (IL2Rγ, the gamma subunit, also known as CD132 in humans). T cell response to IL2 depends on a variety of factors, including: (1) the concentration of IL2; (2) the number of IL2R molecules on the cell surface; and (3) the number of IL2R occupied by IL2 (i.e., the affinity of the binding interaction between IL2 and IL2R (Smith, "Cell Growth Signal Transduction is Quantal" In Receptor Activation by Antigens, Cytokines, Hormones, and Growth Factors 766:263-271, 1995)). The IL2:IL2R complex is internalized upon ligand binding and the different components undergo differential sorting. When administered as an intravenous (i.v.) bolus, IL2 has a rapid systemic clearance (an initial clearance phase with a half-life of 12.9 minutes followed by a slower clearance phase with a half-life of 85 minutes) (Konrad et al., Cancer Res. 50:2009-2017, 1990).

In eukaryotic cells human IL2 is synthesized as a precursor polypeptide of 153 amino acids, from which 20 amino acids are removed to generate mature secreted IL2. Recombinant human IL2 has been produced in E. coli, in insect cells and in mammalian COS cells.

According to the disclosure, IL2 (optionally as a portion of extended-PK IL2) may be naturally occurring IL2 or a fragment or variant thereof. IL2 may be human IL2 and may be derived from any vertebrate, especially any mammal. As used herein, "human IL2" or "wild type human IL2", whether native or recombinant, has the normally occurring 133 amino acid sequence of native human IL2 (less the signal peptide, consisting of an additional 20 N-terminal amino acids), whose amino acid sequence is described in Fujita, et. al, PNAS USA, 80, 7437-7441 (1983), with or without an additional N-terminal Methionine which is necessarily included when the protein is expressed as an intracellular fraction in E. coli.

In one embodiment, IL2 comprises the amino acid sequence of SEQ ID NO: 1 or 2. In one embodiment, a functional variant of IL2 comprises an amino acid sequence that is at least 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 1 or 2. In one embodiment, a functional variant of IL2 binds to the IL2 receptor or a subunit of the IL2 receptor such as the alpha subunit and/or the beta/gamma subunit. In general, for the purposes of this disclosure, the term "IL2" as used herein includes any polypeptide comprising a naturally occurring IL2 moiety or a functional variant thereof, unless contradicted by the circumstances.

According to the disclosure, in certain embodiments, IL2 is attached to a pharmacokinetic modifying group. The resulting molecule, hereafter referred to as "extended-pharmacokinetic (PK) IL2," has a prolonged circulation half-life relative to free IL2. The prolonged circulation half-life of extended-PK IL2 permits *in vivo* serum IL2 concentrations to be maintained within a therapeutic range, potentially leading to the enhanced activation of many types of immune cells, including T cells. Because of its favorable pharmacokinetic profile, extended-PK IL2 can be dosed less frequently and for longer periods of time when compared with unmodified IL2.

Accordingly, in certain embodiments described herein, the IL2 moiety is fused to a heterologous polypeptide (i.e., a polypeptide that is not IL2 and preferably is not a variant of IL2) and thus, is extended-PK IL2. In certain embodiments, the IL2 moiety of the extended-PK IL2 is human IL2. In other embodiments, the IL2 moiety of the extended-PK IL2 is a fragment or variant of human IL2. The heterologous polypeptide can increase the circulating half-life of IL2. As discussed in further detail infra, the polypeptide that increases the circulating half-life may be serum albumin, such as human or mouse serum albumin.

### IL15

In those embodiment disclosed herein involving the use of a polypeptide comprising IL2 or a functional variant thereof or a polynucleotide encoding a polypeptide comprising IL2 or a functional variant thereof, a polypeptide comprising IL15 or a functional variant thereof or a polynucleotide encoding a polypeptide comprising IL15 or a functional variant thereof may be used in addition to or instead of the polypeptide comprising IL2 or a functional variant thereof or the polynucleotide encoding a polypeptide comprising IL2 or a functional variant thereof.

Interleukin-15 (IL15) is a cytokine with structural similarity to Interleukin-2 (IL2). Like IL2, IL15 binds to and signals through a complex composed of IL-2/IL-15 receptor beta chain (CD122) and the common gamma chain (gamma-C, CD132). IL15 induces cell proliferation of natural killer cells.

### Extended-PK group

IL2 polypeptides described herein can be prepared as fusion or chimeric polypeptides that include an IL2 portion and a heterologous polypeptide (i.e., a polypeptide that is not IL2 or a variant thereof). The IL2 may be fused to an extended-PK group, which increases circulation half-life. Non-limiting examples of extended-PK groups are described infra. It should be understood that other PK groups that increase the circulation half-life of cytokines, or variants thereof, are also applicable to the present disclosure. In certain embodiments, the extended-PK group is a serum albumin domain (e.g., mouse serum albumin, human serum albumin).

As used herein, the term "PK" is an acronym for "pharmacokinetic" and encompasses properties of a compound including, by way of example, absorption, distribution, metabolism, and elimination by a subject. As used herein, an "extended-PK group" refers to a protein, peptide, or moiety that increases the circulation half-life of a biologically active molecule when fused to or administered together with the biologically active molecule. Examples of an extended-PK group include serum albumin (e.g., HSA), Immunoglobulin Fc or Fc fragments and variants thereof, transferrin and variants thereof, and human serum albumin (HSA) binders (as disclosed in U.S. Publication Nos. 2005/0287153 and 2007/0003549). Other exemplary extended-PK groups are disclosed in Kontermann, Expert Opin Biol Ther, 2016 Jul;16(7):903-15 which is herein incorporated by reference in its entirety. As used herein, an "extended-PK cytokine" refers to a cytokine moiety in combination with an extended-PK group. In one embodiment, the extended-PK cytokine is a fusion protein in which a cytokine moiety is linked or fused to an extended-PK group. As used herein, an "extended-PK IL" refers to an interleukin (IL) moiety (including an IL variant moiety) in combination with an extended-PK group. In one embodiment, the extended-PK IL is a fusion protein in which an IL moiety is linked or fused to an extended-PK group. An exemplary fusion protein is an HSA/IL2 fusion in which an IL2 moiety is fused with HSA.

In certain embodiments, the serum half-life of an extended-PK IL is increased relative to the IL alone (i.e., the IL not fused to an extended-PK group). In certain embodiments, the serum half-life of the extended-PK IL is at least 20, 40, 60, 80, 100, 120, 150, 180, 200, 400, 600, 800, or 1000% longer relative to the serum half-life of the IL alone. In certain embodiments, the serum half-life of the extended-PK IL is at least 1.5-fold, 2-fold, 2.5-fold, 3-fold, 3.5 fold, 4-fold, 4.5-fold, 5-fold, 6-fold, 7-fold, 8-fold, 10- fold, 12-fold, 13-fold, 15-fold, 17-fold, 20-fold, 22- fold, 25-fold, 27-fold, 30-fold, 35-fold, 40-fold, or 50-fold greater than the serum half-life of the IL alone. In certain embodiments, the serum half-life of the extended-PK IL is at least 10 hours, 15 hours, 20 hours, 25 hours, 30 hours, 35 hours, 40 hours, 50 hours, 60 hours, 70 hours, 80 hours, 90 hours, 100 hours, 110 hours, 120 hours, 130 hours, 135 hours, 140 hours, 150 hours, 160 hours, or 200 hours.

As used herein, "half-life" refers to the time taken for the serum or plasma concentration of a compound such as a peptide or protein to reduce by 50%, *in vivo,* for example due to degradation and/or clearance or sequestration by natural mechanisms. An extended-PK cytokine such as extended-PK interleukin (IL) suitable for use herein is stabilized *in vivo* and its half-life increased by, e.g., fusion to serum albumin (e.g., HSA or MSA), which resist degradation and/or clearance or sequestration. The half-life can be determined in any manner known per se, such as by pharmacokinetic analysis. Suitable techniques will be clear to the person skilled in the art, and may for example generally involve the steps of suitably administering a suitable dose of the amino acid sequence or compound to a subject; collecting blood samples or other samples from said subject at regular intervals; determining the level or concentration of the amino acid sequence or compound in said blood sample; and calculating, from (a plot of) the data thus obtained, the time until the level or concentration of the amino acid sequence or compound has been reduced by 50% compared to the initial level upon dosing. Further details are provided in, e.g., standard handbooks, such as Kenneth, A. et al., Chemical Stability of Pharmaceuticals: A Handbook for Pharmacists and in Peters et al., Pharmacokinetic Analysis: A Practical Approach (1996). Reference is also made to Gibaldi, M. et al., Pharmacokinetics, 2nd Rev. Edition, Marcel Dekker (1982).

In certain embodiments, the extended-PK group includes serum albumin, or fragments thereof or variants of the serum albumin or fragments thereof (all of which for the purpose of the present disclosure are comprised by the term "albumin"). Polypeptides described herein may be fused to albumin (or a fragment or variant thereof) to form albumin fusion proteins. Such albumin fusion proteins are described in U.S. Publication No. 20070048282.

As used herein, "albumin fusion protein" refers to a protein formed by the fusion of at least one molecule of albumin (or a fragment or variant thereof) to at least one molecule of a protein such as a therapeutic protein, in particular IL2 (or variant thereof). The albumin fusion protein may be generated by translation of a nucleic acid in which a polynucleotide encoding a therapeutic protein is joined in-frame with a polynucleotide encoding an albumin. The therapeutic protein and albumin, once part of the albumin fusion protein, may each be referred to as a "portion", "region" or "moiety" of the albumin fusion protein (e.g., a "therapeutic protein portion" or an "albumin protein portion"). In a highly preferred embodiment, an albumin fusion protein comprises at least one molecule of a therapeutic protein (including, but not limited to a mature form of the therapeutic protein) and at least one molecule of albumin (including but not limited to a mature form of albumin). In one embodiment, an albumin fusion protein is processed by a host cell such as a cell of the target organ for administered RNA, e.g. a liver cell, and secreted into the circulation. Processing of the nascent albumin fusion protein that occurs in the secretory pathways of the host cell used for expression of the RNA may include, but is not limited to signal peptide cleavage; formation of disulfide bonds; proper folding; addition and processing of carbohydrates (such as for example, N- and O-linked glycosylation); specific proteolytic cleavages; and/or assembly into multimeric proteins. An albumin fusion protein is preferably encoded by RNA in a non-processed form which in particular has a signal peptide at its N-terminus and following secretion by a cell is preferably present in the processed form wherein in particular the signal peptide has been cleaved off. In a most preferred embodiment, the "processed form of an albumin fusion protein" refers to an albumin fusion protein product which has undergone N-terminal signal peptide cleavage, herein also referred to as a "mature albumin fusion protein".

In preferred embodiments, albumin fusion proteins comprising a therapeutic protein have a higher plasma stability compared to the plasma stability of the same therapeutic protein when not fused to albumin. Plasma stability typically refers to the time period between when the therapeutic protein is administered *in vivo* and carried into the bloodstream and when the therapeutic protein is degraded and cleared from the bloodstream, into an organ, such as the kidney or liver, that ultimately clears the therapeutic protein from the body. Plasma stability is calculated in terms of the half-life of the therapeutic protein in the bloodstream. The half-life of the therapeutic protein in the bloodstream can be readily determined by common assays known in the art.

As used herein, "albumin" refers collectively to albumin protein or amino acid sequence, or an albumin fragment or variant, having one or more functional activities (e.g., biological activities) of albumin. In particular, "albumin" refers to human albumin or fragments or variants thereof especially the mature form of human albumin, or albumin from other vertebrates or fragments thereof, or variants of these molecules. The albumin may be derived from any vertebrate, especially any mammal, for example human, cow, sheep, or pig. Non-mammalian albumins include, but are not limited to, hen and salmon. The albumin portion of the albumin fusion protein may be from a different animal than the therapeutic protein portion.

In certain embodiments, the albumin is human serum albumin (HSA), or fragments or variants thereof, such as those disclosed in US 5,876,969, WO 2011/124718, WO 2013/075066, and WO 2011/0514789.

The terms, human serum albumin (HSA) and human albumin (HA) are used interchangeably herein. The terms, "albumin and "serum albumin" are broader, and encompass human serum albumin (and fragments and variants thereof) as well as albumin from other species (and fragments and variants thereof).

As used herein, a fragment of albumin sufficient to prolong the therapeutic activity or plasma stability of the therapeutic protein refers to a fragment of albumin sufficient in length or structure to stabilize or prolong the therapeutic activity or plasma stability of the protein so that the plasma stability of the therapeutic protein portion of the albumin fusion protein is prolonged or extended compared to the plasma stability in the non-fusion state.

The albumin portion of the albumin fusion proteins may comprise the full length of the albumin sequence, or may include one or more fragments thereof that are capable of stabilizing or prolonging the therapeutic activity or plasma stability. Such fragments may be of 10 or more amino acids in length or may include about 15, 20, 25, 30, 50, or more contiguous amino acids from the albumin sequence or may include part or all of specific domains of albumin. For instance, one or more fragments of HSA spanning the first two immunoglobulin-like domains may be used. In a preferred embodiment, the HSA fragment is the mature form of HSA.

Generally speaking, an albumin fragment or variant will be at least 100 amino acids long, preferably at least 150 amino acids long.

According to the disclosure, albumin may be naturally occurring albumin or a fragment or variant thereof. Albumin may be human albumin and may be derived from any vertebrate, especially any mammal. In one embodiment, albumin comprises the amino acid sequence of SEQ ID NO: 3 or 4 or an amino acid sequence that is at least 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 3 or 4.

Preferably, the albumin fusion protein comprises albumin as the N-terminal portion, and a therapeutic protein as the C-terminal portion. Alternatively, an albumin fusion protein comprising albumin as the C-terminal portion, and a therapeutic protein as the N-terminal portion may also be used. In other embodiments, the albumin fusion protein has a therapeutic protein fused to both the N-terminus and the C-terminus of albumin. In a preferred embodiment, the therapeutic proteins fused at the N- and C-termini are the same therapeutic proteins. In another preferred embodiment, the therapeutic proteins fused at the N- and C-termini are different therapeutic proteins. In one embodiment, the different therapeutic proteins are both cytokines.

In one embodiment, the therapeutic protein(s) is (are) joined to the albumin through (a) peptide linker(s). A linker peptide between the fused portions may provide greater physical separation between the moieties and thus maximize the accessibility of the therapeutic protein portion, for instance, for binding to its cognate receptor. The linker peptide may consist of amino acids such that it is flexible or more rigid. The linker sequence may be cleavable by a protease or chemically.

As used herein, the term "Fc region" refers to the portion of a native immunoglobulin formed by the respective Fc domains (or Fc moieties) of its two heavy chains. As used herein, the term "Fc domain" refers to a portion or fragment of a single immunoglobulin (Ig) heavy chain wherein the Fc domain does not comprise an Fv domain. In certain embodiments, an Fc domain begins in the hinge region just upstream of the papain cleavage site and ends at the C-terminus of the antibody. Accordingly, a complete Fc domain comprises at least a hinge domain, a CH2 domain, and a CH3 domain. In certain embodiments, an Fc domain comprises at least one of: a hinge (e.g., upper, middle, and/or lower hinge region) domain, a CH2 domain, a CH3 domain, a CH4 domain, or a variant, portion, or fragment thereof. In certain embodiments, an Fc domain comprises a complete Fc domain (i.e., a hinge domain, a CH2 domain, and a CH3 domain). In certain embodiments, an Fc domain comprises a hinge domain (or portion thereof) fused to a CH3 domain (or portion thereof). In certain embodiments, an Fc domain comprises a CH2 domain (or portion thereof) fused to a CH3 domain (or portion thereof). In certain embodiments, an Fc domain consists of a CH3 domain or portion thereof. In certain embodiments, an Fc domain consists of a hinge domain (or portion thereof) and a CH3 domain (or portion thereof). In certain embodiments, an Fc domain consists of a CH2 domain (or portion thereof) and a CH3 domain. In certain embodiments, an Fc domain consists of a hinge domain (or portion thereof) and a CH2 domain (or portion thereof). In certain embodiments, an Fc domain lacks at least a portion of a CH2 domain (e.g., all or part of a CH2 domain). An Fc domain herein generally refers to a polypeptide comprising all or part of the Fc domain of an immunoglobulin heavy-chain. This includes, but is not limited to, polypeptides comprising the entire CH1, hinge, CH2, and/or CH3 domains as well as fragments of such peptides comprising only, e.g., the hinge, CH2, and CH3 domain. The Fc domain may be derived from an immunoglobulin of any species and/or any subtype, including, but not limited to, a human IgG1, IgG2, IgG3, IgG4, IgD, IgA, IgE, or IgM antibody. The Fc domain encompasses native Fc and Fc variant molecules. As set forth herein, it will be understood by one of ordinary skill in the art that any Fc domain may be modified such that it varies in amino acid sequence from the native Fc domain of a naturally occurring immunoglobulin molecule. In certain embodiments, the Fc domain has reduced effector function (e.g., FcγR binding).

The Fc domains of a polypeptide described herein may be derived from different immunoglobulin molecules. For example, an Fc domain of a polypeptide may comprise a CH2 and/or CH3 domain derived from an IgG1 molecule and a hinge region derived from an IgG3 molecule. In another example, an Fc domain can comprise a chimeric hinge region derived, in part, from an IgG1 molecule and, in part, from an IgG3 molecule. In another example, an Fc domain can comprise a chimeric hinge derived, in part, from an IgG1 molecule and, in part, from an IgG4 molecule.

In certain embodiments, an extended-PK group includes an Fc domain or fragments thereof or variants of the Fc domain or fragments thereof (all of which for the purpose of the present disclosure are comprised by the term "Fc domain"). The Fc domain does not contain a variable region that binds to antigen. Fc domains suitable for use in the present disclosure may be obtained from a number of different sources. In certain embodiments, an Fc domain is derived from a human immunoglobulin. In certain embodiments, the Fc domain is from a human IgG1 constant region. It is understood, however, that the Fc domain may be derived from an immunoglobulin of another mammalian species, including for example, a rodent (e.g. a mouse, rat, rabbit, guinea pig) or non- human primate (e.g. chimpanzee, macaque) species.

Moreover, the Fc domain (or a fragment or variant thereof) may be derived from any immunoglobulin class, including IgM, IgG, IgD, IgA, and IgE, and any immunoglobulin isotype, including IgG1, IgG2, IgG3, and IgG4.

A variety of Fc domain gene sequences (e.g., mouse and human constant region gene sequences) are available in the form of publicly accessible deposits. Constant region domains comprising an Fc domain sequence can be selected lacking a particular effector function and/or with a particular modification to reduce immunogenicity. Many sequences of antibodies and antibody-encoding genes have been published and suitable Fc domain sequences (e.g. hinge, CH2, and/or CH3 sequences, or fragments or variants thereof) can be derived from these sequences using art recognized techniques.

In certain embodiments, the extended-PK group is a serum albumin binding protein such as those described in US2005/0287153, US2007/0003549, US2007/0178082, US2007/0269422, US201010113339, WO2009/083804, and WO2009/133208, which are herein incorporated by reference in their entirety. In certain embodiments, the extended-PK group is transferrin, as disclosed in US 7,176,278 and US 8,158,579, which are herein incorporated by reference in their entirety. In certain embodiments, the extended-PK group is a serum immunoglobulin binding protein such as those disclosed in US2007/0178082, US2014/0220017, and US2017/0145062, which are herein incorporated by reference in their entirety. In certain embodiments, the extended-PK group is a fibronectin (Fn)-based scaffold domain protein that binds to serum albumin, such as those disclosed in US2012/0094909, which is herein incorporated by reference in its entirety. Methods of making fibronectin-based scaffold domain proteins are also disclosed in US2012/0094909. A non-limiting example of a Fn3-based extended-PK group is Fn3(HSA), i.e., a Fn3 protein that binds to human serum albumin.

In certain aspects, the extended-PK IL, suitable for use according to the disclosure, can employ one or more peptide linkers. As used herein, the term "peptide linker" refers to a peptide or polypeptide sequence which connects two or more domains (e.g., the extended-PK moiety and an IL moiety such as IL2) in a linear amino acid sequence of a polypeptide chain. For example, peptide linkers may be used to connect an IL2 moiety to a HSA domain.

Linkers suitable for fusing the extended-PK group to e.g. IL2 are well known in the art. Exemplary linkers include glycine-serine-polypeptide linkers, glycine-proline-polypeptide linkers, and proline-alanine polypeptide linkers. In certain embodiments, the linker is a glycine-serine-polypeptide linker, i.e., a peptide that consists of glycine and serine residues.

In addition to, or in place of, the heterologous polypeptides described above, an IL2 variant polypeptide described herein can contain sequences encoding a "marker" or "reporter". Examples of marker or reporter genes include β-lactamase, chloramphenicol acetyltransferase (CAT), adenosine deaminase (ADA), aminoglycoside phosphotransferase, dihydrofolate reductase (DHFR), hygromycin-B-hosphotransferase (HPH), thymidine kinase (TK), β-galactosidase, and xanthine guanine phosphoribosyltransferase (XGPRT).

### Antigen

The term "antigen" relates to an agent comprising an epitope against which an immune response or an immune effector molecule such as antibody is directed and/or is to be directed. The term "antigen" includes, in particular, proteins and peptides. In one embodiment, an antigen is a disease-associated antigen, such as a tumor antigen.

The term "disease-associated antigen" is used in its broadest sense to refer to any antigen associated with a disease which preferably contains an epitope that will stimulate a host's immune system to make a cellular antigen-specific immune response and/or a humoral antibody response against the disease. The disease-associated antigen, an epitope thereof, or an agent targeting the disease-associated antigen or epitope may therefore be used for therapeutic purposes. Disease-associated antigens may be associated with infection by microbes, typically microbial antigens, or associated with cancer, typically tumors.

The term "tumor antigen" refers to a constituent of cancer cells which may be derived from the cytoplasm, the cell surface and the cell nucleus. In particular, it refers to those antigens which are produced intracellularly or as surface antigens on tumor cells. A tumor antigen is typically expressed preferentially by cancer cells (e.g., it is expressed at higher levels in cancer cells than in non-cancer cells) and in some instances it is expressed solely by cancer cells. Examples of tumor antigens include, without limitation, p53, ART-4, BAGE, beta-catenin/m, Bcr-abL CAMEL, CAP-1 , CASP-8, CDC27/m, CDK4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1, G250, GAGE, GnT-V, Gap 100, HAGE, HER-2/neu, HPV-E7, HPV-E6, HAST-2, hTERT (or hTRT), LAGE, LDLR/FUT, MAGE-A, preferably MAGE-A1 , MAGE-A2, MAGE- A3, MAGE-A4, MAGE- A5, MAGE-A6, MAGE-A7, MAGE-A8, MAGE-A9, MAGE-A 10, MAGE-A 1 1, or MAGE- A12, MAGE-B, MAGE-C, MART- 1 /Melan-A, MC1R, Myosin/m, MUC1 , MUM-1 , MUM - 2, MUM -3, NA88-A, NF1 , NY-ESO-1 , NY-BR-1 , pl90 minor BCR-abL, Pml/RARa, PRAME, proteinase 3, PSA, PSM, RAGE, RU1 or RU2, SAGE, SART-1 or SART-3, SCGB3A2, SCP1 , SCP2, SCP3, SSX, SURVIVIN, TEL/AML1 , TPI/m, TRP-1 , TRP-2, TRP-2/INT2, TPTE, WT, and WT-1.

The term "expressed on the cell surface", "associated with the cell surface" or a similar term means that a molecule such as an antigen is associated with and located at the plasma membrane of a cell, wherein at least a part of the molecule faces the extracellular space of said cell and is accessible from the outside of said cell, e.g., by antibodies located outside the cell. In this context, a part is preferably at least 4, preferably at least 8, preferably at least 12, more preferably at least 20 amino acids. The association may be direct or indirect. For example, the association may be by one or more transmembrane domains, one or more lipid anchors, or by the interaction with any other protein, lipid, saccharide, or other structure that can be found on the outer leaflet of the plasma membrane of a cell. For example, a molecule associated with the surface of a cell may be a transmembrane protein having an extracellular portion or may be a protein associated with the surface of a cell by interacting with another protein that is a transmembrane protein.

"Cell surface" or "surface of a cell" is used in accordance with its normal meaning in the art, and thus includes the outside of the cell which is accessible to binding by proteins and other molecules.

The term "extracellular portion" or "exodomain" in the context of the present invention refers to a part of a molecule such as a protein that is facing the extracellular space of a cell and preferably is accessible from the outside of said cell, e.g., by binding molecules such as antibodies located outside the cell. Preferably, the term refers to one or more extracellular loops or domains or a fragment thereof.

The term "epitope" refers to an antigenic determinant in a molecule, i.e., to a part or fragment of a molecule such as an antigen that is recognized by the immune system. For example, the epitope may be recognized by T cells, B cells or antibodies. An epitope of an antigen may include a continuous or discontinuous portion of the antigen and may be between about 5 and about 100, such as between about 5 and about 50, more preferably between about 8 and about 30, most preferably between about 10 and about 25 amino acids in length, for example, the epitope may be preferably 9, 10, 11, 12, 13, 14, 15, 16, 17, 18, 19, 20, 21, 22, 23, 24, or 25 amino acids in length. In one embodiment, an epitope is between about 10 and about 25 amino acids in length. The term "epitope" includes B cell epitopes and T cell epitopes.

The term "T cell epitope" refers to a part or fragment of a protein that is recognized by a T cell when presented in the context of MHC molecules. The term "major histocompatibility complex" and the abbreviation "MHC" includes MHC class I and MHC class II molecules and relates to a complex of genes which is present in all vertebrates. MHC proteins or molecules are important for signaling between lymphocytes and antigen presenting cells or diseased cells in immune reactions, wherein the MHC proteins or molecules bind peptide epitopes and present them for recognition by T cell receptors on T cells. The proteins encoded by the MHC are expressed on the surface of cells, and display both self-antigens (peptide fragments from the cell itself) and non-self-antigens (e.g., fragments of invading microorganisms) to a T cell. In the case of class I MHC/peptide complexes, the binding peptides are typically about 8 to about 10 amino acids long although longer or shorter peptides may be effective. In the case of class II MHC/peptide complexes, the binding peptides are typically about 10 to about 25 amino acids long and are in particular about 13 to about 18 amino acids long, whereas longer and shorter peptides may be effective.

### Therapeutic antibody

A "therapeutic antibody" is an antibody that can bind to an antigen, in particular a cell-surface antigen on a target cell, e.g., cancer cell, to cause a therapeutic effect. Therapeutic monoclonal antibodies have been conceived as a class of pharmaceutically active agents which should allow tumor selective treatment by targeting tumor selective antigens or epitopes. In preferred embodiments, the therapeutic antibodies target tumor or cancer antigens.

The term "antibody" refers to a glycoprotein comprising at least two heavy (H) chains and two light (L) chains inter-connected by disulfide bonds, and includes any molecule comprising an antigen binding portion thereof. The term "antibody" includes monoclonal antibodies and fragments or derivatives, i.e., constructs that are derived from an antibody, of antibodies, including, without limitation, human antibodies, humanized antibodies, chimeric antibodies, single chain antibodies, e.g., scFv's and antigen-binding antibody fragments such as Fab and Fab' fragments and also includes all recombinant forms of antibodies, e.g., antibodies expressed in prokaryotes, unglycosylated antibodies, and any antigen-binding antibody fragments and derivatives as described herein. Each heavy chain is comprised of a heavy chain variable region (abbreviated herein as VH) and a heavy chain constant region. Each light chain is comprised of a light chain variable region (abbreviated herein as VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The antibodies described herein may be human antibodies. The term "human antibody", as used herein, is intended to include antibodies having variable and constant regions derived from human germline immunoglobulin sequences. The human antibodies described herein may include amino acid residues not encoded by human germline immunoglobulin sequences (e.g., mutations introduced by random or site-specific mutagenesis *in vitro* or by somatic mutation *in vivo).*

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human, is less likely to elicit an immune response from a human subject when the antibodies are injected than would the constant region from a non human source. However the definition is not limited to this particular example.

The terms "antigen-binding portion" of an antibody (or simply "binding portion") or "antigen-binding fragment" of an antibody (or simply "binding fragment") or similar terms refer to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is binding-domain immunoglobulin fusion proteins comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies.

The term "bispecific molecule" is intended to include any agent, e.g., a protein, peptide, or protein or peptide complex, which has two different binding specificities. For example, the molecule may bind to, or interact with (a) a cell surface antigen, and (b) an Fc receptor on the surface of an effector cell. The term "multispecific molecule" or "heterospecific molecule" is intended to include any agent, e.g., a protein, peptide, or protein or peptide complex, which has more than two different binding specificities. For example, the molecule may bind to, or interact with (a) a cell surface antigen, (b) an Fc receptor on the surface of an effector cell, and (c) at least one other component. Accordingly, the invention includes, but is not limited to, bispecific, trispecific, tetraspecific, and other multispecific molecules which are directed to a tumor antigen, and to other targets, such as Fc receptors on effector cells. The term "bispecific antibodies" also includes multivalent antibodies, such as trivalent antibodies with two different binding specificities, tetravalent antibodies with two or three different binding specificities, and so on. The term "bispecific antibodies" also includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (see e.g. , Holliger, P., et al. (1993) Proc. Natl. Acad. Sci. USA 90: 6444-6448; Poljak, R. J., et al. (1994) Structure 2: 1121-1123).

An antibody may be conjugated to a therapeutic moiety or agent, such as a cytotoxin, a drug (e.g., an immunosuppressant) or a radioisotope. A cytotoxin or cytotoxic agent includes any agent that is detrimental to and, in particular, kills cells. Examples include maytansins (e.g. mertansine, ravtansine or emtanside), auristatins (Monomethyl auristatin F (MMAF), Monomethyl auristatin E (MMAE)), dolastatins, calicheamicins (e.g. ozogamicin), pyrrolobenzidiazepine dimers (e.g. tesirine, tairine), duocarmycins (e.g. Duocarmycin SA, CC-1065, duocarmazine) and α-amanitin, irinotecan or its derivative SN-38, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof, antimetabolites (e.g., methotrexate, 6-mercaptopurine, 6-thioguanine, cytarabine, fludarabin, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In yet another embodiment, the therapeutic agent is GM-CSF. In a preferred embodiment, the therapeutic agent is doxorubicin, cisplatin, bleomycin, sulfate, carmustine, chlorambucil, cyclophosphamide or ricin A.

Antibodies also can be conjugated to a radioisotope, e.g., iodine-131, yttrium-90 or indium-111, to generate cytotoxic radiopharmaceuticals.

The antibody conjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds. ), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pincheraet al. (eds. ), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62: 119-58 (1982).

As used herein, an antibody is "derived from" a particular germline sequence if the antibody is obtained from a system by immunizing an animal or by screening an immunoglobulin gene library, and wherein the selected antibody is at least 90%, more preferably at least 95%, even more preferably at least 96%, 97%, 98%, or 99% identical in amino acid sequence to the amino acid sequence encoded by the germline immunoglobulin gene. Typically, an antibody derived from a particular germline sequence will display no more than 10 amino acid differences, more preferably, no more than 5, or even more preferably, no more than 4, 3, 2, or 1 amino acid difference from the amino acid sequence encoded by the germline immunoglobulin gene.

As used herein, the term "heteroantibodies" refers to two or more antibodies, derivatives thereof, or antigen binding regions linked together, at least two of which have different specificities. These different specificities include a binding specificity for an Fc receptor on an effector cell, and a binding specificity for an antigen or epitope on a target cell, e.g., a tumor cell.

The antibodies described herein may be monoclonal antibodies. The term "monoclonal antibody" as used herein refers to a preparation of antibody molecules of single molecular composition. A monoclonal antibody displays a single binding specificity and affinity. In one embodiment, the monoclonal antibodies are produced by a hybridoma which includes a B cell obtained from a non-human animal, e.g., mouse, fused to an immortalized cell.

The antibodies described herein may be recombinant antibodies. The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

Antibodies described herein may be derived from different species, including but not limited to mouse, rat, rabbit, guinea pig and human.

Antibodies described herein include polyclonal and monoclonal antibodies and include IgA such as IgA1 or IgA2, IgG1, IgG2, IgG3, IgG4, IgE, IgM, and IgD antibodies. In various embodiments, the antibody is an IgG1 antibody, more particularly an IgG1, kappa or IgG1, lambda isotype (i.e. IgG1, κ, λ), an IgG2a antibody (e.g. IgG2a, κ, A), an IgG2b antibody (e.g. IgG2b, κ, A), an IgG3 antibody (e.g. IgG3, κ, λ) or an IgG4 antibody (e.g. IgG4, κ, λ).

The term "transfectoma", as used herein, includes recombinant eukaryotic host cells expressing an antibody, such as CHO cells, NS/0 cells, HEK293 cells, HEK293T cells, plant cells, or fungi, including yeast cells.

As used herein, a "heterologous antibody" is defined in relation to a transgenic organism producing such an antibody. This term refers to an antibody having an amino acid sequence or an encoding nucleic acid sequence corresponding to that found in an organism not consisting of the transgenic organism, and being generally derived from a species other than the transgenic organism.

As used herein, a "heterohybrid antibody" refers to an antibody having light and heavy chains of different organismal origins. For example, an antibody having a human heavy chain associated with a murine light chain is a heterohybrid antibody.

The invention includes all antibodies and derivatives of antibodies as described herein which for the purposes of the invention are encompassed by the term "antibody". The term "antibody derivatives" refers to any modified form of an antibody, e.g., a conjugate of the antibody and another agent or antibody, or an antibody fragment.

The antibodies described herein are preferably isolated. An "isolated antibody" as used herein, is intended to also include an antibody which is substantially free of other antibodies having different antigenic specificities (e.g., an isolated antibody that specifically binds to a tumor antigen is substantially free of antibodies that specifically bind antigens other than the tumor antigen). An isolated antibody that specifically binds to an epitope, isoform or variant of a human tumor antigen may, however, have cross-reactivity to other related antigens, e.g., from other species (e.g., species homologs of the tumor antigen).

The term "binding" according to the invention preferably relates to a specific binding.

According to the present invention, an antibody is capable of binding to a predetermined target if it has a significant affinity for said predetermined target and binds to said predetermined target in standard assays. "Affinity" or "binding affinity" is often measured by equilibrium dissociation constant (K_{D}). Preferably, the term "significant affinity" refers to the binding to a predetermined target with a dissociation constant (K_{D}) of 10⁻⁵ M or lower, 10⁻⁶ M or lower, 10⁻⁷ M or lower, 10⁻⁸ M or lower, 10⁻⁹ M or lower, 10⁻¹⁰ M or lower, 10⁻¹¹ M or lower, or 10⁻¹² M or lower.

An antibody is not (substantially) capable of binding to a target if it has no significant affinity for said target and does not bind significantly, in particular does not bind detectably, to said target in standard assays. Preferably, the antibody does not detectably bind to said target if present in a concentration of up to 2, preferably 10, more preferably 20, in particular 50 or 100 µg/ml or higher. Preferably, an antibody has no significant affinity for a target if it binds to said target with a K_{D} that is at least 10-fold, 100-fold, 10³-fold, 10⁴-fold, 10⁵-fold, or 10⁶-fold higher than the K_{D} for binding to the predetermined target to which the antibody is capable of binding. For example, if the K_{D} for binding of an antibody to the target to which the antibody is capable of binding is 10⁻⁷ M, the K_{D} for binding to a target for which the antibody has no significant affinity would be is at least 10⁻⁶ M, 10⁻⁵ M, 10⁻⁴ M, 10⁻³ M, 10⁻² M, or 10⁻¹ M.

An antibody is specific for a predetermined target if it is capable of binding to said predetermined target while it is not capable of binding to other targets, i.e. has no significant affinity for other targets and does not significantly bind to other targets in standard assays. According to the invention, an antibody is specific for a tumor antigen if it is capable of binding to the tumor antigen but is not (substantially) capable of binding to other targets. Preferably, an antibody is specific for a tumor antigen if the affinity for and the binding to such other targets does not significantly exceed the affinity for or binding to tumor antigen-unrelated proteins such as bovine serum albumin (BSA), casein, human serum albumin (HSA) or non-tumor antigen transmembrane proteins such as MHC molecules or transferrin receptor or any other specified polypeptide. Preferably, an antibody is specific for a predetermined target if it binds to said target with a K_{D} that is at least 10-fold, 100-fold, 10³-fold, 10⁴-fold, 10⁵-fold, or 10⁶-fold lower than the K_{D} for binding to a target for which it is not specific. For example, if the K_{D} for binding of an antibody to the target for which it is specific is 10⁻⁷ M, the K_{D} for binding to a target for which it is not specific would be at least 10⁻⁶ M, 10⁻⁵ M, 10⁻⁴ M, 10⁻³ M, 10⁻² M, or 10⁻¹ M.

Binding of an antibody to a target can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. Affinities may be readily determined using conventional techniques, such as by equilibrium dialysis; by using the BIAcore 2000 instrument, using general procedures outlined by the manufacturer; by radioimmunoassay using radiolabeled target antigen; or by another method known to the skilled artisan. The affinity data may be analyzed, for example, by the method of Scatchard et al., Ann N.Y. Acad. ScL, 51:660 (1949). The measured affinity of a particular antibody-antigen interaction can vary if measured under different conditions, e.g., salt concentration, pH. Thus, measurements of affinity and other antigen-binding parameters, e.g., K_{D}, IC₅₀, are preferably made with standardized solutions of antibody and antigen, and a standardized buffer.

As used herein, "isotype" refers to the antibody class (e.g., IgM or IgG1) that is encoded by heavy chain constant region genes.

As used herein, "isotype switching" refers to the phenomenon by which the class, or isotype, of an antibody changes from one Ig class to one of the other Ig classes.

The term "rearranged" as used herein refers to a configuration of a heavy chain or light chain immunoglobulin locus wherein a V segment is positioned immediately adjacent to a D-J or J segment in a conformation encoding essentially a complete VH or VL domain, respectively. A rearranged immunoglobulin (antibody) gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

According to the invention an anti-tumor antigen antibody is an antibody capable of binding to an epitope present in a tumor antigen, preferably an epitope located within the extracellular domains of a tumor antigen. According to the invention an anti-tumor antigen antibody preferably is an antibody specific for the tumor antigen. Preferably, an anti-tumor antigen antibody is an antibody binding to tumor antigen expressed on the cell surface. In particular preferred embodiments, an anti-tumor antigen antibody binds to native epitopes of a tumor antigen present on the surface of living cells. Preferably, the antibody binds to cancer cells, and does not bind substantially to non-cancerous cells. Preferably, binding of an anti-tumor antigen antibody to cells expressing tumor antigen induces or mediates killing of cells expressing tumor antigen. The cells expressing tumor antigen are preferably cancer cells. Preferably, the antibody induces or mediates killing of cells by inducing one or more of antibody dependent cellular cytotoxicity (ADCC) mediated lysis, antibody dependent cellular phagocytosis (ADCP) mediated lysis, complement dependent cytotoxicity (CDC) mediated lysis, apoptosis, and inhibition of proliferation of cells expressing tumor antigen.

In preferred embodiments, antibodies described herein can be characterized by one or more of the following properties:
a) specificity for tumor antigen;
b) a binding affinity to tumor antigen of about 100 nM or less, preferably, about 5-10 nM or less and, more preferably, about 1-3 nM or less,
c) the ability to induce or mediate ADCC on tumor antigen positive cells;
d) the ability to induce or mediate ADCP on tumor antigen positive cells;
e) the ability to induce or mediate CDC on tumor antigen positive cells;
f) the ability to inhibit the growth of tumor antigen positive cells;
g) the ability to induce apoptosis of tumor antigen positive cells.

The antibodies described herein preferably interact with components of the immune system, preferably through ADCC, ADCP or CDC. Antibodies described herein can also be used to target payloads (e.g., radioisotopes, drugs or toxins) to directly kill tumor cells or can be used with traditional chemotherapeutic agents, attacking tumors through complementary mechanisms of action that may include anti-tumor immune responses that may have been compromised owing to a chemotherapeutic's cytotoxic side effects on T lymphocytes. However, antibodies described herein may also exert an effect simply by binding to tumor antigen on the cell surface, thus, e.g. blocking proliferation of the cells.

### Antibody-dependent cell-mediated cytotoxicity (ADCC)

Antibody-dependent cell-mediated cytotoxicity (ADCC) is the killing of an antibody-coated target cell by a cytotoxic effector cell through a nonphagocytic process, characterised by the release of the content of cytotoxic granules or by the expression of cell death-inducing molecules. ADCC is independent of the immune complement system that also lyses targets but does not require any other cell. ADCC is triggered through interaction of target-bound antibodies (belonging to IgG or IgA or IgE classes) with certain Fc receptors (FcRs), glycoproteins present on the effector cell surface that bind the Fc region of immunoglobulins (Ig). Effector cells that mediate ADCC include natural killer (NK) cells, monocytes, macrophages, neutrophils, eosinophils and dendritic cells. ADCC is a rapid effector mechanism whose efficacy is dependent on a number of parameters (density and stability of the antigen on the surface of the target cell; antibody affinity and FcR-binding affinity). ADCC involving human IgG1, the most used IgG subclass for therapeutic antibodies, is highly dependent on the glycosylation profile of its Fc portion and on the polymorphism of Fcy receptors.

### Antibody-dependent cellular phagocytosis (ADCP)

ADCP is one crucial mechanism of action of many antibody therapies. It is defined as a highly regulated process by which antibodies eliminate bound targets *via* connecting its Fc domain to specific receptors on phagocytic cells, and eliciting phagocytosis. Unlike ADCC, ADCP can be mediated by monocytes, macrophages, neutrophils, and dendritic cells, through FcγRIIa, FcyRI, and FcγRIIIa, of which FcγRIIa (CD32a) on macrophages represent the predominant pathway.

### Complement-dependent cytotoxicity (CDC)

CDC is another cell-killing method that can be directed by antibodies. IgM is the most effective isotype for complement activation. IgG1 and IgG3 are also both very effective at directing CDC via the classical complement-activation pathway. Preferably, in this cascade, the formation of antigen-antibody complexes results in the uncloaking of multiple C1q binding sites in close proximity on the C_{H}2 domains of participating antibody molecules such as IgG molecules (C1q is one of three subcomponents of complement C1). Preferably these uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. Preferably, the complement cascade ends in the formation of a membrane attack complex, which creates pores in the cell membrane that facilitate free passage of water and solutes into and out of the cell.

Antibodies described herein can be produced by a variety of techniques, including conventional monoclonal antibody methodology, e.g., the standard somatic cell hybridization technique of Kohler and Milstein, Nature 256: 495 (1975). Although somatic cell hybridization procedures are preferred, in principle, other techniques for producing monoclonal antibodies can be employed, e.g., viral or oncogenic transformation of B-lymphocytes or phage display techniques using libraries of antibody genes.

The preferred animal system for preparing hybridomas that secrete monoclonal antibodies is the murine system. Hybridoma production in the mouse is a very well established procedure. Immunization protocols and techniques for isolation of immunized splenocytes for fusion are known in the art. Fusion partners (e.g., murine myeloma cells) and fusion procedures are also known.

Other preferred animal systems for preparing hybridomas that secrete monoclonal antibodies are the rat and the rabbit system (e.g. described in Spieker-Polet et al., Proc. Natl. Acad. Sci. U.S.A. 92:9348 (1995), see also Rossi et al., Am. J. Clin. Pathol. 124: 295 (2005)).

In yet another preferred embodiment, human monoclonal antibodies can be generated using transgenic or transchromosomal mice carrying parts of the human immune system rather than the mouse system. These transgenic and transchromosomic mice include mice known as HuMAb mice and KM mice, respectively, and are collectively referred to herein as "transgenic mice." The production of human antibodies in such transgenic mice can be performed as described in detail for CD20 in WO2004 035607 Yet another strategy for generating monoclonal antibodies is to directly isolate genes encoding antibodies from lymphocytes producing antibodies of defined specificity e.g. see Babcock et al., 1996; A novel strategy for generating monoclonal antibodies from single, isolated lymphocytes producing antibodies of defined specificities. For details of recombinant antibody engineering see also Welschof and Kraus, Recombinant antibodes for cancer therapy ISBN-0-89603-918-8 and Benny K.C. Lo Antibody Engineering ISBN 1-58829-092-1.

To generate antibodies, mice can be immunized with carrier-conjugated peptides derived from the antigen sequence, i.e. the sequence against which the antibodies are to be directed, an enriched preparation of recombinantly expressed antigen or fragments thereof and/or cells expressing the antigen, as described. Alternatively, mice can be immunized with DNA encoding the antigen or fragments thereof. In the event that immunizations using a purified or enriched preparation of the antigen do not result in antibodies, mice can also be immunized with cells expressing the antigen, e.g., a cell line, to promote immune responses.

The immune response can be monitored over the course of the immunization protocol with plasma and serum samples being obtained by tail vein or retroorbital bleeds. Mice with sufficient titers of immunoglobulin can be used for fusions. Mice can be boosted intraperitonealy or intravenously with antigen expressing cells 3 days before sacrifice and removal of the spleen to increase the rate of specific antibody secreting hybridomas.

To generate hybridomas producing monoclonal antibodies, splenocytes and lymph node cells from immunized mice can be isolated and fused to an appropriate immortalized cell line, such as a mouse myeloma cell line. The resulting hybridomas can then be screened for the production of antigen-specific antibodies. Individual wells can then be screened by ELISA for antibody secreting hybridomas. By Immunofluorescence and FACS analysis using antigen expressing cells, antibodies with specificity for the antigen can be identified. The antibody secreting hybridomas can be replated, screened again, and if still positive for monoclonal antibodies can be subcloned by limiting dilution. The stable subclones can then be cultured *in vitro* to generate antibody in tissue culture medium for characterization.

Antibodies also can be produced in a host cell transfectoma using, for example, a combination of recombinant DNA techniques and gene transfection methods as are well known in the art (Morrison, S. (1985) Science 229: 1202).

For example, in one embodiment, the gene(s) of interest, e.g., antibody genes, can be ligated into an expression vector such as a eukaryotic expression plasmid such as used by the GS gene expression system disclosed in WO 87/04462, WO 89/01036 and EP 338 841 or other expression systems well known in the art. The purified plasmid with the cloned antibody genes can be introduced in eukaryotic host cells such as CHO cells, NS/0 cells, HEK293T cells or HEK293 cells or alternatively other eukaryotic cells like plant derived cells, fungal or yeast cells. The method used to introduce these genes can be methods described in the art such as electroporation, lipofectine, lipofectamine or others. After introduction of these antibody genes in the host cells, cells expressing the antibody can be identified and selected. These cells represent the transfectomas which can then be amplified for their expression level and upscaled to produce antibodies. Recombinant antibodies can be isolated and purified from these culture supernatants and/or cells.

Alternatively, the cloned antibody genes can be expressed in other expression systems, including prokaryotic cells, such as microorganisms, e.g. E. coli. Furthermore, the antibodies can be produced in transgenic non-human animals, such as in milk from sheep and rabbits or in eggs from hens, or in transgenic plants; see e.g. Verma, R., et al. (1998) J. Immunol. Meth. 216: 165-181; Pollock, et al. (1999) J. Immunol. Meth. 231: 147-157; and Fischer, R., et al. (1999) Biol. Chem. 380: 825-839.

### Chimerization

Murine monoclonal antibodies can be used as therapeutic antibodies in humans when labeled with toxins or radioactive isotopes. Nonlabeled murine antibodies are highly immunogenic in man when repetitively applied leading to reduction of the therapeutic effect. The main immunogenicity is mediated by the heavy chain constant regions. The immunogenicity of murine antibodies in man can be reduced or completely avoided if respective antibodies are chimerized or humanized. Chimeric antibodies are antibodies, the different portions of which are derived from different animal species, such as those having a variable region derived from a murine antibody and a human immunoglobulin constant region. Chimerisation of antibodies is achieved by joining of the variable regions of the murine antibody heavy and light chain with the constant region of human heavy and light chain (e.g. as described by Kraus et al., in Methods in Molecular Biology series, Recombinant antibodies for cancer therapy ISBN-0-89603-918-8). In a preferred embodiment chimeric antibodies are generated by joining human kappa-light chain constant region to murine light chain variable region. In an also preferred embodiment chimeric antibodies can be generated by joining human lambda-light chain constant region to murine light chain variable region. The preferred heavy chain constant regions for generation of chimeric antibodies are IgG1, IgG3 and IgG4.

Other preferred heavy chain constant regions for generation of chimeric antibodies are IgG2, IgA, IgD and IgM.

### Humanization

Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332: 323-327; Jones, P. et al. (1986) Nature 321: 522-525; and Queen, C. et al. (1989) Proc. Natl. Acad. Sci. U. S. A. 86: 10029-10033). Such framework sequences can be obtained from public DNA databases that include germline antibody gene sequences. These germline sequences will differ from mature antibody gene sequences because they will not include completely assembled variable genes, which are formed by V (D) J joining during B cell maturation. Germline gene sequences will also differ from the sequences of a high affinity secondary repertoire antibody at individual evenly across the variable region.

The ability of antibodies to bind an antigen can be determined using standard binding assays (e.g., ELISA, Western Blot, Immunofluorescence and flow cytometric analysis).

To purify antibodies, selected hybridomas can be grown in two-liter spinner-flasks for monoclonal antibody purification. Alternatively, antibodies can be produced in dialysis based bioreactors. Supernatants can be filtered and, if necessary, concentrated before affinity chromatography with protein G-sepharose or protein A-sepharose. Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80°C.

To determine if the selected monoclonal antibodies bind to unique epitopes, site-directed or multi-site directed mutagenesis can be used.

To determine the isotype of antibodies, isotype ELISAs with various commercial kits (e.g. Zymed, Roche Diagnostics) can be performed. Wells of microtiter plates can be coated with anti-mouse Ig. After blocking, the plates are reacted with monoclonal antibodies or purified isotype controls, at ambient temperature for two hours. The wells can then be reacted with either mouse IgG1, IgG2a, IgG2b or IgG3, IgA or mouse IgM-specific peroxidase-conjugated probes. After washing, the plates can be developed with ABTS substrate (1 mg/ml) and analyzed at OD of 405-650. Alternatively, the IsoStrip Mouse Monoclonal Antibody Isotyping Kit (Roche, Cat. No. 1493027) may be used as described by the manufacturer.

In order to demonstrate presence of antibodies in sera of immunized mice or binding of monoclonal antibodies to living cells expressing antigen, flow cytometry can be used. Cell lines expressing naturally or after transfection antigen and negative controls lacking antigen expression (grown under standard growth conditions) can be mixed with various concentrations of monoclonal antibodies in hybridoma supernatants or in PBS containing 1% FBS, and can be incubated at 4°C for 30 min. After washing, the APC- or Alexa647-labeled anti IgG antibody can bind to antigen-bound monoclonal antibody under the same conditions as the primary antibody staining. The samples can be analyzed by flow cytometry with a FACS instrument using light and side scatter properties to gate on single, living cells. In order to distinguish antigen-specific monoclonal antibodies from non-specific binders in a single measurement, the method of co-transfection can be employed. Cells transiently transfected with plasmids encoding antigen and a fluorescent marker can be stained as described above. Transfected cells can be detected in a different fluorescence channel than antibody-stained cells. As the majority of transfected cells express both transgenes, antigen-specific monoclonal antibodies bind preferentially to fluorescence marker expressing cells, whereas non-specific antibodies bind in a comparable ratio to non-transfected cells. An alternative assay using fluorescence microscopy may be used in addition to or instead of the flow cytometry assay. Cells can be stained exactly as described above and examined by fluorescence microscopy.

In order to demonstrate presence of antibodies in sera of immunized mice or binding of monoclonal antibodies to living cells expressing antigen, immunofluorescence microscopy analysis can be used. For example, cell lines expressing either spontaneously or after transfection antigen and negative controls lacking antigen expression are grown in chamber slides under standard growth conditions in DMEM/F12 medium, supplemented with 10 % fetal calf serum (FCS), 2 mM L-glutamine, 100 IU/ml penicillin and 100 µg/ml streptomycin. Cells can then be fixed with methanol or paraformaldehyde or left untreated. Cells can then be reacted with monoclonal antibodies against the antigen for 30 min. at 25°C. After washing, cells can be reacted with an Alexa555-labelled anti-mouse IgG secondary antibody (Molecular Probes) under the same conditions. Cells can then be examined by fluorescence microscopy.

Cell extracts from cells expressing antigen and appropriate negative controls can be prepared and subjected to sodium dodecyl sulfate (SDS) polyacrylamide gel electrophoresis. After electrophoresis, the separated antigens will be transferred to nitrocellulose membranes, blocked, and probed with the monoclonal antibodies to be tested. IgG binding can be detected using anti-mouse IgG peroxidase and developed with ECL substrate.

Antibodies can be further tested for reactivity with antigen by Immunohistochemistry in a manner well known to the skilled person, e.g. using paraformaldehyde or acetone fixed cryosections or paraffin embedded tissue sections fixed with paraformaldehyde from non-cancer tissue or cancer tissue samples obtained from patients during routine surgical procedures or from mice carrying xenografted tumors inoculated with cell lines expressing spontaneously or after transfection antigen. For immunostaining, antibodies reactive to antigen can be incubated followed by horseradish-peroxidase conjugated goat anti-mouse or goat anti-rabbit antibodies (DAKO) according to the vendors instructions.

Antibodies can be tested for their ability to mediate phagocytosis and killing of cells expressing tumor antigen. The testing of monoclonal antibody activity *in vitro* will provide an initial screening prior to testing *in vivo* models.

### Antibody-dependent cell-mediated cytotoxicity (ADCC):

Briefly, polymorphonuclear cells (PMNs), NK cells, monocytes, mononuclear cells or other effector cells, from healthy donors can be purified by Ficoll Hypaque density centrifugation, followed by lysis of contaminating erythrocytes. Washed effector cells can be suspended in RPMI supplemented with 10% heat-inactivated fetal calf serum or, alternatively with 5% heat-inactivated human serum and mixed with ⁵¹Cr labeled target cells expressing tumor antigen, at various ratios of effector cells to target cells. Alternatively, the target cells may be labeled with a fluorescence enhancing ligand (BATDA). A highly fluorescent chelate of Europium with the enhancing ligand which is released from dead cells can be measured by a fluorometer. Another alternative technique may utilize the transfection of target cells with luciferase. Added lucifer yellow may then be oxidated by viable cells only. Purified anti-tumor antigen IgGs can then be added at various concentrations. Irrelevant human IgG can be used as negative control. Assays can be carried out for 4 to 20 hours at 37°C depending on the effector cell type used. Samples can be assayed for cytolysis by measuring ⁵¹Cr release or the presence of the EuTDA chelate in the culture supernatant. Alternatively, luminescence resulting from the oxidation of lucifer yellow can be a measure of viable cells.

Anti-tumor antigen monoclonal antibodies can also be tested in various combinations to determine whether cytolysis is enhanced with multiple monoclonal antibodies.

### Antibody-dependent cellular phagocytosis (ADCP):

Briefly, a classic ADCP assay format is based on using peripheral blood mononuclear cell (PBMC)-derived macrophages as effector cells. After extraction of fresh human PBMCs, monocytes can be isolated and differentiated in culture to macrophages. Phagocytosis events can be analyzed using FACS screening, and a dose-dependent curve can be generated to assess the ADCP potency in detail. Effector cells from distinct donors cannot be pooled due to MHC restrictions. Hence, samples from a battery of donors must be applied to reduce donor-specific variability.

### Complement dependent cytotoxicity (CDC):

Monoclonal anti-tumor antigen antibodies can be tested for their ability to mediate CDC using a variety of known techniques. For example, serum for complement can be obtained from blood in a manner known to the skilled person. To determine the CDC activity of mAbs, different methods can be used. ⁵¹Cr release can for example be measured or elevated membrane permeability can be assessed using a propidium iodide (PI) exclusion assay. Briefly, target cells can be washed and 5 x 10⁵/ml can be incubated with various concentrations of mAb for 10-30 min. at room temperature or at 37°C. Serum or plasma can then be added to a final concentration of 20% (v/v) and the cells incubated at 37°C for 20-30 min. All cells from each sample can be added to the PI solution in a FACS tube. The mixture can then be analyzed immediately by flow cytometry analysis using FACSArray.

In an alternative assay, induction of CDC can be determined on adherent cells. In one embodiment of this assay, cells are seeded 24 h before the assay with a density of 3 x 10⁴/well in tissue-culture flat-bottom microtiter plates. The next day growth medium is removed and the cells are incubated in triplicates with antibodies. Control cells are incubated with growth medium or growth medium containing 0.2% saponin for the determination of background lysis and maximal lysis, respectively. After incubation for 20 min. at room temperature supernatant is removed and 20% (v/v) human plasma or serum in DMEM (prewarmed to 37°C) is added to the cells and incubated for another 20 min. at 37°C. All cells from each sample are added to propidium iodide solution (10 µg/ml). Then, supernatants are replaced by PBS containing 2.5 µg/ml ethidium bromide and fluorescence emission upon excitation at 520 nm is measured at 600 nm using a Tecan Safire. The percentage specific lysis is calculated as follows: % specific lysis = (fluorescence sample-fluorescence background)/ (fluorescence maximal lysis-fluorescence background) x 100.

### Induction of apoptosis and inhibition of cell proliferation by monoclonal antibodies:

To test for the ability to initiate apoptosis, monoclonal anti-tumor antigen antibodies can, for example, be incubated with tumor antigen positive tumor cells, or tumor antigen transfected tumor cells at 37°C for about 20 hours. The cells can be harvested, washed in Annexin-V binding buffer (BD biosciences), and incubated with Annexin V conjugated with FITC or APC (BD biosciences) for 15 min. in the dark. All cells from each sample can be added to PI solution (10 µg/ml in PBS) in a FACS tube and assessed immediately by flow cytometry (as above). Alternatively, a general inhibition of cell-proliferation by monoclonal antibodies can be detected with commercially available kits. The DELFIA Cell Proliferation Kit (Perkin-Elmer, Cat. No. AD0200) is a non-isotopic immunoassay based on the measurement of 5-bromo-2'-deoxyuridine (BrdU) incorporation during DNA synthesis of proliferating cells in microplates. Incorporated BrdU is detected using europium labelled monoclonal antibody, To allow antibody detection, cells are fixed and DNA denatured using Fix solution. Unbound antibody is washed away and DELFIA inducer is added to dissociate europium ions from the labelled antibody into solution, where they form highly fluorescent chelates with components of the DELFIA Inducer. The fluorescence measured - utilizing time-resolved fluorometry in the detection - is proportional to the DNA synthesis in the cell of each well.

### Preclinical studies

Monoclonal antibodies which bind to tumor antigen also can be tested in an *in vivo* model (e.g. in immune deficient mice carrying xenografted tumors inoculated with cell lines expressing tumor antigen, or after transfection, e.g. HEK293) to determine their efficacy in controlling growth of tumor antigen-expressing tumor cells.

*In vivo* studies after xenografting tumor antigen expressing tumor cells into immunocompromised mice or other animals can be performed using antibodies described herein. Antibodies can be administered to tumor free mice followed by injection of tumor cells to measure the effects of the antibodies to prevent formation of tumors or tumor-related symptoms. Antibodies can be administered to tumor-bearing mice to determine the therapeutic efficacy of respective antibodies to reduce tumor growth, metastasis or tumor related symptoms. Antibody application can be combined with application of other substances as immune checkpoint inhibitors, cystostatic drugs, growth factor inhibitors, cell cycle blockers, angiogenesis inhibitors or other antibodies to determine efficacy and potential toxicity of combinations. To analyze toxic side effects mediated by antibodies animals can be inoculated with antibodies or control reagents and thoroughly investigated for symptoms possibly related to tumor antigen-antibody therapy. Possible side effects of *in vivo* application of tumor antigen antibodies particularly include toxicity at tumor antigen expressing tissues including stomach. Antibodies recognizing tumor antigen in human and in other species, e.g. mice, are particularly useful to predict potential side effects mediated by application of monoclonal tumor antigen-antibodies in humans.

Mapping of epitopes recognized by antibodies can be performed as described in detail in "Epitope Mapping Protocols (Methods in Molecular Biology) by Glenn E. Morris ISBN-089603-375-9 and in "Epitope Mapping: A Practical Approach" Practical Approach Series, 248 by Olwyn M. R. Westwood, Frank C. Hay.

As used herein, "tumor antigen" or "cancer antigen" includes (i) tumor-specific antigens, (ii) tumor-associated antigens, (iii) embryonic antigens on tumors, (iv) tumor-specific membrane antigens, (v) tumor-associated membrane antigens, (vi) growth factor receptors, and (xi) any other type of antigen or material that is associated with a cancer.

Therapeutic antibodies that can be used according to the present invention include, but are not limited to, any of the art-recognized anti-cancer antibodies that are approved for use, in clinical trials, or in development for clinical use. In certain embodiments, more than one anticancer antibody can be included in the combination therapy of the present invention.

For example, the following tumor antigens can be targeted by therapeutic antibodies disclosed herein.

The tumor antigen may be an epithelial cancer antigen, (e.g., breast, gastrointestinal, lung), a prostate specific cancer antigen (PSA) or prostate specific membrane antigen (PSMA), a bladder cancer antigen, a lung (e.g., small cell lung) cancer antigen, a colon cancer antigen, an ovarian cancer antigen, a brain cancer antigen, a gastric cancer antigen, a renal cell carcinoma antigen, a pancreatic cancer antigen, a liver cancer antigen, an esophageal cancer antigen, a head and neck cancer antigen, or a colorectal cancer antigen. In certain embodiments, the tumor antigen is a lymphoma antigen (e.g., non-Hodgkin's lymphoma or Hodgkin's lymphoma), a B- cell lymphoma cancer antigen, a leukemia antigen, a myeloma (e.g., multiple myeloma or plasma cell myeloma) antigen, an acute lymphoblastic leukemia antigen, a chronic myeloid leukemia antigen, or an acute myelogenous leukemia antigen. It should be understood that the described tumor antigens are only exemplary and that any tumor antigen can be targeted according to the invention.

Tumor antigens are well known in the art and include those described herein. The tumor antigen may be, for example, a glioma-associated antigen, carcinoembryonic antigen (CEA), β-human chorionic gonadotropin, alphafetoprotein (AFP), lectin-reactive AFP, thyroglobulm, RAGE-1, MN-CA IX, human telomerase reverse transcriptase, RU1, RU2 (AS), intestinal carboxy esterase, mut hsp70-2, M-CSF, prostase, prostate-specific antigen (PSA), PAP, NY-ESO-1, LAGE-la, p53, tyrosinase, prostein, PSMA, ras, Her2/neu, TRP-1, TRP-2, TAG-72, KSA, CA-125, PSA, BRCI, BRC-II, bcr-abl, pax3-fkhr, ews-fli-I, survivin and telomerase, prostate-carcinoma tumor antigen-1 (PCTA-1), MAGE, GAGE, GP-100, MUC-1, MUC-2, ELF2M, neutrophil elastase, ephrinB2, CD22, insulin growth factor (IGF)-I, IGF-II, IGF-I receptor, VEGF, Claudin molecules such as Claudin 18 Isoform 2 and Claudin 6, and mesothelin,

The tumor antigen may be a unique antigen (usually caused by mutations) (e.g., p53, ras, β-catenin, CDK4, CDC27, α actinin-4), a differentiation antigen (e.g., tyrosinase, TRP1/gp75, TRP2, gp100, Melan-A/MART1, gangliosides, PSMA), an overexpressed antigen (e.g., HER2, WT1, EphA3, EGFR, CD20), a cancer-testis antigen (e.g., MAGE, BAGE, GAGE, NY-ESO-1) or a universal antigen (e.g., telomerase, surviving)

The tumor antigen may also be a tumor-specific antigen (TSA) or a tumor-associated antigen (TAA). A TSA is unique to tumor cells and does not occur on other cells in the body. A TAA is not unique to a tumor cell and instead is also expressed on a normal cell under conditions that fail to induce a state of immunologic tolerance to the antigen. The expression of the antigen on the tumor may occur under conditions that enable the immune system to respond to the antigen. TAAs may be antigens that are expressed on normal cells during fetal development when the immune system is immature and unable to respond or they may be antigens that are normally present at extremely low levels on normal cells but which are expressed at much higher levels on tumor cells.

Non-limiting examples of anti-cancer antibodies include the following, without limitation:
trastuzumab (HERCEPTIN^{™}; target: HER2/neu), which is used to treat HER-2/neu positive breast cancer or metastatic breast cancer;
bevacizumab (AVASTIN^{™}; target: VEGF-A), which is used to treat colorectal cancer, metastatic colorectal cancer, breast cancer, metastatic breast cancer, non-small cell lung cancer, or renal cell carcinoma;
rituximab (RITUXAN^{™}; target: CD20), which is used to treat non-Hodgkin's lymphoma or chronic lymphocytic leukemia;
pertuzumab (OMNITARG^{™}; target: HER2/neu), which is used to treat breast cancer, prostate cancer, non-small cell lung cancer, or ovarian cancer;
cetuximab (ERBITUX^{™}; target: EGFR), which can be used to treat colorectal cancer, metastatic colorectal cancer, lung cancer, head and neck cancer, colon cancer, breast cancer, prostate cancer, gastric cancer, ovarian cancer, brain cancer, pancreatic cancer, esophageal cancer, renal cell cancer, prostate cancer, cervical cancer, or bladder cancer;
tositumomab (BEXXAR^{™}; target: CD20), which is used to treat non-Hodgkin's lymphoma, and follicular, non-Hodgkin's lymphoma;
ofatumumab (ARZERRA^{™}; target: CD20) for chromic lymphocytic leukemia;
panitumumab (VECTIBIX^{™}; target: EGFR) for colorectal cancer;
alemtuzumab (CAMPATH^{™}; target: CD52) for chronic lymphocytic leukemia;
obinutuzumab (Gazyva ^{™}; target: CD20) for chronic lymphatic leukemia;

### Chemotherapy

In addition to a polypeptide comprising IL2 or a functional variant thereof or a polynucleotide encoding a polypeptide comprising IL2 or a functional variant thereof; and antibody-based immunotherapy against cancer additional treatments may be administered to a patient. Such additional treatments includes classical cancer therapy, e.g., radiation therapy, surgery, hyperthermia therapy and/or chemotherapy.

Chemotherapy is a type of cancer treatment that uses one or more anti-cancer drugs (chemotherapeutic agents), usually as part of a standardized chemotherapy regimen. The term chemotherapy has come to connote non-specific usage of intracellular poisons to inhibit mitosis. The connotation excludes more selective agents that block extracellular signals (signal transduction). The development of therapies with specific molecular or genetic targets, which inhibit growth-promoting signals from classic endocrine hormones (primarily estrogens for breast cancer and androgens for prostate cancer) are now called hormonal therapies. By contrast, other inhibitions of growth-signals like those associated with receptor tyrosine kinases are referred to as targeted therapy.

Importantly, the use of drugs (whether chemotherapy, hormonal therapy or targeted therapy) constitutes systemic therapy for cancer in that they are introduced into the blood stream and are therefore in principle able to address cancer at any anatomic location in the body. Systemic therapy is often used in conjunction with other modalities that constitute local therapy (i.e. treatments whose efficacy is confined to the anatomic area where they are applied) for cancer such as radiation therapy, surgery or hyperthermia therapy.

Traditional chemotherapeutic agents are cytotoxic by means of interfering with cell division (mitosis) but cancer cells vary widely in their susceptibility to these agents. To a large extent, chemotherapy can be thought of as a way to damage or stress cells, which may then lead to cell death if apoptosis is initiated.

Chemotherapeutic agents include alkylating agents, antimetabolites, anti-microtubule agents, topoisomerase inhibitors, and cytotoxic antibiotics.

Alkylating agents have the ability to alkylate many molecules, including proteins, RNA and DNA. The subtypes of alkylating agents are the nitrogen mustards, nitrosoureas, tetrazines, aziridines, cisplatins and derivatives, and non-classical alkylating agents. Nitrogen mustards include mechlorethamine, cyclophosphamide, melphalan, chlorambucil, ifosfamide and busulfan. Nitrosoureas include N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide. Aziridines include thiotepa, mytomycin and diaziquone (AZQ). Cisplatin and derivatives include cisplatin, carboplatin and oxaliplatin. They impair cell function by forming covalent bonds with the amino, carboxyl, sulfhydryl, and phosphate groups in biologically important molecules. Non-classical alkylating agents include procarbazine and hexamethylmelamine. In one particularly preferred embodiment, the alkylating agent is cyclophosphamide.

Anti-metabolites are a group of molecules that impede DNA and RNA synthesis. Many of them have a similar structure to the building blocks of DNA and RNA. Anti-metabolites resemble either nucleobases or nucleosides, but have altered chemical groups. These drugs exert their effect by either blocking the enzymes required for DNA synthesis or becoming incorporated into DNA or RNA. Subtypes of the anti-metabolites are the anti-folates, fluoropyrimidines, deoxynucleoside analogues and thiopurines. The anti-folates include methotrexate and pemetrexed. The fluoropyrimidines include fluorouracil and capecitabine. The deoxynucleoside analogues include cytarabine, gemcitabine, decitabine, azacitidine, fludarabine, nelarabine, cladribine, clofarabine, and pentostatin. The thiopurines include thioguanine and mercaptopurine.

Anti-microtubule agents block cell division by preventing microtubule function. The vinca alkaloids prevent the formation of the microtubules, whereas the taxanes prevent the microtubule disassembly. Vinca alkaloids include vinorelbine, vindesine, and vinflunine. Taxanes include docetaxel (Taxotere) and paclitaxel (Taxol).

Topoisomerase inhibitors are drugs that affect the activity of two enzymes: topoisomerase I and topoisomerase II and include irinotecan, topotecan, camptothecin, etoposide, doxorubicin, mitoxantrone, teniposide, novobiocin, merbarone, and aclarubicin.

The cytotoxic antibiotics are a varied group of drugs that have various mechanisms of action. The common theme that they share in their chemotherapy indication is that they interrupt cell division. The most important subgroup is the anthracyclines (e.g., doxorubicin, daunorubicin, epirubicin, idarubicin pirarubicin, and aclarubicin) and the bleomycins; other prominent examples include mitomycin C, mitoxantrone, and actinomycin.

### Immune checkpoint inhibitors

In certain embodiments, immune checkpoint inhibitors are used in combination with other therapeutic agents described herein.

As used herein, "immune checkpoint" refers to co-stimulatory and inhibitory signals that regulate the amplitude and quality of immune cell activity such as NK cell activity. In certain embodiments, the immune checkpoint is an inhibitory signal. In certain embodiments, the inhibitory signal is the interaction between PD-1 and PD-L1. In certain embodiments, the inhibitory signal is the interaction between CTLA-4 and CD80 or CD86 to displace CD28 binding. In certain embodiments the inhibitory signal is the interaction between LAG3 and MHC class II molecules. In certain embodiments, the inhibitory signal is the interaction between TIM3 and galectin 9.

As used herein, "immune checkpoint inhibitor" refers to a molecule that totally or partially reduces, inhibits, interferes with or modulates one or more checkpoint proteins. In certain embodiments, the immune checkpoint inhibitor prevents inhibitory signals associated with the immune checkpoint. In certain embodiments, the immune checkpoint inhibitor is an antibody, or fragment thereof that disrupts inhibitory signaling associated with the immune checkpoint. In certain embodiments, the immune checkpoint inhibitor is a small molecule that disrupts inhibitory signaling. In certain embodiments, the immune checkpoint inhibitor is an antibody, fragment thereof, or antibody mimic, that prevents the interaction between checkpoint blocker proteins, e.g., an antibody, or fragment thereof, that prevents the interaction between PD-1 and PD-L1. In certain embodiments, the immune checkpoint inhibitor is an antibody, or fragment thereof, that prevents the interaction between CTLA-4 and CD80 or CD86. In certain embodiments, the immune checkpoint inhibitor is an antibody, or fragment thereof, that prevents the interaction between LAG3 and its ligands, or TIM-3 and its ligands. The checkpoint inhibitor may also be in the form of the soluble form of the molecules (or variants thereof) themselves, e.g., a soluble PD-L1 or PD-L1 fusion.

The "Programmed Death-1 (PD-1)" receptor refers to an immuno-inhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1.

"Programmed Death Ligand-1 (PD-L1)" is one of two cell surface glycoprotein ligands for PD-1 (the other being PD-L2) that downregulates T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1.

"Cytotoxic T Lymphocyte Associated Antigen-4 (CTLA-4)" is an immune cell surface molecule and is a member of the immunoglobulin superfamily. This protein downregulates the immune system by binding to CD80 and CD86. The term "CTLA-4" as used herein includes human CTLA-4 (hCTLA-4), variants, isoforms, and species homologs of hCTLA-4, and analogs having at least one common epitope with hCTLA-4.

"Lymphocyte Activation Gene-3 (LAG3)" is an inhibitory receptor associated with inhibition of immune cell activity by binding to MHC class II molecules. This receptor enhances the function of Treg cells and inhibits CD8+ effector T cell function. The term "LAG3" as used herein includes human LAG3 (hLAG3), variants, isoforms, and species homologs of hLAG3, and analogs having at least one common epitope.

"T Cell Membrane Protein-3 (TIM3)" is an inhibitory receptor involved in the inhibition of immune cell activity by inhibition of TH1 cell responses. Its ligand is galectin 9, which is upregulated in various types of cancers. The term "TIM3" as used herein includes human TIM3 (hTIM3), variants, isoforms, and species homologs of hTIM3, and analogs having at least one common epitope.

The "B7 family" refers to inhibitory ligands with undefined receptors. The B7 family encompasses B7-H3 and B7-H4, both upregulated on tumor cells and tumor infiltrating cells.

In certain embodiments, the immune checkpoint inhibitor suitable for use in the methods disclosed herein, is an antagonist of inhibitory signals, e.g., an antibody which targets, for example, PD-1, PD-L1, CTLA-4, LAG3, B7-H3, B7-H4, or TIM3. These ligands and receptors are reviewed in Pardoll, D., Nature. 12: 252-264, 2012.

In certain embodiments, the immune checkpoint inhibitor is an antibody or an antigen-binding portion thereof, that disrupts or inhibits signaling from an inhibitory immunoregulator. In certain embodiments, the immune checkpoint inhibitor is a small molecule that disrupts or inhibits signaling from an inhibitory immunoregulator.

In certain embodiments, the inhibitory immunoregulator is a component of the PD-1/PD-L1 signaling pathway. Accordingly, certain embodiments of the disclosure provide for administering to a subject an antibody or an antigen-binding portion thereof that disrupts the interaction between the PD-1 receptor and its ligand, PD-L1. Antibodies which bind to PD-1 and disrupt the interaction between the PD-1 and its ligand, PD-L1, are known in the art. In certain embodiments, the antibody or antigen-binding portion thereof binds specifically to PD-1. In certain embodiments, the antibody or antigen-binding portion thereof binds specifically to PD-L1 and inhibits its interaction with PD-1, thereby increasing immune activity.

In certain embodiments, the inhibitory immunoregulator is a component of the CTLA4 signaling pathway. Accordingly, certain embodiments of the disclosure provide for administering to a subject an antibody or an antigen-binding portion thereof that targets CTLA4 and disrupts its interaction with CD80 and CD86.

In certain embodiments, the inhibitory immunoregulator is a component of the LAG3 (lymphocyte activation gene 3) signaling pathway. Accordingly, certain embodiments of the disclosure provide for administering to a subject an antibody or an antigen-binding portion thereof that targets LAG3 and disrupts its interaction with MHC class II molecules.

In certain embodiments, the inhibitory immunoregulator is a component of the B7 family signaling pathway. In certain embodiments, the B7 family members are B7-H3 and B7-H4. Accordingly, certain embodiments of the disclosure provide for administering to a subject an antibody or an antigen-binding portion thereof that targets B7-H3 or H4. The B7 family does not have any defined receptors but these ligands are upregulated on tumor cells or tumor-infiltrating cells. Preclinical mouse models have shown that blockade of these ligands can enhance anti-tumor immunity.

In certain embodiments, the inhibitory immunoregulator is a component of the TIM3 (T cell membrane protein 3) signaling pathway. Accordingly, certain embodiments of the disclosure provide for administering to a subject an antibody or an antigen-binding portion thereof that targets TIM3 and disrupts its interaction with galectin 9.

It will be understood by one of ordinary skill in the art that other immune checkpoint targets can also be targeted by antagonists or antibodies, provided that the targeting results in the stimulation of an immune response such as an anti-tumor immune response as reflected in, e.g., an increase in immune cell proliferation, enhanced immune cell activation, and/or increased cytokine production (e.g., IFN-γ, IL2).

### RNA Targeting

It is particularly preferred according to the invention that the peptides, proteins or polypeptides described herein, in particular the IL2 polypeptides and/or antibodies, are administered in the form of RNA encoding the peptides, proteins or polypeptides described herein. In one embodiment, different peptides, proteins or polypeptides described herein are encoded by different RNA molecules.

In one embodiment, the RNA is formulated in a delivery vehicle. In one embodiment, the delivery vehicle comprises particles. In one embodiment, the delivery vehicle comprises at least one lipid. In one embodiment, the at least one lipid comprises at least one cationic lipid. In one embodiment, the lipid forms a complex with and/or encapsulates the RNA. In one embodiment, the lipid is comprised in a vesicle encapsulating the RNA. In one embodiment, the RNA is formulated in liposomes.

According to the disclosure, after administration of the RNA described herein, at least a portion of the RNA is delivered to a target cell. In one embodiment, at least a portion of the RNA is delivered to the cytosol of the target cell. In one embodiment, the RNA is translated by the target cell to produce the encoded peptide or protein.

Some aspects of the disclosure involve the targeted delivery of the RNA disclosed herein (e.g., RNA encoding IL2 polypeptides, RNA encoding antibodies).

RNA may be delivered by so-called lipoplex formulations, in which the RNA is bound to liposomes comprising a cationic lipid and optionally an additional or helper lipid to form injectable nanoparticle formulations. The liposomes may be obtained by injecting a solution of the lipids in ethanol into water or a suitable aqueous phase. RNA lipoplex particles may be prepared by mixing the liposomes with RNA.

In the context of the present disclosure, the term "RNA lipoplex particle" relates to a particle that contains lipid, in particular cationic lipid, and RNA. Electrostatic interactions between positively charged liposomes and negatively charged RNA results in complexation and spontaneous formation of RNA lipoplex particles. Positively charged liposomes may be generally synthesized using a cationic lipid, such as DOTMA, and additional lipids, such as DOPE. In one embodiment, a RNA lipoplex particle is a nanoparticle.

As used herein, a "cationic lipid" refers to a lipid having a net positive charge. Cationic lipids bind negatively charged RNA by electrostatic interaction to the lipid matrix. Generally, cationic lipids possess a lipophilic moiety, such as a sterol, an acyl or diacyl chain, and the head group of the lipid typically carries the positive charge. Examples of cationic lipids include, but are not limited to 1,2-di-O-octadecenyl-3-trimethylammonium propane (DOTMA), dimethyldioctadecylammonium (DDAB); 1,2-dioleoyl-3-trimethylammonium propane (DOTAP); 1,2-dioleoyl-3-dimethylammonium-propane (DODAP); 1,2-diacyloxy-3-dimethylammonium propanes; 1,2-dialkyloxy-3- dimethylammonium propanes; dioctadecyldimethyl ammonium chloride (DODAC), 2,3-di(tetradecoxy)propyl-(2-hydroxyethyl)-dimethylazanium (DMRIE), 1,2-dimyristoyl-sn-glycero-3-ethylphosphocholine (DMEPC), I,2-dimyristoyl-3-trimethylammonium propane (DMTAP), 1,2-dioleyloxypropyl-3-dimethyl-hydroxyethyl ammonium bromide (DORIE), and 2,3-dioleoyloxy- N-[2(spermine carboxamide)ethyl]-N,N-dimethyl-I-propanamium trifluoroacetate (DOSPA). Preferred are DOTMA, DOTAP, DODAC, and DOSPA. In specific embodiments, the cationic lipid is DOTMA and/or DOTAP.

An additional lipid may be incorporated to adjust the overall positive to negative charge ratio and physical stability of the RNA lipoplex particles. In certain embodiments, the additional lipid is a neutral lipid. As used herein, a "neutral lipid" refers to a lipid having a net charge of zero. Examples of neutral lipids include, but are not limited to, 1,2-di-(9Z-octadecenoyl)-sn-glycero-3-phosphoethanolamine (DOPE), 1,2-dioleoyl-sn-glycero-3-phosphocholine (DOPC), diacylphosphatidyl choline, diacylphosphatidyl ethanol amine, ceramide, sphingoemyelin, cephalin, cholesterol, and cerebroside. In specific embodiments, the additional lipid is DOPE, cholesterol and/or DOPC.

In certain embodiments, the RNA lipoplex particles include both a cationic lipid and an additional lipid. In an exemplary embodiment, the cationic lipid is DOTMA and the additional lipid is DOPE.

In some embodiments, the molar ratio of the at least one cationic lipid to the at least one additional lipid is from about 10:0 to about 1:9, about 4:1 to about 1:2, or about 3:1 to about 1:1. In specific embodiments, the molar ratio may be about 3:1, about 2.75:1, about 2.5:1, about 2.25:1, about 2:1, about 1.75:1, about 1.5:1, about 1.25:1, or about 1:1. In an exemplary embodiment, the molar ratio of the at least one cationic lipid to the at least one additional lipid is about 2:1.

RNA lipoplex particles described herein have an average diameter that in one embodiment ranges from about 200 nm to about 1000 nm, from about 200 nm to about 800 nm, from about 250 to about 700 nm, from about 400 to about 600 nm, from about 300 nm to about 500 nm, or from about 350 nm to about 400 nm. In specific embodiments, the RNA lipoplex particles have an average diameter of about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1000 nm. In an embodiment, the RNA lipoplex particles have an average diameter that ranges from about 250 nm to about 700 nm. In another embodiment, the RNA lipoplex particles have an average diameter that ranges from about 300 nm to about 500 nm. In an exemplary embodiment, the RNA lipoplex particles have an average diameter of about 400 nm.

The electric charge of the RNA lipoplex particles of the present disclosure is the sum of the electric charges present in the at least one cationic lipid and the electric charges present in the RNA. The charge ratio is the ratio of the positive charges present in the at least one cationic lipid to the negative charges present in the RNA. The charge ratio of the positive charges present in the at least one cationic lipid to the negative charges present in the RNA is calculated by the following equation: charge ratio=[(cationic lipid concentration (mol)) * (the total number of positive charges in the cationic lipid)] / [(RNA concentration (mol)) * (the total number of negative charges in RNA)].

Cytokines such as extended-PK cytokines, in particular extended-PK interleukins, such as those described herein may be provided to a subject by administering to the subject RNA encoding a cytokine in a formulation for preferential delivery of RNA to liver or liver tissue. The delivery of RNA to such target organ or tissue is preferred, in particular, if it is desired to express large amounts of the cytokine and/or if systemic presence of the cytokine, in particular in significant amounts, is desired or required.

RNA delivery systems have an inherent preference to the liver. This pertains to lipid-based particles, cationic and neutral nanoparticles, in particular lipid nanoparticles such as liposomes, nanomicelles and lipophilic ligands in bioconjugates. Liver accumulation is caused by the discontinuous nature of the hepatic vasculature or the lipid metabolism (liposomes and lipid or cholesterol conjugates).

For *in vivo* delivery of RNA to the liver, a drug delivery system may be used to transport the RNA into the liver by preventing its degradation. For example, polyplex nanomicelles consisting of a poly(ethylene glycol) (PEG)-coated surface and an mRNA-containing core is a useful system because the nanomicelles provide excellent *in vivo* stability of the RNA, under physiological conditions. Furthermore, the stealth property provided by the polyplex nanomicelle surface, composed of dense PEG palisades, effectively evades host immune defenses.

### Pharmaceutical compositions

The agents described herein may be administered in pharmaceutical compositions or medicaments and may be administered in the form of any suitable pharmaceutical composition.

In one embodiment of all aspects of the invention, the components described herein such as nucleic acid encoding a cytokine (IL2) or antibody either together or separate from each other may be administered in a pharmaceutical composition which may comprise a pharmaceutically acceptable carrier and may optionally comprise one or more adjuvants, stabilizers etc. In one embodiment, the pharmaceutical composition is for therapeutic or prophylactic treatments, e.g., for use in treating or preventing a disease involving an antigen such as a cancer disease such as those described herein.

The term "pharmaceutical composition" relates to a formulation comprising a therapeutically effective agent, preferably together with pharmaceutically acceptable carriers, diluents and/or excipients. Said pharmaceutical composition is useful for treating, preventing, or reducing the severity of a disease or disorder by administration of said pharmaceutical composition to a subject. A pharmaceutical composition is also known in the art as a pharmaceutical formulation.

The pharmaceutical compositions of the present disclosure may comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (e.g., Freund's adjuvants), mineral compounds (such as alum), bacterial products (such as Bordetella pertussis toxin), or immune-stimulating complexes. Examples of adjuvants include, without limitation, LPS, GP96, CpG oligodeoxynucleotides, growth factors, and cytokines, such as monokines, lymphokines, interleukins, chemokines. The cytokines may be IL1, IL2, IL3, IL4, IL5, IL6, IL7, IL8, IL9, IL10, IL12, IL18, IFNα, IFNγ, GM-CSF, LT-a. Further known adjuvants are aluminium hydroxide, Freund's adjuvant or oil such as Montanide^{®} ISA51. Other suitable adjuvants for use in the present disclosure include lipopeptides, such as Pam3Cys.

The pharmaceutical compositions according to the present disclosure are generally applied in a "pharmaceutically effective amount" and in "a pharmaceutically acceptable preparation".

The term "pharmaceutically acceptable" refers to the non-toxicity of a material which does not interact with the action of the active component of the pharmaceutical composition.

The term "pharmaceutically effective amount" or "therapeutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the compositions described herein may depend on various of such parameters. In the case that a reaction in a patient is insufficient with an initial dose, higher doses (or effectively higher doses achieved by a different, more localized route of administration) may be used.

The pharmaceutical compositions of the present disclosure may contain salts, buffers, preservatives, and optionally other therapeutic agents. In one embodiment, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants.

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactidelglycolide copolymers or polyoxyethylene/polyoxy-propylene copolymers. In one embodiment, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

In one embodiment, pharmaceutical compositions described herein may be administered intravenously, intraarterially, subcutaneously, intradermally or intramuscularly. In certain embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In a preferred embodiment, the pharmaceutical compositions is formulated for systemic administration. In another preferred embodiment, the systemic administration is by intravenous administration.

The term "co-administering" as used herein means a process whereby different compounds or compositions (e.g., RNA encoding a IL2 polypeptide, and antibody) are administered to the same patient. The different compounds or compositions may be administered simultaneously, at essentially the same time, or sequentially. In one embodiment, IL2 polypeptide or nucleic acid encoding IL2 polypeptide is administered first, followed by administration of antibody.

### Treatments

The agents, compositions and methods described herein can be used to treat a subject with a disease, e.g., a disease characterized by the presence of diseased cells expressing an antigen. Particularly preferred diseases are cancer diseases, in particular cancer diseases wherein cancer cells express a tumor antigen.

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

In the present context, the term "treatment", "treating" or "therapeutic intervention" relates to the management and care of a subject for the purpose of combating a condition such as a disease or disorder. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The terms "individual" and "subject" are used herein interchangeably. They refer to a human or another mammal (e.g. mouse, rat, rabbit, dog, cat, cattle, swine, sheep, horse or primate) that can be afflicted with or is susceptible to a disease or disorder (e.g., cancer) but may or may not have the disease or disorder. In many embodiments, the individual is a human being. Unless otherwise stated, the terms "individual" and "subject" do not denote a particular age, and thus encompass adults, elderlies, children, and newborns. In embodiments of the present disclosure, the "individual" or "subject" is a "patient".

The term "patient" means an individual or subject for treatment, in particular a diseased individual or subject.

As used herein, "immune response" refers to an integrated bodily response to an antigen or a cell expressing an antigen and refers to a cellular immune response and/or a humoral immune response.

"Cell-mediated immunity", "cellular immunity", "cellular immune response", or similar terms are meant to include a cellular response directed to cells characterized by expression of an antigen, in particular characterized by presentation of an antigen with class I or class II MHC. The cellular response relates to cells called T cells or T lymphocytes which act as either "helpers" or "killers". The helper T cells (also termed CD4⁺ T cells) play a central role by regulating the immune response and the killer cells (also termed cytotoxic T cells, cytolytic T cells, CD8+ T cells or CTLs) kill diseased cells such as cancer cells, preventing the production of more diseased cells.

The term "immunotherapy" relates to the treatment of a disease or condition by inducing, or enhancing an immune response. The term "immunotherapy" includes antigen immunization or antigen vaccination.

The terms "immunization" or "vaccination" describe the process of administering an antigen to an individual with the purpose of inducing an immune response, for example, for therapeutic or prophylactic reasons.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In one embodiment, the macrophages are splenic macrophages.

The term "dendritic cell" (DC) refers to another subtype of phagocytic cells belonging to the class of antigen presenting cells. In one embodiment, dendritic cells are derived from hematopoietic bone marrow progenitor cells. These progenitor cells initially transform into immature dendritic cells. These immature cells are characterized by high phagocytic activity and low T cell activation potential. Immature dendritic cells constantly sample the surrounding environment for pathogens such as viruses and bacteria. Once they have come into contact with a presentable antigen, they become activated into mature dendritic cells and begin to migrate to the spleen or to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors in T cell activation such as CD80, CD86, and CD40 greatly enhancing their ability to activate T cells. They also upregulate CCR7, a chemotactic receptor that induces the dendritic cell to travel through the blood stream to the spleen or through the lymphatic system to a lymph node. Here they act as antigen-presenting cells and activate helper T cells and killer T cells as well as B cells by presenting them antigens, alongside non-antigen specific co-stimulatory signals. Thus, dendritic cells can actively induce a T cell- or B cell-related immune response. In one embodiment, the dendritic cells are splenic dendritic cells.

The term "antigen presenting cell" (APC) is a cell of a variety of cells capable of displaying, acquiring, and/or presenting at least one antigen or antigenic fragment on (or at) its cell surface. Antigen-presenting cells can be distinguished in professional antigen presenting cells and non-professional antigen presenting cells.

The term "professional antigen presenting cells" relates to antigen presenting cells which constitutively express the Major Histocompatibility Complex class II (MHC class II) molecules required for interaction with naive T cells. If a T cell interacts with the MHC class II molecule complex on the membrane of the antigen presenting cell, the antigen presenting cell produces a co-stimulatory molecule inducing activation of the T cell. Professional antigen presenting cells comprise dendritic cells and macrophages.

The term "non-professional antigen presenting cells" relates to antigen presenting cells which do not constitutively express MHC class II molecules, but upon stimulation by certain cytokines such as interferon-gamma, Exemplary, non-professional antigen presenting cells include fibroblasts, thymic epithelial cells, thyroid epithelial cells, glial cells, pancreatic beta cells or vascular endothelial cells.

"Antigen processing" refers to the degradation of an antigen into procession products, which are fragments of said antigen (e.g., the degradation of a protein into peptides) and the association of one or more of these fragments (e.g., via binding) with MHC molecules for presentation by cells, such as antigen presenting cells to specific T cells.

The term "disease involving an antigen" refers to any disease which implicates an antigen, e.g. a disease which is characterized by the presence of an antigen. The disease involving an antigen can be a cancer disease or simply cancer. As mentioned above, the antigen may be a disease-associated antigen, such as a tumor-associated antigen. In one embodiment, a disease involving an antigen is a disease involving cells expressing an antigen, preferably on the cell surface.

The terms "cancer disease" or "cancer" refer to or describe the physiological condition in an individual that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the disclosure also comprises cancer metastases.

Citation of documents and studies referenced herein is not intended as an admission that any of the foregoing is pertinent prior art. All statements as to the contents of these documents are based on the information available to the applicants and do not constitute any admission as to the correctness of the contents of these documents.

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

### Examples

### Example 1: Selection and generation of MHC class I deficient or IFN signaling deficient murine tumor models

Our first aim was to analyze how MHC class I loss affects the interaction of tumor cells with the immune system. We assumed that CD8+ T cells might play a major role in this regard, since their recognition of tumor cells strictly depends on MHC class I and compared the CD8+ T cell infiltration in different murine tumor models. Balb/c mice (Balb/c JRj, Janvier Labs) (n=8) were inoculated subcutaneously (s.c.) with 5x10⁵ CT26 cells and C57BI/6 mice (C57BI/6 JOlaHsd, Jackson Laboratory) (n=8 per cell line) were inoculated s.c. with 5x10⁵ MC38 cells, 3x10⁵ B16F10 cells or 1x10⁵ TC1 cells. 20 days after tumor inoculation, mice were sacrificed by cervical dislocation and the tumors were excised. Tumors were chopped into pieces and digested using the Tumor Dissociation Kit, mouse (Miltenyi Biotec, cat. No. 130-096-730) according to the manufacturer's instruction. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14). Antibodies against CD45 (BD, cat No. 564279) and CD8 (BD, cat No. 563046 or cat No. 553031) were used. Flow cytometric analysis was performed on a BD LSRFortessa^{™} flow cytometer (Becton Dickinson GmbH) and acquired data was analyzed using FlowJo software version 10 (TreeStar).

We observed varying proportions of CD8+ T cells in all viable cells across the different tumor models and classified the infiltration as high (CD8^{high}) for CT26 and MC38, intermediate (CD8^{int}) for B16F10 and low (CD8^{low}) for TC1 (Figure 1 A). The proportion of CD8+ T cells correlated with the proportion of total CD45⁺ immune cells in all viable cells, as CT26 and MC38 exhibited high, B16F10 intermediate and TC1 low infiltration by immune cells in general (Figure 1 B).

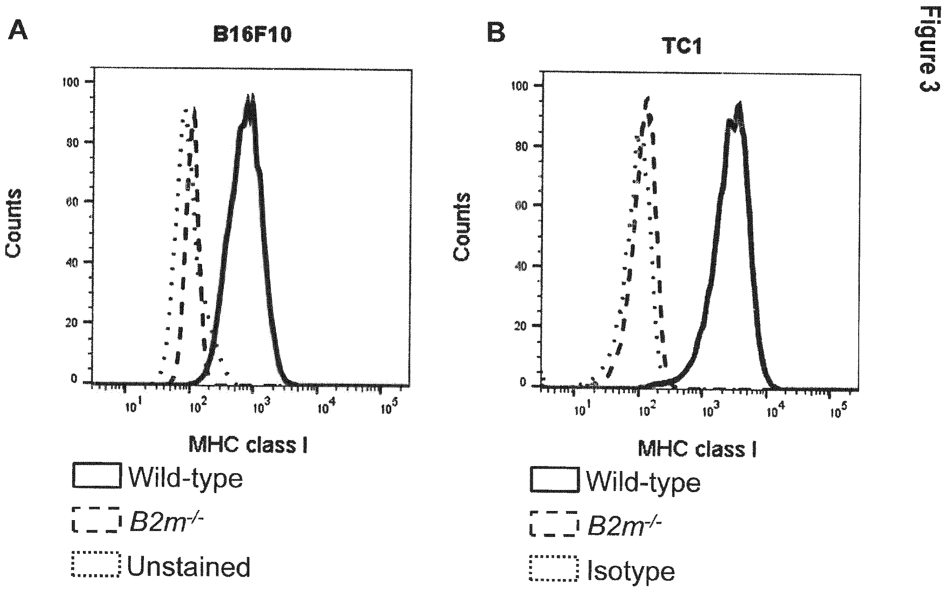

In order to genetically knock out MHC class I in the tumor cell lines, we generated a single guide RNA (sgRNA) targeting *B2m* using publicly available tools (CT26, B16F10, TC1: CATGGCTCGCTCGGTGACCC; MC38: GACAAGCACCAGAAAGACCA). Tumor cells were transiently transfected with sgRNA and mRNA coding for Cas9 as well as mRNAs coding for E3 and B18 (in order to inhibit a cellular response to dsRNA) by lipofection with RNAiMAX (Invitrogen, cat. No. 13778030) or by electroporation in 4 mm cuvettes (VWR, cat. No. 732-1137). Single clones were obtained by limiting dilution, expanded and the successful knock-out of *B2m* was confirmed by absence of MHC class I on the cell surface analyzed by flow cytometry (Figure 2 and 3). Since B16F10 cells express only minute amounts of MHC class I *in vitro,* the cells were cultured in 6-well plates for 24 h at 37 °C and 5% CO₂ in the presence of 25 ng/mL IFNγ (Peprotech, cat. No. 315-05) to induce MHC class I upregulation.

Given that defects in the IFN signaling pathway are another clinically relevant mechanism of resistance against cancer immunotherapy, we also generated a B16F10 derived tumor cell line that lacks IFN response. To achieve this, the central IFN signaling molecule *Jak1* was knocked out using the CRISPR/Cas9 system as described above with a *Jak1* targeting sgRNA (TGGCGTTCTGTGCTAAAATG). Parental B16F10 cells were seeded into 6-well plates and were cultured for 24 h at 37 °C and 5% CO₂ in the presence of 1000 U/mL IFNα (PBL, cat. No. 12100-1) or cultured without IFN (Control). Subsequently, cells were subjected to flow cytometry analysis to determine the expression levels of PD-L1 and MHC class I. B16F10 cells showed strong upregulation of both IFN inducible markers (Figure 4). Similar treatment of B16F10-*Jak1*^{-/-} did not result in detectable upregulation of either marker, confirming a defect IFN response pathway (Figure 5).

### Example 2: Loss of MHC class I affects tumor progression and composition of the tumor microenvironment

To investigate whether MHC class I deficiency affects tumor progression, mice were inoculated with wild type or B2m defective (*B2m*^{-/-}) tumor cells as described in Example 1 (CT26, MC38: n=5 per group; B16F10, TC1: n=10 per group).

The growth of *B2m*^{-/-} tumors was compared to the growth of corresponding control tumors. Both CT26-*B2m*^{*-*/*-*} and MC38-*B2m*^{-/-} tumors showed significantly enhanced tumor growth (Figure 6), while no significant differences were observed for B16F10-*B2m*^{-/-} and TC1-*B2m*^{-/-} tumors (Figure 7).

We concluded that spontaneous CD8+ T cell responses are likely to control the growth of untreated CD8^{high} tumor models, resulting in enhanced growth of the derived MHC class I deficient tumors. We further hypothesized that the absence of spontaneous antigen recognition in the derived MHC class I deficient tumor models could also affect the immune cell composition of the tumor microenvironment (TME). To investigate this, mice were inoculated with normal or *B2m*^{-/-} tumor cells as described in Example 1 (n=8 mice per group). 20 days after tumor inoculation, tumors were excised and digested as described in Example 1. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14) or with Fixable Yellow Dead Cell Stain (Life technologies, cat No. L34967). Antibodies against CD3 (BD, cat. No. 100227), CD4 (BD, cat. No. 564298), CD8 (BD, cat. No. 563046 or cat No. 553031), CD11b (BD, cat. No. 553310), CD11c (Miltenyi Biotec, cat. No. 130-102-493), CD25 (BD, cat. No. 564023), CD45 (BD, cat. No. 564279), CD49b (BD, cat. No. 740133), CD103 (ebioscience, cat. No. 12-1031-83), F4/80 (BD, cat. No. 123146), GR-1 (BD, cat. No. 108423), NK1.1 (Biolegend, cat. No. 108738), XCR1 (Biolegend, cat. No. 148220) and FoxP3 (ebioscience, cat No. 12-5773-82) were used. Flow cytometric analysis was performed on a BD LSRFortessa^{™} flow cytometer (Becton Dickinson GmbH) and acquired data was analyzed using FlowJo software version 10 (TreeStar).

We compared the immune cell infiltration of *B2m*^{-/-} tumors to corresponding control tumors. Both CT26-*B2m*^{-/-} and MC38-*B2m*^{-/-} tumors exhibited significantly reduced infiltration by CD8+ T cells, NK cells and cross-presenting dendritic cells (cDC1s) (Figure 8 and 10). Furthermore, MC38-*B2m*^{-/-} tumors showed significantly reduced infiltration by macrophages and monocytes. No differences were observed in the cellular composition of tumor-draining lymph nodes (TDLNs) from CT26 and CT26-*B2m*^{-/-} tumor bearing mice (Figure 9). In the MC38-*B2m*^{-/-} model, we observed slightly increased amounts of CD8+ T cells and Tregs, as well as reduced numbers of cDC1 and CD11b⁺ DCs in the TDLNs. B16F10-*B2m*^{-/-} tumors showed no differences in the infiltration by CD8+ T cells, NK cells and cDC1s, but increased numbers of CD4 Th cells and decreased numbers of macrophages, monocytes and CD11b⁺ DCs were observable (Figure 12). Numbers of cDC1s in the TDLNs of B16F10-*B2m*^{-/-} tumor bearing mice were increased (Figure 13). TC1-*B2m*^{-/-} tumors showed no detectable differences in the immune cell composition of both tumors and TDLNs (Figure 14 and 15). The changes in the immune cell infiltration observed across the different tumor models correlated well with the classification of CD8+ T cell infiltration, with the largest differences observed in CD8^{high} tumor models. This suggests that spontaneous CD8+ T cell recognition of tumor cells dictates the immune cell attraction into the TME to a large degree. As the changes observed in the TDLNs were not correlated to the TME and had a much lesser extent, the impact of MHC class I loss seems to be mainly confined to the TME. Importantly, the immune cell subsets that were reduced in CT26-*B2m*^{-/-} and MC38-*B2m*^{-/-} tumors are regarded as essential for antitumor immunity, while subsets considered as immunosuppressive were unaffected except for macrophages in the MC38 model. This implies that an active, inflamed TME can be turned into a immunosuppressive TME upon MHC class I loss.

Our next aim was to analyze whether there are differences in the actively ongoing migration of immune cells into the TME of *B2m*^{-/-} compared to corresponding control tumors. We chose the CT26 model for this characterization as it exhibits the highest CD8+ T cell infiltrate. Mice were inoculated with normal or derived *B2m*^{-/-} tumor cells as described in Example 1 (n=5 mice per group and per time point). Tumors were excised on different time points (8, 12, 20 and 26 days after inoculation) and digested as described in Example 1. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14). Antibodies against CD45 (BD, cat. No. 564279), CD3 (Biolegend, cat. No. 100233) and CD49b (BD, cat. No. 553875) were used. Cells were transferred to Absolute Counting Tubes and flow cytometric analysis was performed on a BD LSRFortessa^{™} flow cytometer (Becton Dickinson GmbH) and acquired data was analyzed using FlowJo software version 10 (TreeStar).

The numbers of tumor-infiltrating CD3⁺ T cells and NK cells did not show differences by day 8 after tumor inoculation. However, CT26 tumors showed increasing infiltration by both cell types as time progressed (Figure 16). In CT26-*B2m*^{-/-} tumors, there was no such increase observable, demonstrating that CD3⁺ T cells and NK cells become actively attracted into the CT26, but not the CT26-*B2m*^{-/-} TME. These data further corroborate that an actively ongoing inflammation in the TME leading to immune cell infiltration is impaired once the tumor cells lose MHC class I expression.

As the described observations underline the role of CD8+ T cells in shaping the TME of MHC class I expressing tumors, we analyzed markers of antigen encounter expressed by CD8+ T cells. In this experiment, we included the TC1 model for comparison as the model with the lowest CD8+ T cell infiltration. Mice were inoculated with normal or derived *B2m*^{-/-} tumor cells as described in Example 1 (n=8 mice per group). 20 days after tumor inoculation, tumors were excised and digested as described in Example 1. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14) or with Fixable Yellow Dead Cell Stain (Life technologies, cat No. L34967). Antibodies against CD8 (BD, cat. No. 563046), CD11b (BD, cat. No. 553310), CD45 (BD, cat. No. 564279), F4/80 (BD, cat. No. 123146), MHC class II (BD, cat No. 742894), PD-1 (ebioscience, cat. No. 46-9981-82) and PD-L1 (Biolegend, cat. No. 124333) and gp70 tetramer (MBI, cat. No. MBL-TB-M521-7) were used. Flow cytometric analysis was performed on a BD LSRFortessa^{™} flow cytometer (Becton Dickinson GmbH) and acquired data was analyzed using FlowJo software version 10 (TreeStar). We compared the CD8+ T cell phenotype from *B2m*^{-/-} tumors to corresponding control tumors. gp70 is a viral CD8⁺T cell antigen, which is expressed by CT26 cells. CD8+ T cells exhibited a significantly reduced proportion of gp70 antigen-specific cells (Figure 17 A). Moreover, the percentage of PD-1⁺ CD8+ T cells was significantly reduced in CT26-*B2m*^{-/-} tumors (Figure 17 B). In TC1-*B2m*^{-/-} tumors, we also observed a reduced percentage of PD-1⁺ CD8+ T cells (Figure 17 C). With gp70 as a measure of antigen specificity and PD-1 being a marker for CD8+ T cell activation and antigen encounter, the differences in the CD8+ T cell phenotype suggest diminished recognition of antigen in the CT26-*B2m*^{-/-} TME. The magnitude of the difference in the TC1 model was minor compared to the CT26 model, indicating lesser spontaneous CD8+ T cell recognition of the TC1 tumor cells. We sought for evidence that antigen recognition by CD8+ T cells in MHC class I proficient tumors has a direct impact on other immune cells located in the TME. Analysis of IFNγ (the signature cytokine of CD8+ T cells released upon antigen encounter) inducible markers expressed by tumor-associated macrophages (TAMs) and tumor cells in CT26-*B2m*^{-/-} and CT26 tumors revealed a massive reduction in the expression of PD-L1 and MHC class II by TAMs and PD-L1 by tumor cells (Figure 18). Analysis of the same markers in the TC1 model revealed no differences in MHC class II expression by TAMs (Figure 19 A). PD-L1 expression by TAMs and tumors cells was significantly reduced, but the magnitude was again lower compared to the CT26 model, in accordance with the magnitude of differences in PD-1⁺ expression by CD8+ T cells (Figure 19 C and D). These data propose that spontaneous CD8+ T cell recognition leads to a strong IFNγ signature in the CD8^{high} CT26 TME, which is impaired in the CT26-*B2m*^{-/-} TME. In the CD8^{low} TC1 model on the other hand, the spontaneous immune response against the tumor cells and therefore the IFNγ signature does apparently not reach the necessary threshold to induce an inflamed TME. For this reason, the TME is not affected by MHC class I loss as it is the case in the CD8^{high} models.

Our last aim was to confirm that the absence of IFNγ released by antigen-recognizing CD8+ T cells is the reason that less antitumoral immune cells (CD8⁺ T cells, NK cells and cDC1) are attracted into the TME of CT26 and MC38 tumors after they lost MHC class I expression. Mice were inoculated with normal or derived *B2m*^{-/-} tumor cells as described in Example 1 (CT26: n=3 mice per group, MC38: n=5 mice per group). 19 days after tumor inoculation, tumors were excised and snap-frozen in liquid nitrogen. Total RNA was extracted from the tumors using the RNeasy Mini Kit (Qiagen, cat. No. 74106) according to the manufacturer's instruction and RNA concentration and integrity were determined using a NanoDrop 200c (Thermo Fisher) and a Fragment Analyzer capillary gel electrophoresis instrument (Agilent Advanced Analytical). 1 µg RNA per sample was used as template for reverse transcription using the PrimeScript RT Reagent Kit with gDNA Eraser (Takara, cat. No. RR047A). The resulting cDNA was used for quantitative real-time PCR (qRT-PCR) analysis using the SsoAdvanced Universal SYBR Green Supermix (Biorad, cat. No. 1725271) according to the manufacturer's instruction on a CFX384 Touch Real-Time PCR Detection System (BioRad) using 40 cycles two-step PCR at an annealing temperature of 60 °C with a reaction volume of 15 µL including 1.25 µL cDNA per reaction and a final primer concentration of 333 nM per primer. Ct-values were determined using the CFX Manager 3.1 software (Biorad) and ΔΔCt values were computed by normalization to the *Hprt* reference gene in order to determine relative gene expression values. For reliability reasons a cut-off was used to exclude Ct-values > 35. Primer sequences: Hprt forward primer (CCCTGGTTAAGCAGTACAGC), Hprt reverse primer (CTCATTATAGTCAAGGGCATATCC); Ifng forward primer (CT26) (TTCTTCAGCAACAGCAAGGCG); Ifng reverse primer (CT26) (TGGACCACTCGGATGAGCT); Ifng forward primer (MC38) (GAGGAACTGGCAAAAGGATGG), Ifng reverse primer (MC38) (GCCTTGCTGTTGCTGAAGAAG); Cxcl9 forward primer (GCAACAAAACTGAAATCATTGCTAC), Cxcl9 reverse primer (GTTTTTCATGTTCTTTTGATGTTTTTTCC); Cxcl10 forward primer (GAGTGGGACTCAAGGGATC), Cxcl10 reverse primer (TTCTTTTTCATCGTGGCAATGATCTC); Cxcl11 forward primer (GCGACAAAGTTGAAGTGATTGTTAC), Cxcl11 reverse primer (AGTCAGACGTTCCCAGGATG); Ccl5 forward primer (GGAGTATTTCTACACCAGCAGC), Ccl5 reverse primer (CAGAATCAAGAAACCCTCTATCC); Xcl1 forward primer (ATGGGTTGTGGAAGGTGTGG), Xcl1 reverse primer (CCATTTGGCTTCTGGATCAGC)

We compared the gene expression levels of *Ifng* as well as IFN-inducible chemokines *Cxcl*9, Cxcl10 and *Cxcl11* (CXCR3 ligands) from *B2m*^{-/-} tumors to corresponding control tumors. Expression of *Ifng* was reduced in both tumor models (Figure 20 A and 21 A). The expression levels of CXCR3 ligands, which are essential for the recruitment of T cells and NK cells into the TME, were likewise reduced in both tumor models (Figure 20 B, C, D and 21 B, C, D) (Nagarsheth et al. Nat Rev Immunol 17, 559-72 (2017)). These observations confirm the reduced IFNy signature in MHC class I deficient tumors that were originally CD8^{high}. Induction of CXCR3 ligands by CD8⁺ T cell derived IFNy explains the continuous attraction of additional CD3⁺ T cells and NK cells into the TME of CD8^{high} tumors, which is lost by MHC class I deficient counterparts. Moreover, the expression levels of cDC1 attracting chemokines Ccl5 and *Xcl1* were reduced (Figure 20 E, F and 21 E, F). NK cells are known to be the main producers of these chemokines and their impaired infiltration might be the reason for reduced chemokine expression and therefore less infiltration by cDC1 (Böttcher et al. Cell 172, 1022-37 (2018)).

Our results imply that spontaneous CD8⁺ T cell recognition of tumor antigens can control the growth of MHC class I expressing tumors to some degree and maintains an inflamed TME, triggering a chain reaction of chemokine induction, which results in attraction of further antitumoral immune cells. In summary, it is shown that originally inflamed tumors become highly immunosuppressive if MHC class I is lost, which leads to unforeseen effects on the TME.

### Example 3: MHC class I loss is a comprehensive mechanism of resistance against immunotherapy and classical cancer therapy

After the characterization of untreated MHC class I deficient tumor models, we set out to analyze their reluctance to cancer therapies. We started by testing the resistance of CT26-*B2m*^{-/-} and MC38-*B2m*^{-/-} tumors against ICB antibodies blocking PD-1 and CTLA4 and an agonistic antibody against 4-1BB. Mice were inoculated with normal or *B2m*^{*-*/*-*} tumor cells as described in Example 1 (n=10 per group for tumor growth, n=5-7 per group for flow cytometry) and injected intraperitoneally (i.p.) with 200 µg anti-PD-1 (Bioxcell, cat. No. BE0146), anti-CTLA (Bioxcell, cat. No. BE0164) or anti-4-1BB (Bioxcell, cat. No. BE0239) antibody on day 0, 3, 7 and 10 after the tumors reached a mean volume of 32-36 mm³ (MC38: two additional treatments on day 14 and 17). Control groups were injected with an irrelevant antibody (Bioxcell, cat. No. 0089). Anti-tumor efficacy was determined as tumor growth inhibition in the test groups compared to the control group. CT26 and CT26-*B2m*^{-/-} tumors were excised 7 days (anti-4-1BB) or 10 days (anti-PD1 and anti-CTLA4) after the first treatment and digested as described in Example 1. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14). Antibodies against CD8 (BD, cat. No. 563046) and CD45 (BD, cat. No. 564279) were used. Cells were transferred to Absolute Counting Tubes and flow cytometric analysis was performed on a BD LSRFortessa^{™} flow cytometer (Becton Dickinson GmbH) and acquired data was analyzed using FlowJo software version 10 (TreeStar).

The tested treatments resulted in a significant growth inhibition of CT26 and MC38 tumors compared to the control groups (Figure 22 A and 24 A). The treated CT26-*B2m*^{-/-} and MC38-*B2m*^{-/-} tumors grew with similar kinetics as the control groups and no significant growth inhibition was detected (Figure 22 B and 24 B). Analysis of CD8⁺ T cell infiltration after the different treatments showed increased numbers of CD8⁺ T cells in treated CT26 tumors relative to the control group (Figure 23 A and B), reaching statistical significance after anti-CTLA4 or anti-4-1BB treatment. In comparison to that, CD8⁺ T cell numbers did not increase in CT26-*B2m*^{-/-} after any of the tested treatments. These results confirm that loss of MHC class I confers complete resistance against antibody-based immunotherapies and that intratumoral immune responses are confined to the responding tumor models, which express functional MHC class I.

We subsequently tested whether CT26-*B2m*^{-/-} and TC1-*B2m*^{-/-} tumors become resistant to therapeutic RNA vaccination. Mice were inoculated with normal or *B2m*^{-/-} tumor cells as described in Example 1 (n=10 per group for tumor growth, n=5 per group for flow cytometry). CT26 and CT26-*B2m*^{-/-} bearing mice were vaccinated intravenously (i.v.) as described in Kranz *et al.* (Kranz, L. M. et al. Nature 534, 396-401 (2016)) with 40 µg RNA-LPX coding for gp70 (SPSAYAHQF) on day 5, 8, 12 and 19 after tumor inoculation and TC1 or TC1-*B2m*^{-/-} bearing mice were vaccinated intravenously (i.v.) with 40 µg RNA-LPX coding for E7 (RAHYNIVTF) on day 0 and 7 after the tumors reached a mean volume of approximately 20 mm³. E7 is a viral CD8⁺ T cell antigen, which is expressed by TC1 cells. Additional groups were vaccinated with irrelevant RNA-LPX (not coding for any antigen) and control groups were untreated. Anti-tumor efficacy was determined as tumor growth inhibition in the test groups compared to the control group. CT26 and CT26-*B2m*^{-/-} tumors were excised 17 days, TC1 and TC1-*B2m*^{-/-} tumors were excised 9 days after the first treatment and digested as described in Example 1. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14). Antibodies against CD8 (BD, cat. No. 563046 or Invitrogen, cat No. 1825015) and CD45 (BD, cat. No. 564279) and gp70 tetramer (MBI, cat. No. MBL-TB-M521-7) or E7 dextramer (Immudex, cat. No. JA2195-PE) were used. Cells were transferred to Absolute Counting Tubes and flow cytometric analysis was performed on a BD LSRFortessa^{™} flow cytometer (Becton Dickinson GmbH) and acquired data was analyzed using FlowJo software version 10 (TreeStar).

Vaccination with antigen-coding RNA-LPX resulted in significant growth inhibition of CT26 and TC1 tumors, while irrelevant RNA-LPX did not significantly affect the tumor growth compared to the control groups (Figure 25 A and 27 A). Vaccination with antigen-coding or irrelevant RNA-LPX did not inhibit the tumor growth of derived *B2m*^{-/-} tumors (Figure 25 B and 27 B). Analysis of CD8⁺ T cell infiltration and antigen specificity revealed no significant effect of irrelevant RNA-LPX in any tumor model (Figure 26 and 28). Vaccination with antigen-coding RNA-LPX led to significantly enhanced infiltration of CD8⁺ T cells into *B2m*^{*-*/*-*} and control tumors. Related to the increased infiltration, antigen-coding RNA-LPX enhanced the frequency of antigen-specific CD8⁺ T cells in both *B2m*^{*-*/*-*} and control tumors. This shows that vaccination primes and expands CD8⁺ T cells, which infiltrate the different tumor models even if they are MHC class I negative. Despite that, the infiltration by antigen-specific CD8⁺ T cells does not affect the growth of MHC class I deficient tumors, confirming their resistance against therapeutic vaccination. Recent reports indicate that the immune system plays an important role during for response to chemotherapy or radiotherapy (classical cancer therapy) ( Galluzzi et. al. Cancer Cell 28, 690-714 (2015), Weichselbaum et. al. Nat Rev Clin Oncol 14, 365-379 (2017)). We hypothesized that the silencing of inflamed tumors through MHC class I loss might affect also the efficacy of classical cancer therapy. To test this hypothesis, mice were inoculated with CT26 or CT26-*B2m*^{-/-} tumor cells as described in Example 1 (n=9-10 per group for tumor growth, n=5 per group for flow cytometry). For chemotherapy, mice were injected intraperitoneally (i.p.) with 5 mg/kg Oxaliplatin (OX) (Medac, Medoxa) and intravenously (i.v.) with 60 mg/kg 5-fluorouracil (5-FU) (Medac, 5-FU medac) on day 0, 7 and 14 after the tumors reached a mean volume of approximately 6 mm³. The control groups received vehicle. For radiotherapy, tumors were locally irradiated with 12 Gy using an X-RAD 320 (Precision X-Ray Instruments) after they reached a mean volume of approximately 30 mm³. The control groups were untreated. Anti-tumor efficacy was determined as tumor growth inhibition in the test groups compared to the control group and survival during an observation period of 100 days. Tumors were excised 13 days (chemotherapy) or 8 days (radiotherapy) after the first treatment and digested as described in Example 1. The samples were processed to single cell suspensions and stained as described in Grunwitz *et al*. (Grunwitz, C. et al. Oncoimmunology 8, published online (2019)). Dead cells were stained with eF780 (ebioscience, cat. No. 65-0865-14). Antibodies against CD8 (BD, cat. No. 553031) and CD45 (BD, cat. No. 564279) and gp70 tetramer (MBI, cat. No. MBL-TB-M521-7) were used. Cells were transferred to Absolute Counting Tubes and flow cytometric analysis was performed on a BD LSRFortessa^{™} flow cytometer (Becton Dickinson GmbH) and acquired data was analyzed using FlowJo software version 10 (TreeStar).

Treatment with chemotherapy or radiotherapy resulted in significantly inhibited growth of CT26 as well as CT26-*B2m*^{-/-} tumors, which led to significantly improved survival of all treated groups over control groups (Figure 29 and 31). After both chemotherapy or radiotherapy, 3/10 (30%) of CT26 tumor bearing mice showed a complete response, which resulted in tumor rejection and long-term survival until 100 days after tumor inoculation, while all control animals had to be sacrificed due to progressing tumors. In contrast, no CT26-*B2m*^{-/-} tumor bearing mouse showed a complete response after any treatment and all mice had to be sacrificed before day 100. Analysis of CD8⁺ T cell infiltration and antigen specificity in CT26 tumors revealed tendencies towards increased CD8⁺ T cell numbers after chemo- or radiotherapy compared to control groups as well as higher gp70 antigen-specificity after chemotherapy (Figure 30 A and C and 32 A and C). The same tendencies in the numbers of infiltrating CD8⁺ T cells were observable in CT26-*B2m*^{-/-} tumors and after both therapies, a significant increase of antigen specificity of infiltrating CD8⁺ T cells was detected (Figure 30 B and D and 32 B and D). These data suggest that classical cancer therapy can lead to priming of a CD8⁺ T cell response, which is able to mediate complete response resulting in rejection of MHC class I expressing tumors. For this reason, MHC class I deficient tumors are able to avoid immune-mediated complete response to classical cancer therapies.

### Example 4: Antibody and IL2 combination immunotherapy induces inflammation in the TME and leads to therapeutic immune responses against MHC class I deficient tumors

We next sought to identify a treatment regimen that enables therapeutic immune responses against MHC class I deficient tumors. The adaptive immune system preferentially recognizes tumor cells via antigens presented on MHC class I. Since this is no longer possible for MHC I deficient tumors, re-establishing antigen-specific recognition of tumors through an alternative way is necessary. To achieve this, we based the regimen on an antibody binding to a tumor-associated antigen (TAA) in order to brand the tumor cells as foreign. Innate immune cells (for example NK cells or macrophages) can recognize antibody-opsonized tumor cells via Fcy receptors (FcyRs) and eliminate the cells in antigen-specific manner through antibody-dependent cellular cytotoxicity (ADCC) or phagocytosis (ADCP). We furthermore hypothesized that a second compound serving as adjuvant would be necessary to activate and license immune cells with FcyRs for tumor cell elimination. For this purpose, we chose the cytokine IL2 as it is a potent activator of innate and adaptive immunity. IL2 activates NK cells and enhances IFNy production (Weigent et al. Infect Immun 41, 992-7 (1983)). As secondary effects, IFNy released by NK cells in the TME can induce the expression of several chemokines, leading to immune cell influx, and is also able to activate macrophages as potent ADCP effector cells (Shi et al. J Immunol 194, 4379-86 (2015)). Mice were inoculated with B16F10 and derived *B2m*^{*-*/*-*} tumor cells as described in Example 1 (n=9-10 per group). Mice were injected intraperitoneally (i.p.) with 200 µg anti-Trp1 antibody (TA99) or isotype control on day 5, 8, 12, 15, 19, 22 and 26 and intravenously (i.v.) with 1 µg RNA coding for mAlb-mIL2 or mAlb (not coding for any cytokine) as control formulated with TransIT^{®} (Mirus, cat. No. MIR2255) on day 5, 12, 19 and 26 after tumor inoculation. mAlb-mlL2 is a fusion protein where albumin is linked to the N-terminus of IL2 in order to extend the serum half-life of IL2 and to enrich the protein in the tumor through the enhanced permeability and retention (EPR) effect. The protein is encoded on N1-methyl-pseudouridine-modified mRNA, which leads to high expression of protein in the liver for several days. The control group was treated with isotype and mAlb RNA. Anti-tumor efficacy was determined as tumor growth inhibition in the test groups compared to the control group and survival during an observation period of 100 days. Additional mice were inoculated with B16F10-*B2m*^{-/-} as described in Example 1 (n=7-8 per group) and treatment as described above was initiated 9 days after tumor inoculation. Tumors were excised 11 days after the first treatment and digested as described in Example 1. The samples were processed to single cell suspensions and stained as described in Grunwitz *et al.* (Grunwitz, C. et al. Oncoimmunology 8, published online (2019)). Dead cells were stained with eF780 (ebioscience, cat. No. 65-0865-14), Fixable Yellow Dead Cell Stain (Life technologies, cat No. L34967) or Fixable Red Dead Cell Stain (Life technologies, cat. No. L34971). Antibodies against CD3 (Biolegend, cat. No. 100227), CD4 (BD, cat. No. 564298) CD8 (BD, cat. No. 553031), CD11b (BD, cat. No. 553310) CD11c (Miltenyi, cat. No. 130-102-493), CD25 (BD, cat. No. 564023), CD45 (BD, cat. No. 564279), CD103 (ebioscience, cat. No. 12-1031-83), F4/80 (Biolegend, cat. No. 123132 and 123146), FoxP3 (ebioscience, cat. No. 12-5773-82), FcγRI (BD, cat. No. 740622), FcγRII/III (BD, cat. No. 558636), FcyRIV (BD, cat. No. 742564), TCR γδ (BD, cat. No. 563993), Ly6C (BD, cat. No. 553104), Ly6G (BD, cat. No. 551461), NK1.1 (BD, cat. No. 108738), SiglecF (BD, cat. No. 565183) and XCR1 (Biolegend, cat. No. 148220) were used. Cells were transferred to Absolute Counting Tubes and flow cytometric analysis was performed on a BD LSRFortessa^{™} flow cytometer (Becton Dickinson GmbH) and acquired data was analyzed using FlowJo software version 10 (TreeStar). TA99 or mAlb-mlL2 RNA monotherapies did not have significant effects on the tumor growth of B16F10 tumors (Figure 33 A). mAlb-mlL2 monotherapy significantly inhibited the growth of B16F10-*B2m*^{-/-} tumors. (Figure 33 B). TA99 and mAlb-mIL2 combination therapy significantly inhibited the growth of both B16F10 and B16F10-*B2m*^{*-*/-} tumors. The tumor growth inhibition was reflected in statistically improved survival of mice treated with TA99 and mAlb-mIL2 therapy compared to the control group in both tumor models (Figure 34). All mice bearing B16F10 and B16F10-*B2m*^{-/-} tumors in the control groups or mAlb-mlL2 monotherapy treated groups had to be sacrificed due to progressing tumors. TA99 monotherapy led to complete tumor rejection and survival until day 100 by 1/10 mice (10%) in the B16F10 model and 2/10 mice (20%) in the B16F10-*B2m*^{-/-} model. The TA99 and mAlb-mlL2 combination treatment was superior to the monotherapies in both models and induced complete tumor rejection by 3/10 mice (30%) in the B16F10 model and 6/10 mice (60%) in the B16F10-*B2m*^{-/-} model.

We analyzed the infiltration of several immune cell subsets in B16F10-*B2m*^{-/-} tumors after monotherapies as well as TA99 and mAlb-mIL2 combination therapy and compared them to the control group. mAlb-mIL2 monotherapy significantly enhanced the infiltration by CD4⁺ Th cells, Treg cells, CD8⁺ T cells, γδ T cells, NK cells, NKT cells, macrophages, eosinophils and CD11b⁺ DCs (Figure 35, 36, 37 and 38). Slightly enhanced, but not statistically significant, were cDC1s, monocytes and neutrophils. TA99 monotherapy did not have any detectable effects on the immune infiltration, while the infiltration of tumors after TA99 and mAlb-mIL2 combination therapy was similar to mAlb-mIL2 monotherapy in all cases. Furthermore, we analyzed the expression of FcyRs by macrophages and monocytes after monotherapies and TA99 and mAlb-mIL2 combination therapy and compared them to the control group. mAlb-mIL2 monotherapy, but not TA99 monotherapy, enhanced the expression of FcyRI, FcyRII/III and FcγRVI by intratumoral macrophages and monocytes (Figure 39 and 40). The expression of FcyRs was lower in the TA99 and mAlb-mIL2 combination therapy treated group compared to the group treated with mAlb-mlL2 monotherapy, which is likely due to TA99-induced internalization of FcyRs.

In order to identify cell types that are important for the antitumoral effect against MHC class I deficient tumor cells, mice (n=8-15 per group) were inoculated subcutaneously (s.c.) with 3x10⁵ B16F10-*B2m*^{-/-} cells and treated as described in Figure 33 with anti-Trp1 (TA99) antibody on day 5, 8, 12, 15, 19, 22 and with RNA coding for mAlb-mlL2 on day 5, 12 and 19 after tumor inoculation. The control group received an isotype control antibody and RNA coding for mAlb (not coding for any cytokine). Depleting antibodies against NK1.1 (Bioxcell, cat. No. BE0036), CSF1R (Bioxcell, cat. No.BE0213) or Ly6G (Bioxcell, cat. No. BE0075-1) or an irrelevant control antibody (non-depleting) (Bioxcell, cat. No. BE0089) were injected intraperitoneally (i.p.) with a loading dose of 400 µg on day 4 and following doses of 200 µg (irrelevant antibody, NK1.1 or Ly6G) or 300 µg (CSF1R) on day 7, 11, 14, 18 and 20 after tumor inoculation. Survival of groups that were injected with depleting antibodies was compared to the survival of the group receiving an irrelevant antibody to determine differences in the therapeutic effect upon immune cell depletion. Successful depletion of NK cells and neutrophils was confirmed by flow cytometric analysis of blood samples (staining as described in Kranz *et al.* (Kranz, L. M. et al. Nature 534, 396-401 (2016)) stained with antibodies against CD3 (Biolegend, cat. No. 100227), CD11b (BD, cat. No. 550993), CD45 (BD, cat. No. 564279), Ly6C (BD, cat. No. 553104), Ly6G (BD, cat. No. 551461) and NK1.1 (Biolegend, cat. No. 108720). Flow cytometric analysis was performed on a BD LSRFortessa^{™} flow cytometer (Becton Dickinson GmbH) and acquired data was analyzed using FlowJo software version 10 (TreeStar).

We did not observe significant effects of the depletion of NK cells (NK1.1), macrophages (CSF1R) or neutrophils (Ly6G) on the survival of mice treated with TA99 and mAlb-mIL2 combination therapy (Figure 41). However, we observed the tendency towards a weaker antitumoral effect after macrophage depletion, since several mice had to be sacrificed earlier and only 4/13 (30.8%) compared to 8/15 (53.3%) survived until day 100. It has to be noted, that depletion of NK cells and neutrophils was confirmed by flow cytometric analysis of stained blood samples (Figure 42). Successful depletion of macrophages was not confirmed in this experiment and the rather small difference in the survival could be due to incomplete macrophage depletion. For this reason, the experiment will be repeated and handed in later after full macrophage depletion could be confirmed.

Taken together, we identified anti-TAA antibody and IL2 combination therapy as an efficient treatment for MHC class I deficient murine tumor models. While the antibody mediated antigen-specific immune recognition of the tumor cells, IL2 induced inflammation in the TME, which lead to influx of a broad range of immune cells. Furthermore, macrophages and monocytes, which are able to clear opsonized target cells through ADCP, strongly upregulated FcyRs in response to IL2, suggesting that IL2 licenses innate immune cells for antibody effector mechanisms. The combination therapy lead to complete rejection of MHC class I deficient tumors. This is a particularly striking finding, as a significant role of CD8⁺ T cell immunity in the antitumoral effect of tumor-targeting antibodies and especially the combination of antibodies with IL2 in murine tumor models is assumed (Park et al. Cancer Cell 18, 160-70 (2010), Yang et al. Mol Ther 21, 91-100 (2013), Zhu et al. Cancer Cell 27, 489-501 (2015), Kwan et al. J Exp Med 214, 1679-90 (2017)). Nevertheless, our data clearly demonstrates the unanticipated suitability of antibody and IL2 combination therapy against MHC class I deficient tumors through re-establishment of antigen-specific tumor cell recognition by innate immune cells and induction of inflammation in the TME.

### Example 5: Classical cancer therapy enhances control of MHC class I deficient tumors by antibody and IL2 combination immunotherapy

Our last aim was to investigate whether the control of MHC class I deficient tumors could be further enhanced by classical cancer therapy. Mice (n=12-13 per group) were inoculated subcutaneously (s.c.) with 3x10⁵ B16F10-*B2m*^{*-*/-} cells and injected intraperitoneally (i.p.) with 200 mg/kg cyclophosphamide (CTX) (Baxter, Endoxan) or vehicle as control on day 6 after tumor inoculation and then treated with or without anti-Trp1 (TA99) antibody on day 7, 10, 14, 17 and 21 and RNA coding for mAlb-mIL2 on day 7, 14 and 21 as described in Example 4. Control groups were treated with isotype control antibody or RNA coding for mAlb (not coding for any cytokine). Anti-tumor efficacy was determined as tumor growth inhibition in the group treated with CTX and TA99 and mAlb-mlL2 combination therapy compared to groups treated with CTX monotherapy, TA99 and mAlb-mlL2 combination therapy or control. Comparison of the tumor growth showed that the tumor progression was nearly completely inhibited by treatment with CTX followed by TA99 and mAlb-mIL2 combination therapy (Figure 43). The growth inhibition was significantly improved relative to CTX monotherapy or TA99 and mAlb-mIL2 combination therapy. In conclusion, classical cancer therapy combines well with antibody and IL2 combination therapy and results in synergistic antitumoral control of MHC class I deficient tumors.

### Example 6: Antibody and IL2 combination immunotherapy leads to therapeutic immune responses against IFN signaling deficient tumors

Besides the loss of MHC class I, deficiency in IFN signaling is another way for tumors to escape T cell elimination and is frequently observed in patients (Gao et al. Cell 167, 397-404 (2016)). Our goal was the identification of a treatment that is applicable for T cell resistant tumors independently of the particular resistance mechanism. For this reason, we also investigated whether the treatment would be effective against tumors that have defect IFN signaling pathways through mutation of *Jak1.* Mice (n=10 per group) were inoculated subcutaneously (s.c.) with 3x10⁵ B16F10-*Jak1*^{-/-} cells and treated as described in Figure 33 with anti-Trp1 (TA99) antibody on day 5, 8, 12, 15, 19 and with RNA coding for mAlb-mlL2 on day 5, 12 and 19 after tumor inoculation. The control group received an isotype control antibody and RNA coding for mAlb (not coding for any cytokine).

TA99, but not mAlb-mlL2 RNA monotherapy significantly retarded the tumor growth of B16F10-*Jak1*^{-/-} tumors, while TA99 and mAlb-mIL2 combination therapy led to complete tumor growth inhibition (Figure 44 A). The tumor growth inhibition was reflected in statistically improved survival of mice treated with TA99 monotherapy or TA99 and mAlb-mlL2 combination therapy compared to the control group (Figure 44 B). 2/10 (20%) B16F10-*Jak1*^{-/-} tumors in the control group regressed spontaneously, and mAlb-mIL2 monotherapy led to regression of 5/10 (50%) tumors. TA99 monotherapy led to complete tumor rejection and survival until day 100 by 8/10 mice (80%) and the TA99 and mAlb-mlL2 combination treatment induced complete tumor rejection by 10/10 mice (100%). In summary, antibody and IL2 combination therapy resulted in therapeutic immune responses against IFN signaling deficient tumors. In conclusion, the treatment regimen is a versatile approach for the therapy of tumors that acquired resistance against elimination through CD8⁺ T cells via loss of MHC class I or impaired IFN signaling.

### Example 7: Antibody and IL2 combination immunotherapy requires macrophages, CD8⁺ T cells and IFNγ

Given that macrophage depletion resulted in a tendency towards a weaker antitumoral effect of antibody and mAlb-mlL2 combination therapy as described in Example 4, an improved protocol for the depletion of macrophages was established using higher antibody doses to validate these results. C57BI/6 mice (n=6) were inoculated subcutaneously (s.c.) with 3x10⁵ B16F10*-B2m*^{*-*/*-*} cells as described in Example 1. An antibody against CSF1R (Bioxcell, cat. No. BE0213) was injected intraperitoneally (i.p.) into 5 mice with a dose of 600 µg on day 10 and a dose of 350 µg on day 12 after tumor inoculation and 1 mouse was left untreated as control. Tumors were excised 13 days after tumor inoculation and digested as described in Example 1. The samples were processed to single cell suspensions and stained as described in Grunwitz *et al.* (Grunwitz, C. et al. Oncoimmunology 8, published online (2019)). Dead cells were stained with Fixable Yellow Dead Cell Stain (Life technologies, cat No. L34967). Antibodies against CD11b (BD, cat. No. 553310), CD45 (BD, cat. No. 564279), F4/80 (Biolegend, cat. No. 123132) and GR-1 (Biolegend, cat. No. 123132 and 123146) were used. Flow cytometric analysis was performed on a BD LSRFortessa^{™} flow cytometer (Becton Dickinson GmbH) and acquired data was analyzed using FlowJo software version 10 (TreeStar).

A distinct CD11b⁺ F4/80⁺ macrophage population was observable in the untreated control tumor, whereas injection of anti-CSF1R resulted in reduction of the macrophage population in 4/5 mice (Figure 45). Thus, injection of high doses of anti-CSF1R is able to deplete macrophages within 3 days.

In order to utilize the improved macrophage depletion protocol to obtain valid data on the importance of macrophages for antibody and mAlb-mIL2 combination therapy and to investigate the role of lymphocytes, C57BI/6 mice (n=10-15 per group) were inoculated subcutaneously (s.c.) with 3x10⁵ B16F10*-B2m*^{*-*/*-*} cells and treated as described in Figure 33 with anti-Trp1 (TA99) antibody on day 5, 8, 12, 15 and 19 and with RNA coding for mAlb-mIL2 on day 5, 12 and 19 after tumor inoculation. The control group was untreated. Depleting antibodies against CD4 (Bioxcell, cat. No. BE0119) and CD90.2 (Bioxcell, cat. No. BE0066) or an irrelevant control antibody (non-depleting) (Bioxcell, cat. No. BE0089) were injected intraperitoneally (i.p.) with a loading dose of 400 µg on day 3 and following doses of 200 µg on day 7, 10, 14 and 20 (on day 20 only control and anti-CD4) after tumor inoculation. The antibody against CSF1R (Bioxcell, cat. No. BE0213) was injected intraperitoneally (i.p.) with a loading dose of 600 µg on day 2 and following doses of 350 µg on day 5, 7, 10, 12 and 14 after tumor inoculation. Survival of groups that were injected with depleting antibodies was compared to the survival of the group receiving an irrelevant antibody to determine differences in the therapeutic effect upon immune cell depletion. Successful depletion of CD4⁺ T cells and total CD90.2⁺ lymphocytes was confirmed by flow cytometric analysis of blood samples (staining as described in Kranz *et al.* (Kranz, L. M. et al. Nature 534, 396-401 (2016)) stained with antibodies against CD4 (BD, cat. No. 564298), CD45 (BD, cat. No. 564279) and CD90.2 (BD, cat. No. 553003). Flow cytometric analysis was performed on a BD LSRFortessa^{™} flow cytometer (Becton Dickinson GmbH) and acquired data was analyzed using FlowJo software version 10 (TreeStar).

The successful depletion of CD4⁺ T cells upon injection with anti-CD4 and total lymphocytes upon injection with anti-CD90.2 was confirmed in the blood of mice at the time point of antibody and mAlb-mIL2 treatment initiation (Figure 46). Depletion of CD4⁺ T cells did not affect the antitumoral activity of antibody and mAlb-mlL2 combination therapy, while a significant reduction in the survival upon total lymphocyte depletion indicated that a lymphocytic population must be required for the antitumoral effect (Figure 47). The depletion of macrophages nearly completely inhibited the antitumoral activity and confirmed that macrophages are essential for the therapeutic activity.

Given that the depletion of NK cells or CD4⁺ T cells did not affect the antitumoral activity of antibody and mAlb-mIL2 combination therapy, it was investigated whether CD8⁺ T cells are the required lymphocytic cell population. It was further asked whether IFNy is required for the antitumoral activity of antibody and mAlb-mIL2 combination therapy against MHC class I deficient tumors, given that IFNy secreted by CD8⁺ T cells polarizes macrophages into a proinflammatory, antitumoral phenotype (Gubin et al. Cell 175(4), 1014-30 (2018)) and IFNy activation of macrophages enhances the elimination of antibody-opsonized tumor cells through ADCP (Shi et al. J Immunol 194, 4379-86 (2015)). C57BI/6 mice (n=8-15 per group) were inoculated subcutaneously (s.c.) with 3x10⁵ B16F10-*B2m*^{-/-} cells and treated as described in Figure 33 with anti-Trp1 (TA99) antibody on day 5, 8, 12, 15 and 19 and with RNA coding for mAlb-mIL2 on day 5, 12 and 19 after tumor inoculation. The control group was untreated. Depleting antibody against CD8 (Bioxcell, cat. No. BE0117) or neutralizing antibody against IFNγ (Bioxcell, cat. No. BE0055) or an irrelevant control antibody (non-depleting) (Bioxcell, cat. No. BE0088) were injected intraperitoneally (i.p.) with a loading dose of 400 µg on day 3 and following doses of 200 µg anti-CD8 on day 7 and 10 or 250 µg control antibody or anti-IFNγ on day 5, 7 and 10 after tumor inoculation. Survival of groups that were injected with depleting antibodies was compared to the survival of the group receiving an irrelevant antibody to determine differences in the therapeutic effect upon immune cell depletion. Successful depletion of CD8⁺ T cells was confirmed by flow cytometric analysis of blood samples (staining as described in Kranz *et al.* (Kranz, L. M. et al. Nature 534, 396-401 (2016)) stained with antibodies against CD8 (BD, cat. No. 553032) and CD45 (BD, cat. No. 564279). Flow cytometric analysis was performed on a BD LSRFortessa^{™} flow cytometer (Becton Dickinson GmbH) and acquired data was analyzed using FlowJo software version 10 (TreeStar).

The successful depletion of CD8⁺ T cells upon injection with anti-CD8 was confirmed in the blood of mice at the time point of antibody and mAlb-mIL2 treatment initiation (Figure 48). The depletion of CD8⁺ T cells or the neutralization of IFNγ similarly inhibited the antitumoral activity of antibody and mAlb-mIL2 therapy, showing that CD8⁺ T cells are the required lymphocytic population and suggesting that these cells contribute *via* secretion of IFNy (Figure 49).

The next aim was to analyze whether proinflammatory polarization of macrophages occurs upon treatment with antibody and mAlb-mIL2 combination therapy and to confirm the direct relationship between macrophages, CD8⁺ T cells and IFNγ, C57BI/6 mice (n=7-8 per group) were inoculated subcutaneously (s.c.) with 3x10⁵ B16F10*-B2m*^{*-*/*-*} cells and treated as described in Figure 33 with anti-Trp1 (TA99) antibody on day 9, 12 and 16 and with RNA coding for mAlb-mIL2 on day 9 and 16 after tumor inoculation. The control group received an isotype control antibody and RNA coding for mAlb (not coding for any cytokine). Tumors were excised 11 days after the first treatment. In a second experiment, C57BI/6 mice (n=15 per group) were inoculated and treated in the same way and additionally injected intraperitoneally (i.p.) with depleting antibody against CD8 (Bioxcell, cat. No. BE0117) or neutralizing antibody against IFNy (Bioxcell, cat. No. BE0055) or an irrelevant control antibody (non-depleting) (Bioxcell, cat. No. BE0088) with a loading dose of 400 µg on day 7 and following doses of 200 µg anti-CD8 on day 11 and 14 or 250 µg control antibody or anti-IFNγ on day 9, 11 and 14 after tumor inoculation. Tumors were excised 9 days after the first treatment. All tumors were digested as described in Example 1. The samples were processed to single cell suspensions and stained as described in Grunwitz *et al.* (Grunwitz, C. et al. Oncoimmunology 8, published online (2019)). Dead cells were stained with Fixable Yellow Dead Cell Stain (Life technologies, cat No. L34967). Antibodies against CD11b (BD, cat. No. 562127), CD45 (BD, cat. No. 564279), CD206 (Biolegend, cat. No. 141720), GR-1 (Biolegend, cat No. 108423), F4/80 (Biolegend, cat. No. 123132) and MHC II (BD, cat. No. 551799) were used. Flow cytometric analysis was performed on a BD LSRFortessa^{™} flow cytometer (Becton Dickinson GmbH) and acquired data was analyzed using FlowJo software version 10 (TreeStar).

In order to determine the proinflammatory state of tumor-infiltrating macrophages, the proportions of proinflammatory M1-like macrophages (MHC II^{high}, CD206^{low}) and antiinflammatory M2-like macrophages (MHC II^{low}, CD206^{high}) were quantified by flow cytometry and the M1/M2 ratio was calculated. Antibody therapy alone did not significantly alter the M1/M2 ratio, whereas mAlb-mIL2 in monotherapy or in combination with TA99 significantly increased the M1/M2 ratio, indicating proinflammatory remodeling of the macrophage compartment (Figure 50A). The proinflammatory polarization of the macrophages was similarly abrogated upon CD8⁺ T-cell depletion or IFNy neutralization, showing that CD8⁺ T cells and IFNy shape the macrophage phenotype in response to mAlb-mlL2 and highlighting that the significance of CD8⁺ T cells most likely relies on the production of IFNy, but not on direct tumor cell elimination (Figure 50B).

In conclusion, these results highlight a previously unrecognized role of CD8⁺ T cells in the control of tumors that avoid direct T-cell recognition through absence of MHC class I. Given the fact that macrophages are the only FcyR-expressing cell type identified to be essential for the therapeutic activity of antibody and mAlb-mlL2 therapy, they are most likely responsible for the elimination of antibody-opsonized tumor cells. CD8⁺ T cells activate the macrophages *via* IFNy in order to enable them for efficient tumor cell elimination.

### Example 8: Antibody and mAlb-mIL2 therapy prevents acquired resistance during the course of ICB

A substantial fraction of tumor patients treated with ICB experiences relapse after initial disease control due to the development acquired resistance (Schoenfeld et al. Cancer Cell 37(4), 443-55 (2020)) resulting from the outgrowth of initially small numbers of MHC class I deficient tumor cell clones that evade T-cell recognition (Zaretsky et al. N Engl J Med 375, 819-29 (2016)). In order to model acquired resistance, C57BI/6 mice (n=15 per group) were inoculated subcutaneously (s.c.) with 3x10⁵ cells of a mixture containing 75% B16F10 and 25% B16F10-*B2m*^{-/-} cells. Mice were treated as described in Figure 33 with anti-Trp1 (TA99) antibody on day 3, 7, 10, 14 and 17 and with RNA coding for mAlb-mlL2 on day 3, 10 and 17 after tumor inoculation. An isotype antibody and RNA coding for mAlb (not coding for any cytokine) were used as controls. Mice were additionally injected intraperitoneally (i.p.) with 200 µg anti-PD-1 (Bioxcell, cat. No. BE0146) on day 3, 7, 10, 14 and 17 combined with anti-CTLA4 (Bioxcell, cat. No. BE0131) with a loading dose of 200 µg on day 3 and following doses of 100 µg on day 7, 10, 14 and 17. Isotype antibodies (Bioxcell, cat. No. BE0089 and BE0087) served as controls. The survival of the group receiving anti-PD-1 and anti-CTLA4 was compared to the group receiving anti-PD-1, anti-CTLA4, TA99 and mAlb-mIL2 to determine whether the addition of tumor-binding antibody and mAlb-mIL2 augments the antitumoral activity of ICB. Tumors were excised when the mice reached endpoint criteria and digested as described in Example 1. The samples were processed to single cell suspensions and stained as described in Grunwitz *et al.* (Grunwitz, C. et al. Oncoimmunology 8, published online (2019)) after the single cell suspensions were stimulated with IFNy as described in Example 1. Dead cells were stained with Fixable Yellow Dead Cell Stain (Life technologies, cat No. L34967). Antibodies against CD45 (BD, cat. No. 564279), H2-K^{b} (BD, cat. No. 553570) and H2-D^{b} (BD, cat. No. 553574) were used. Flow cytometric analysis was performed on a BD LSRFortessa^{™} flow cytometer (Becton Dickinson GmbH) and acquired data was analyzed using FlowJo software version 10 (TreeStar). The fraction of MHC class I positive tumor cells was compared across the different groups to determine whether ICB leads to positive selection of resistant MHC class I deficient cells and antibody and mAlb-mIL2 combination therapy is able to prevent the positive selection.

Anti-PD-1 and anti-CTLA4 ICB resulted in tumor growth delay, which was followed by tumor outgrowth in 80% (12/15) of the mice, while 20% (3/15) of the mice rejected the tumors, perhaps due to bystander elimination of MHC class I deficient cells (Spiotto et al. Nat Med 10(3), 294-8 (2004)) (Figure 51). TA99 and mAlb-mIL2 therapy without ICB resulted in the rejection of 33.3% (5/15) of the tumors and the combination of TA99 and mAlb-mIL2 with ICB resulted in superior tumor rejection given that 60% (9/15) of the mice were tumor free at the end of the observation period. This was reflected by significantly improved survival compared to the group treated only with ICB.

The analysis of the proportion of MHC class I positive tumor cells in the tumors showed that the tumor cell ratio remained stable in the group that was treated only with controls, while ICB resulted in highly significant enrichment of MHC class I deficient tumor cells. This indicated the selective depletion of T-cell sensitive tumor cells followed by outgrowth of ICB-resistant tumor cells, leading to acquired resistance and tumor relapse. The addition of TA99 and mAlb-mlL2 completely prevented the selective enrichment of MHC class I deficient cells.

In conclusion, the addition of antibody and mAlb-mIL2 combination therapy to ICB is able to prevent the emergence of acquired resistance by undermining the selection advantage of T-cell resistant tumor cells during the course of immunotherapy that induces an MHC class I-dependent T-cell response.

### Example 9: Antibody and mAlb-mIL2 combination immunotherapy is efficient against MC38-Her2-B2m^{-l-} tumors and restores complete responses of MHC class I deficient tumors to classical cancer therapy

In order to investigate the efficacy of antibody and mAlb-mIL2 therapy against a second MHC class I deficient tumor model, MC38-*B2m*^{-/-} were transduced using a lentiviral vector containing the gene encoding for rat Her2/neu (synthesized by GeneArt, Thermo Fisher Scientific) under control of the Ef1a promoter as described in Beissert *et al.* (Beissert et al. Mol Ther 28(1), 119-28 (2019)). The transduced cells were cultured in medium containing 4 µg/mL Blasticidin for 1 week at 37 °C and 5% CO₂ and subsequently, single clones were obtained as described in Example 1 and screened for Her2 expression by flow cytometry. Her2 positive clones were cultured in 6-well plates for 4 weeks at 37 °C and 5% CO₂ without Blasticidin and screened for Her2 expression. One clone showing stable integration of the transgene was denoted MC38-Her2-*B2m*^{-/-} and used for further studies (Figure 53).

To test whether MC38-Her2*-B2m*^{-/-} respond to antibody and mAlb-mlL2 combination therapy, C57BI/6 mice (n=10 per group) were inoculated subcutaneously (s.c.) with 5x10⁵ MC38-Her2-*B2m*^{-/-} cells. Mice were injected intraperitoneally (i.p.) with 200 µg anti-Her2 antibody (7.16.4) (Bioxcell, cat. No. BE0277) or isotype control (Bioxcell, cat. No. BE0085) on day 3, 6, 10, 13, 17 and 24 and with RNA coding for mAlb-mIL2 as described in Figure 33 on day 3, 10, 17 and 24 after tumor inoculation. Anti-tumor efficacy was determined as tumor growth inhibition in the test groups compared to the control group and survival during an observation period of 100 days.

Treatment with 7.16.4 or mAlb-mIL2 as monotherapies and in combination therapy significantly inhibited the growth of the tumors (Figure 54A). Treatment with 7.16.4 did not induce tumor rejection, while 10% (1/10) of the mice treated with mAlb-mlL2 in monotherapy rejected the tumor. 50% (5/10) mice treated with antibody and mAlb-mIL2 combination therapy rejected the tumors and remained tumor-free until the end of the experiment, which resulted in significantly improved survival compared to the group that received mAlb-mIL2 monotherapy (Figure 54B).

Given that MC38-Her2-*B2m*^{-/-} tumors were efficiently targetable using antibody and mAlb-mlL2 combination therapy, we asked whether the treatment is able to restore complete responses to chemotherapy in this tumor model. First, in order to determine the resistance of MC38-*B2m*^{-/-} to chemotherapy, mice were inoculated with MC38 (n=15 per group) or MC38-*B2m*^{-/-} (n=10 per group) as described in Example 1 and injected with OX as described in Figure 29 on day 4, 11 and 18 after tumor inoculation. The control groups received vehicle. Anti-tumor efficacy was determined as survival of the test groups compared to the control groups during an observation period of 100 days.

Chemotherapy significantly improved the survival of mice bearing MC38 or MC38-*B2m*^{-/-} tumors (Figure 55). All MC38-*B2m*^{-/-} tumors eventually progressed, while chemotherapy doubled the rejection rate of MC38 tumors from 13.3% (2/15) in the control group to 26.7% (4/15), showing that functional MHC class I expression is required for complete responses of MC38 tumors to chemotherapy.

In order to analyze whether the addition of antibody and mAlb-mlL2 combination therapy re-enables complete responses to chemotherapy, mice (n=15 per group) were inoculated with MC38-Her2-*B2m*^{*-*/-} cells and injected with OX on day 4, 11 and 18 after tumor inoculation as described in Figure 29 and treated with anti-Her2 (7.16.4) antibody on day 5, 8, 12, 15 and 19 and RNA coding for mAlb-mIL2 on day 7, 14 and 21 as described in Figure 54. Control groups were treated with vehicle, isotype control antibody or RNA coding for mAlb (not coding for any cytokine). Anti-tumor efficacy was determined as survival of the group treated with OX and 7.16.4 and mAlb-mlL2 combination therapy compared to groups treated with OX monotherapy or 7.16.4 and mAlb-mIL2 combination therapy,

100% (15/15) of the tumors treated with OX in monotherapy or with 7.16.4 and mAlb-mlL2 combination therapy progressed during the course of the therapy (Figure 56). The addition of 7.16.4 and mAlb-mIL2 to OX enabled the rejection of 53.3% (8/15) of the tumors and resulted in significantly improved survival compared to the other treatment groups.

In summary, the efficient targeting of MHC class I deficient tumors was verified in a second tumor model using a different antibody target. It was furthermore confirmed in a second model that MHC class I deficiency results in the resistance of tumors to complete responses induced by classical cancer therapy. The addition of antibody and mAlb-mIL2 to classical cancer therapy re-enables complete responses and results in synergistic tumor control, highlighting the applicability of this approach.

### Example 10: Classical cancer therapy enhances control of IFN signaling deficient tumors by antibody and IL2 combination immunotherapy

The synergistic activity of classical cancer therapy and antibody and mAlb-mIL2 combination therapy was furthermore investigated against tumors that are deficient in IFN signaling. Mice (n=14-15 per group) were inoculated subcutaneously (s.c.) with 5x10⁵ B16F10-*Jak1*^{-/-} cells and treated with 150 mg/kg CTX as described in Example 5 on day 7 after tumor inoculation and with anti-Trp1 (TA99) antibody on day 8, 11, 15, 18 and 22 and RNA coding for mAlb-mlL2 on day 8, 15 and 22 after tumor inoculation as described in Example 4. Control groups were treated with vehicle, isotype control antibody or RNA coding for mAlb (not coding for any cytokine). Anti-tumor efficacy was determined as survival of the group treated with CTX and TA99 and mAlb-mlL2 combination therapy compared to groups treated with CTX monotherapy or TA99 and mAlb-mlL2 combination therapy.

CTX induced tumor growth delay and led to the rejection of 6.7% (1/15) of the tumors, while TA99 and mAlb-mIL2 combination therapy induced the rejection of 69.2% (9/15) of the tumors (Figure 57). CTX sensitized the tumors for superior rejection upon treatment with TA99 and mAlb-mIL2, which augmented the tumor rejection rate to 86.7% (13/15). In conclusion, classical cancer therapy improves the control of IFN signaling deficient tumors treated with antibody and mAlb-mlL2 combination therapy.

The invention provides, in particular, the following:
1. A method of treating a subject with cancer that is at least partially resistant to an MHC-dependent T cell response comprising administering to the subject:
   a. a polypeptide comprising IL2 or a functional variant thereof or a polynucleotide encoding a polypeptide comprising IL2 or a functional variant thereof; and
   b. antibody-based immunotherapy against cancer.
2. The method of item 1, wherein the cancer does not adequately respond to treatment based on T cells such as MHC-dependent T cells, in particular CD8⁺ T cells.
3. The method of item 1 or 2, wherein the cancer is deficient in processing and/or presenting antigens.
4. The method of any one of items 1 to 3, wherein the cancer is MHC-I deficient.
5. The method of any one of items 1 to 4, wherein the deficiency in MHC-I is due to a mutation or partial or full loss of MHC-I alleles such as beta2-microglobulin (B2M).
6. The method of any one of items 1 to 5, wherein the cancer is deficient in stimulating T cells.
7. The method of any one of items 1 to 6, wherein the cancer is deficient in IFN-signaling such as type I IFN or IFNy signaling.
8. A method of preventing a cancer to develop a resistance to an MHC-dependent T cell response in a subject with cancer comprising administering to the subject:
   a. a polypeptide comprising IL2 or a functional variant thereof or a polynucleotide encoding a polypeptide comprising IL2 or a functional variant thereof; and
   b. antibody-based immunotherapy against cancer.
9. The method of item 8, wherein the resistance comprises that the cancer does not adequately respond to treatment based on T cells such as MHC-dependent T cells, in particular CD8⁺ T cells.
10. The method of item 8 or 9, wherein the resistance comprises that the cancer is deficient in processing and/or presenting antigens.
11. The method of any one of items 8 to 10, wherein the resistance comprises that the cancer is MHC-I deficient.
12. The method of any one of items 8 to 11, wherein the deficiency in MHC-I is due to a mutation or partial or full loss of MHC-I alleles such as beta2-microglobulin (B2M).
13. The method of any one of items 8 to 12, wherein the resistance comprises that the cancer is deficient in stimulating T cells.
14. The method of any one of items 8 to 13, wherein the resistance comprises that the cancer is deficient in IFN-signaling such as type I IFN or IFNy signaling.
15. The method of any one of items 1 to 14, wherein the polynucleotide encoding a polypeptide comprising IL2 or a functional variant thereof is RNA.
16. The method of any one of items 1 to 15, wherein the antibody-based immunotherapy against cancer comprises administering a therapeutic antibody against cancer or a polynucleotide encoding a therapeutic antibody against cancer.
17. The method of item 16, wherein the therapeutic antibody against cancer is directed against a tumor antigen expressed by cells of the cancer.
18. The method of item 16 or 17, wherein the polynucleotide encoding a therapeutic antibody against cancer is RNA.
19. The method of any one of items 1 to 18 which comprises administering to the subject:
   a. RNA encoding a polypeptide comprising IL2 or a functional variant thereof; and
   b. a therapeutic antibody against cancer.
20. The method of any one of items 1 to 19, wherein the cancer is associated with expression or elevated expression of an antigen.
21. The method of item 20, wherein the antigen is a tumor antigen.
22. The method of item 20 or 21, wherein the antibody-based immunotherapy against cancer is directed against said antigen.
23. The method of any one of items 1 to 22, wherein the polypeptide comprising IL2 or a functional variant thereof is extended pharmacokinetic (PK) IL2.
24. The method of item 23, wherein the extended-PK IL2 comprises a fusion protein.
25. The method of item 24, wherein the fusion protein comprises a moiety of IL2 or a functional variant thereof and a moiety selected from the group consisting of serum albumin, an immunoglobulin fragment, transferrin, Fn3, and variants thereof.
26. The method of item 25, wherein the serum albumin comprises mouse serum albumin or human serum albumin.
27. The method of item 25, wherein the immunoglobulin fragment comprises an immunoglobulin Fc domain.
28. A medical preparation comprising:
   a. a polypeptide comprising IL2 or a functional variant thereof or a polynucleotide encoding a polypeptide comprising IL2 or a functional variant thereof;
   b. a therapeutic antibody against cancer or a polynucleotide encoding a therapeutic antibody against cancer; and
   c. instructions for use of the medical preparation for treating or preventing cancer that is at least partially resistant to an MHC-dependent T cell response.
29. A medical preparation comprising:
   a. a polypeptide comprising IL2 or a functional variant thereof or a polynucleotide encoding a polypeptide comprising IL2 or a functional variant thereof; and
   b. a therapeutic antibody against cancer or a polynucleotide encoding a therapeutic antibody against cancer
      for treating or preventing cancer that is at least partially resistant to an MHC-dependent T cell response.
30. A medical preparation comprising:
   a. a polypeptide comprising IL2 or a functional variant thereof or a polynucleotide encoding a polypeptide comprising IL2 or a functional variant thereof;
   b. a therapeutic antibody against cancer or a polynucleotide encoding a therapeutic antibody against cancer; and
   c. instructions for use of the medical preparation for preventing a cancer to develop a resistance to an MHC-dependent T cell response.
31. A medical preparation comprising:
   a. a polypeptide comprising IL2 or a functional variant thereof or a polynucleotide encoding a polypeptide comprising IL2 or a functional variant thereof; and
   b. a therapeutic antibody against cancer or a polynucleotide encoding a therapeutic antibody against cancer
      for preventing a cancer to develop a resistance to an MHC-dependent T cell response.
32. The medical preparation of any one of items 28 to 31, which is a kit.
33. The medical preparation of any one of items 28 to 32, which comprises the polypeptide comprising IL2 or a functional variant thereof or polynucleotide encoding a polypeptide comprising IL2 or a functional variant thereof, and the therapeutic antibody against cancer or polynucleotide encoding a therapeutic antibody against cancer in separate containers.
34. The medical preparation of any one of items 28 to 31, which is a pharmaceutical composition.
35. The medical preparation of item 34, wherein the pharmaceutical composition further comprises one or more pharmaceutically acceptable carriers, diluents and/or excipients.
36. A polypeptide comprising IL2 or a functional variant thereof or a polynucleotide encoding a polypeptide comprising IL2 or a functional variant thereof for treating or preventing cancer that is at least partially resistant to an MHC-dependent T cell response, wherein the polypeptide comprising IL2 or a functional variant thereof or polynucleotide encoding a polypeptide comprising IL2 or a functional variant thereof is to be administered together with a therapeutic antibody against cancer or a polynucleotide encoding a therapeutic antibody against cancer.
37. A therapeutic antibody against cancer or a polynucleotide encoding a therapeutic antibody against cancer for treating or preventing cancer that is at least partially resistant to an MHC-dependent T cell response, wherein the therapeutic antibody against cancer or polynucleotide encoding a therapeutic antibody against cancer is to be administered together with a polypeptide comprising IL2 or a functional variant thereof or a polynucleotide encoding a polypeptide comprising IL2 or a functional variant thereof.
38. A polypeptide comprising IL2 or a functional variant thereof or a polynucleotide encoding a polypeptide comprising IL2 or a functional variant thereof for preventing a cancer to develop a resistance to an MHC-dependent T cell response, wherein the polypeptide comprising IL2 or a functional variant thereof or polynucleotide encoding a polypeptide comprising IL2 or a functional variant thereof is to be administered together with a therapeutic antibody against cancer or a polynucleotide encoding a therapeutic antibody against cancer.
39. A therapeutic antibody against cancer or a polynucleotide encoding a therapeutic antibody against cancer for preventing a cancer to develop a resistance to an MHC-dependent T cell response, wherein the therapeutic antibody against cancer or polynucleotide encoding a therapeutic antibody against cancer is to be administered together with a polypeptide comprising IL2 or a functional variant thereof or a polynucleotide encoding a polypeptide comprising IL2 or a functional variant thereof.

## Claims

1. A medical preparation for use in a method of treating a patient with a cancer that is at least partially resistant to an MHC-dependent T cell response, wherein the method comprises administering to the patient the medical preparation, which medical preparation comprises:
a. a polypeptide comprising IL2 or a functional variant thereof or a polynucleotide encoding the polypeptide or functional variant, wherein the functional variant of IL2 is functionally equivalent to wild-type IL2; and
b. a therapeutic antibody against cancer or a polynucleotide encoding a therapeutic antibody against cancer,
wherein the at least partially resistant cancer (i) does not adequately respond to treatment based on T cells such as MHC-dependent T cells, in particular CD8⁺ T cells, and/or (ii) is deficient in processing and/or presenting antigens and/or (iii) is deficient in stimulating T cells.

2. The medical preparation for use of claim 1, wherein the cancer is MHC-I deficient, preferably wherein the deficiency in MHC-I is due to a mutation or partial or full loss of MHC-1 alleles such as beta2-microglobulin (B2M).

3. The medical preparation for use of claim 1 or 2, wherein the cancer is deficient in IFN-signaling such as type I IFN or IFNγ signaling.

4. A medical preparation for use in a method of preventing a cancer in a patient to develop resistance to an MHC-dependent T cell response, wherein the method comprises administering to the patient the medical preparation, which medical preparation comprises:
a. a polypeptide comprising IL2 or a functional variant thereof or a polynucleotide encoding the polypeptide or functional variant, wherein the functional variant of IL2 is functionally equivalent to wild-type IL2; and
b. a therapeutic antibody against cancer or a polynucleotide encoding a therapeutic antibody against cancer,
wherein the at least partially resistant cancer (i) does not adequately respond to treatment based on T cells such as MHC-dependent T cells, in particular CD8⁺ T cells, and/or (ii) is deficient in processing and/or presenting antigens and/or (iii) is deficient in stimulating T cells.

5. The medical preparation for use of claim 4, wherein the resistance comprises that the cancer is MHC-I deficient, preferably wherein the deficiency in MHC-I is due to a mutation or partial or full loss of MHC-1 alleles such as beta2-microglobulin (B2M).

6. The medical preparation for use of claim 4 or 5, wherein the resistance comprises that the cancer is deficient in IFN-signaling such as type I IFN or IFNγ signaling.

7. The medical preparation for use of any one of claims 1 to 6, wherein the polynucleotide encoding a polypeptide comprising IL2 or a functional variant thereof is RNA.

8. The medical preparation for use of any one of claims 1 to 7, wherein the therapeutic antibody against cancer is directed against a tumor antigen expressed by cells of the cancer.

9. The medical preparation for use of any one of claims 1 to 8, wherein the polynucleotide encoding a therapeutic antibody against cancer is RNA.

10. The medical preparation for use of any one of claims 1 to 9, wherein the cancer is associated with expression or elevated expression of an antigen, preferably wherein the antigen is a tumor antigen.

11. The medical preparation for use of any one of claims 1 to 10, wherein the polypeptide comprising IL2 or a functional variant thereof is extended pharmacokinetic (PK) IL2, preferably wherein the extended-PK IL2 comprises a fusion protein, wherein the fusion protein optionally comprises a moiety of IL2 or a functional variant thereof and a moiety selected from the group consisting of serum albumin, an immunoglobulin fragment, transferrin, Fn3, and variants thereof.

12. The medical preparation for use of any one of claims 1 to 11, which is a pharmaceutical composition.

13. The medical preparation for use of claim 12, wherein the pharmaceutical composition further comprises one or more pharmaceutically acceptable carriers, diluents and/or excipients.

14. The medical preparation for use of any one of claims 1 to 13, wherein the medical preparation comprising the polypeptide comprising IL2 or a functional variant thereof or polynucleotide encoding a polypeptide comprising IL2 or a functional variant thereof, and the therapeutic antibody against cancer or polynucleotide encoding a therapeutic antibody against cancer are in separate containers.
